(19) Europäisches Patentamt / European Patent Office / Office européen des brevets

(11) **EP 3 699 916 A1**

(12) **EUROPEAN PATENT APPLICATION**
published in accordance with Art. 153(4) EPC

(43) Date of publication:
26.08.2020 Bulletin 2020/35

(51) Int Cl.:
*G16B 15/30* (2019.01)

(21) Application number: 18868536.6

(22) Date of filing: 03.10.2018

(86) International application number:
PCT/JP2018/037051

(87) International publication number:
WO 2019/078006 (25.04.2019 Gazette 2019/17)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 17.10.2017 JP 2017201025
26.03.2018 JP 2018058849

(71) Applicant: FUJIFILM Corporation
Tokyo 106-8620 (JP)

(72) Inventors:
• TSUMURA, Kyosuke
Minami-Ashigara-shi
Kanagawa 250-0193 (JP)
• OHIRA, Shino
Minami-Ashigara-shi
Kanagawa 250-0193 (JP)
• NAKABAYASHI, Jun
Minami-Ashigara-shi
Kanagawa 250-0193 (JP)
• TAKEI, Mizuki
Tokyo 106-8620 (JP)

(74) Representative: Klunker IP
Patentanwälte PartG mbB
Destouchesstraße 68
80796 München (DE)

(54) **FEATURE QUANTITY CALCULATING METHOD, FEATURE QUANTITY CALCULATING PROGRAM AND FEATURE QUANTITY CALCULATING DEVICE, SCREENING METHOD, SCREENING PROGRAM AND SCREENING DEVICE, COMPOUND CREATING METHOD, COMPOUND CREATING PROGRAM AND COMPOUND CREATING DEVICE**

(57) Provided are a feature quantity calculating method, a feature quantity calculating program, and a feature quantity calculating device which enable calculation of a feature quantity accurately showing chemical properties of a target structure, a screening method, a screening program, and a screening device which enable efficient screening of a pharmaceutical candidate compound using a feature quantity, and a compound creating method, a compound creating program, and a compound creating device which enable efficient creation of a three-dimensional structure of a pharmaceutical candidate compound using a feature quantity. Since the chemical properties of the target structures are exhibited as the result of an interaction between the target structure and a probe in the periphery thereof, the fact that the degree of accumulation (feature quantity) of probes is similar between target structures indicates that the chemical properties of the target structures are similar. Therefore, the feature quantity accurately showing the chemical properties of the target structure can be calculated using the feature quantity calculating method according to one aspect of the present invention.

FIG. 5

```
            START
              │
  INPUT STRUCTURAL FORMULA          S100
       OF COMPOUND
              │
 FORM INPUT STRUCTURAL FORMULA INTO  S102
 THREE-DIMENSIONAL STRUCTURE FORMULA
              │
  CALCULATE SPATIAL DISTRIBUTION     S104
 ΔG_{aμ} (r) OF FREE ENERGY FELT BY EACH
 ATOM "μ" OF AMINO ACID "a" (r = (x, y, z))
              │
  CALCULATE DISTRIBUTION FUNCTION    S106
       g_{aμ} (r) OF EACH ATOM "μ"
 OF AMINO ACID "a" FROM ΔG_{aμ} (r)
              │
 CALCULATE DISTRIBUTION FUNCTION g_a (r)  S108
 OF CENTER OF GRAVITY OF AMINO ACID
   FROM DISTRIBUTION FUNCTION g_{aμ} (r)
              │
             END
```

**Description**

**BACKGROUND OF THE INVENTION**

1. Field of the Invention

The present invention relates to a method, a program, and a device which perform calculation of a feature quantity, screening of a compound, and creation of a three-dimensional structure of a compound and particularly relates to a technique for searching for a pharmaceutical candidate compound.

2. Description of the Related Art

In the related art, in the drug discovery research using a computer, pharmaceutical candidate compounds (hereinafter, referred to as "hits") have been searched for by preparing a library having tens of thousands to hundreds of thousands of existing compounds, providing the structural formulae of the compounds, and examining the binding force with respect to one target protein. For example, US9373059B predicts a binding force by providing the structural formula of a compound. Further, JP5946045B also describes that a compound having a desired binding force is searched for gradually by repeating generation of a structural formula and prediction of a binding force (trial and error).

Further, JP4564097B describes that a search is performed using a descriptor referred to as "compound fingerprint". The "descriptor" indicates information obtained from the structural formula of a compound, and the "compound fingerprint" indicates information related to the presence or absence of various functional groups. Such a descriptor has a characteristic in that "in a case where the descriptors of compounds are similar, the skeletons of the compounds are similar".

**SUMMARY OF THE INVENTION**

Recently, highly required target proteins have become complicated and difficult, it is difficult to find hits simply by screening libraries. Meanwhile, the theoretical number of compounds is (the 60th power of 10) even limiting the number to low molecules with a molecular weight of 500 or less. The number thereof is further increased in a case of widening the range to middle molecules with a molecular weight of approximately 1000, and thus there is still a possibility of finding hits, considering that the number of compounds synthesized since the dawn of history is approximately (the ninth power of 10). However, it is almost impossible to examine the binding force with respect to all the astronomical numbers of compounds by experiments or simulations. Even in a case of examination of the binding force with respect to some compounds, the efficiency is low only by repeating trial and error as described in US9373059B and JP5946045B. Further, in the case of a descriptor (feature quantity) in the related art such as the fingerprint described in JP4564097B, the feature quantities of compounds are not necessarily similar even in a case where the compounds exhibit the same drug efficacy. Further, since the feature quantities did not accurately show the chemical properties of the target structure, the efficiency of search using the feature quantities was low.

As described above, in the related art, feature quantities do not accurately show the chemical properties of the target structures, and thus the efficiency of screening using the feature quantity and creation of a three-dimensional structure is low.

The present invention has been made in consideration of the above-described circumstances, and an object thereof is to provide a feature quantity calculating method, a feature quantity calculating program, and a feature quantity calculating device which enable calculation of a feature quantity accurately showing chemical properties of a target structure. Further, another object of the present invention is to provide a screening method, a screening program, and a screening device which enable efficient screening of a pharmaceutical candidate compound using a feature quantity. Further, still another object of the present invention is to provide a compound creating method, a compound creating program, and a compound creating device which enable efficient creation of a three-dimensional structure of a pharmaceutical candidate compound using a feature quantity.

In order to achieve the above-described object, according to a first aspect of the present invention, there is provided a feature quantity calculating method comprising: a target structure designating step of designating a target structure formed of a plurality of unit structures having chemical properties; a three-dimensional structure generating step of generating a three-dimensional structure using the plurality of unit structures for the target structure; and a feature quantity calculating step of calculating a feature quantity obtained by quantifying, in a three-dimensional space, a degree of accumulation of one or more kinds of probes in a periphery of the three-dimensional structure, in which the probe is a structure in which a plurality of points having a real electric charge and generating a van der Waals force are disposed to be separated from each other.

Since the chemical properties of target structures are exhibited as the result of an interaction between the target structure and one or more kinds of probes in the periphery thereof, the fact that the degree of accumulation of the probes is similar between target structures indicates that the chemical properties of the target structures are similar. That is, target structures having similar feature quantities calculated according to the first aspect exhibit similar chemical properties. Therefore, according to the first aspect, the feature quantity accurately showing the chemical properties of a target structure can be calculated. In the first aspect and each of the following aspects, the probe may be "a structure in which a plurality of points having a real electric charge and generating a van der Waals force are disposed to be separated from each other at a certain distance".

In the feature quantity calculating method according to a second aspect, in the first aspect, a compound is designated as the target structure in the target structure designating step, a three-dimensional structure of the compound is generated with a plurality of atoms in the three-dimensional structure generating step, and a first feature quantity which is a feature quantity obtained by quantifying, in the three-dimensional space, a degree of accumulation of amino acids as the probes in the periphery of the three-dimensional structure of the compound generated in the three-dimensional structure generating step is calculated in the feature quantity calculating step. In the second aspect, the "probe", the "target structure", and the "plurality of unit structures" in the first aspect are respectively an amino acid, a compound, and a plurality of atoms. Further, the number of kinds of amino acids used for quantifying the degree of accumulation is not limited to one, and a peptide in which two or more kinds of amino acids are bound may be used.

Similar to the first aspect, since the drug efficacy of a compound (the binding force with respect to the target protein) is locally exhibited as the result of an interaction between the compound and each amino acid, in a case where the degree of accumulation of amino acids is similar between compounds, the compounds have similar binding forces with respect to the proteins. That is, the compounds with similar feature quantities according to the second aspect exhibit similar drug efficacies. Therefore, according to the second aspect, the feature quantity accurately showing the chemical properties of a compound can be calculated. In the second aspect, a compound such as a biological ligand which has a three-dimensional structure and whose binding to a target protein is known can be designated as a target structure.

The feature quantity calculating method according to a third aspect, in the second aspect, further comprises an invariant conversion step of converting the first feature quantity into an invariant with respect to rotation and translation of the compound to calculate a first invariant feature quantity. In the third aspect, since the first feature quantity is converted into an invariant with respect to rotation and translation of the compound, the feature quantity is easily handled and the data capacity can be reduced. The conversion of the first feature quantity into an invariant can be performed by Fourier transform, angular integration of a correlation function, or the like.

In the feature quantity calculating method according to a fourth aspect, in the third aspect, the first feature quantity of two different kinds of amino acids is calculated in the feature quantity calculating step, and the first invariant feature quantity is calculated using the first feature quantity of the two different kinds of amino acids in the invariant conversion step. According to the fourth aspect, since the conversion into an invariant can be performed while information related to the interaction between the amino acids is maintained using the first feature quantity of two different kinds of amino acids in the calculation of the first invariant feature quantity, the comparison of compounds (determination of the drug efficacy) can be accurately performed based on the feature quantity (the first invariant feature quantity).

In the feature quantity calculating method according to the fifth aspect, in the first aspect, a pocket structure bound to a pocket that is an active site of a target protein is designated as the target structure in the target structure designating step, a three-dimensional structure of the pocket structure is generated with a plurality of virtual spheres in the three-dimensional structure generating step, and a second feature quantity which is a feature quantity obtained by quantifying, in the three-dimensional space, a degree of accumulation of amino acids as the probes in the periphery of the three-dimensional structure of the pocket structure generated in the three-dimensional structure generating step is calculated in the feature quantity calculating step. In the fifth aspect, the "probe", the "target structure", and the "unit structure" in the first aspect are respectively an amino acid, a pocket structure, and a plurality of virtual spheres. The "active site" of the target protein indicates a site where the activity of the target protein is promoted or suppressed by binding a pocket structure, and the "virtual sphere" can be considered to have chemical properties such as the van der Waals radius and the electric charge.

The degree of accumulation of amino acids with respect to the provided compound is calculated in the second aspect described above, while the degree of accumulation of amino acids with respect to the pocket structure bound to a pocket of the provided target protein is calculated in the fifth aspect. Similar to the description of the second aspect, since the pocket structures having similar feature quantities according to the fourth aspect exhibit similar chemical properties, the feature quantity accurately showing the chemical properties of the pocket structure can be calculated according to the fifth aspect. Further, the pocket structure corresponds to a compound that is bound to a pocket of the target protein. In the fifth aspect, as the result of actual measurement on the three-dimensional structure of the target protein, simulation based on position information and the like of the pocket can be used for calculation of the second feature quantity. Further, the measuring techniques (for example, an X-ray crystal structure, an NMR structure (NMR: Nuclear Magnetic Resonance), and a cryo-TEM structure (TEM: Transmission Electron Microscopy)) are not limited as long as the three-dimensional structure of the target protein is a three-dimensional structure with a resolution that enables identification of each residue of an amino acid.

The feature quantity calculating method according to the sixth aspect, in the fifth aspect, further comprises an invariant conversion step of converting the second feature quantity into an invariant with respect to rotation and translation of the pocket structure to calculate a second invariant feature quantity. According to the sixth aspect, similarly to the third aspect, the feature quantity can be easily handled and the data capacity can be reduced. Similar to the third aspect, the conversion of the second feature quantity into an invariant can be performed by Fourier transform, angular integration of a correlation function, or the like.

In the feature quantity calculating method according to a seventh aspect, in the sixth aspect, the second feature quantity of two different kinds of amino acids is calculated in the feature quantity calculating step, and the second invariant feature quantity is calculated using the second feature quantity of the two different kinds of amino acids in the invariant conversion step. According to the seventh aspect, since the conversion into an invariant can be performed while information related to the interaction between the amino acids is maintained using the second feature quantity of two different kinds of amino acids in the calculation of the second invariant feature quantity, the comparison of compounds (determination of the drug efficacy) can be accurately performed based on the feature quantity (second invariant feature quantity).

In the feature quantity calculating method according to the eighth aspect, in the first aspect, a compound is designated as the target structure in the target structure designating step, a three-dimensional structure of the compound is generated with a plurality of atoms in the three-dimensional structure generating step, and a third feature quantity which is a feature quantity obtained by quantifying, in the three-dimensional space, a degree of accumulation of the probes in the periphery of the three-dimensional structure of the compound generated in the three-dimensional structure generating step which is the degree of accumulation using one or more selected from one or more kinds of nucleic acid bases, one or more kinds of lipid molecules, one or more kinds of monosaccharide molecules, water, and one or more kinds of ions, as the probes is calculated in the feature quantity calculating step. In the eighth aspect, the "probe", the "target structure", and the "plurality of unit structures" in the first aspect are respectively one or more kinds of nucleic acid bases (the kind, the number, and the combination thereof may be optional), a compound, and a plurality of atoms.

In the present invention, deoxyribonucleic acid (DNA), ribonucleic acid (RNA), cell membranes, and polysaccharides, which are biopolymers (compounds) other than proteins can be treated as the targets of drugs. In the eighth aspect, the method of calculating the feature quantities of these target compounds is defined, and the probe is not an amino acid but another substance (a building block of each target). Specifically, in a case where the targets are DNA, RNA, a cell membrane, and a polysaccharide, the probes are respectively one or more kinds of nucleic acid bases, one or more kinds of nucleic acid bases, one or more kinds of lipid molecules, and one or more kinds of monosaccharide molecules. Further, water and one or more kinds of ions may be considered during the quantification of the degree of accumulation using these as probes. Similar to the second and fifth aspects, since the drug efficacy of a compound (the binding force with respect to the target such as DNA) is locally exhibited as the result of an interaction between the compound and a nucleic acid base (probe) or the like, in a case where the degree of accumulation of nucleic acid bases or the like is similar between compounds, the compounds have similar binding forces with respect to the targets. That is, the compounds with similar feature quantities according to the eighth aspect exhibit similar drug efficacies. Therefore, according to the eighth aspect, the feature quantity accurately showing the chemical properties of a compound can be calculated.

The feature quantity calculating method according to the ninth aspect, in the eighth aspect, further comprises an invariant conversion step of converting the third feature quantity into an invariant with respect to rotation and translation of the compound to calculate a third invariant feature quantity. According to the ninth aspect, similar to the third and sixth aspects, the feature quantity is easily handled and the data capacity can be reduced. Similar to the third and sixth aspects, the conversion of the third feature quantity into an invariant can be performed by Fourier transform, angular integration of a correlation function, or the like.

In the feature quantity calculating method according to the tenth aspect, in the ninth aspect, the third feature quantity of a first probe that is formed of one or more selected from the one or more kinds of nucleic acid bases, the one or more kinds of lipid molecules, the one or more kinds of monosaccharide molecules, the water, and the one or more kinds of ions and a second probe that is formed of one or more selected from the one or more kinds of nucleic acid bases, the one or more kinds of lipid molecules, the one or more kinds of monosaccharide molecules, the water, and the one or more kinds of ions and different from the first probe is calculated in the feature quantity calculating step, and the third invariant feature quantity is calculated using the third feature quantity of the first probe and the third feature quantity of the second probe in the invariant conversion step. According to the tenth aspect, since the conversion into an invariant can be performed while information related to the interaction between the probes is maintained using the third feature quantity of two different kinds of probes (the first and second probes) in the calculation of the third invariant feature quantity, the comparison of compounds (determination of the drug efficacy) can be accurately performed based on the feature quantity (the third invariant feature quantity). In the tenth aspect, a case where at least one of the kind, the number, or the combination of constituent elements (one or more kinds of nucleic acid bases and the like) of the first and second probes varies corresponds to the case where "the second probe is different from the first probe".

In the feature quantity calculating method according to the eleventh aspect, in the first aspect, a compound is designated as the target structure in the target structure designating step, a three-dimensional structure of the compound is generated with a plurality of atoms in the three-dimensional structure generating step, and a fourth feature quantity which is a feature quantity obtained by quantifying, in the three-dimensional space, a degree of accumulation of the probes in the periphery of the three-dimensional structure of the compound generated in the three-dimensional structure generating step which is the degree of accumulation using a first probe that is one or more kinds of amino acids and a second probe that is one or more selected from one or more kinds of nucleic acid bases, one or more kinds of lipid molecules, water, one or more kinds of monosaccharide molecules, and one or more kinds of ions, as the probes is calculated in the feature quantity calculating step. In the eleventh aspect, the "probe", the "target structure", and the "plurality of unit structures" in the first aspect are respectively one or more kinds of amino acids (the first probe) and one or more kinds of nucleic acid bases (the second probe; the kind, the number, and the combination thereof may be optional), a compound, and a plurality of atoms.

Similar to the second, fifth, and eighth aspects, since the drug efficacy of a compound (the binding force with respect to the target such as a protein, DNA, or the like) is locally exhibited as the result of an interaction between the compound, an amino acid, and a nucleic acid base, in a case where the degree of accumulation of amino acids, nucleic acid bases, and the like is similar between compounds, the compounds have similar binding forces with respect to the target. That is, the compounds with similar feature quantities according to the eleventh aspect exhibit similar drug efficacies. Therefore, the feature quantity accurately showing the chemical properties of the target structure even in a case where a composite structure is used as a target can be calculated by employing a building block such as a protein, DNA, or the like as a probe.

The feature quantity calculating method according to the twelfth aspect, in the eleventh aspect, further comprises an invariant conversion step of converting the fourth feature quantity into an invariant with respect to rotation and translation of the compound to calculate a fourth invariant feature quantity. According to the twelfth aspect, similarly to the third, sixth, and ninth aspects, the feature quantity can be easily handled and the data capacity can be reduced. Similar to the third, sixth, and ninth aspects, the conversion of the fourth feature quantity into an invariant can be performed by Fourier transform, angular integration of a correlation function, or the like.

In the feature quantity calculating method according to a thirteenth aspect, in the twelfth aspect, the fourth feature quantity of two kinds of the probes in which at least one of the first probe or the second probe varies is calculated in the feature quantity calculating step, and the fourth invariant feature quantity is calculated using the fourth feature quantity of the two different kinds of the probes in the invariant conversion step. According to the thirteenth aspect, since the conversion into an invariant can be performed while information related to the interaction between the probes is maintained using the fourth feature quantity of two different kinds of probes in the calculation of the fourth invariant feature quantity, the comparison of compounds (determination of the drug efficacy) can be accurately performed based on the feature quantity (the fourth invariant feature quantity).

In the thirteenth aspect, "two kinds of probes in which at least one of the first probe or the second probe varies" are probes formed of the first probe and the second probe. Among two kinds of probes in which the combination of the first probe and the second probe varies, the above-described "two kinds of probes" include a case where "the first probes are the same as each other and the second probes are different from each other", for example, a case where one set of probes are formed of lysine (one kind of amino acid; an example of the first probe) and a nucleic acid base (an example of the second probe) and the other set of probes are formed of lysine (an example of the first probe) and a lipid molecule (another example of the second probe); a case where "the first probes are different from each other and the second probes are the same as each other", for example, a case where one set of probes are formed of lysine (an example of the first probe) and a nucleic acid base (an example of the second probe) and the other set of probes are formed of arginine (one kind of amino acid; another example of the first probe) and a nucleic acid base (an example of the second probe); and a case where "both the first probes and the second probes are different from each other", for example, a case where one set of probes are formed of lysine (an example of the first probe) and a nucleic acid base (an example of the second probe) and the other set of probes are formed of arginine (another example of the first probe) and a lipid molecule (another example of the second probe). Here, a case where at least one of the kind, the number, or the combination of constituent elements of the probes varies corresponds to the case where "one probe is different from the other probe".

In the feature quantity calculating method according to a fourteenth aspect, in the first aspect, a compound is designated as the target structure in the target structure designating step, a three-dimensional structure of the compound is generated with a plurality of atoms in the three-dimensional structure generating step, and a fifth feature quantity which is a feature quantity obtained by quantifying, in the three-dimensional space, the degree of accumulation of the probes in the periphery of the three-dimensional structure of the compound generated in the three-dimensional structure generating step which is the degree of accumulation using one or more (the kind, the number, and the combination may be optional) selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0, as the probes is calculated in the feature quantity calculating step. In the fourteenth aspect, the feature quantity calculating method for virtual probes is defined. However, similar to the second, fifth, eighth, and eleventh aspects, the compounds with similar feature quantities according to the fourteenth aspect exhibit similar drug efficacies. Therefore, even in a case where virtual probes are used, the feature quantity accurately showing the chemical properties of the target structure can be calculated.

The feature quantity calculating method according to a fifteenth aspect, in the fourteenth aspect, further comprises an invariant conversion step of converting the fifth feature quantity into an invariant with respect to rotation and translation of the compound to calculate a fifth invariant feature quantity. According to the fifteenth aspect, similarly to the third, sixth, ninth, and twelfth aspects, the feature quantity can be easily handled and the data capacity can be reduced. Similar to the third, sixth, ninth, and twelfth aspects, the conversion of the fifth feature quantity into an invariant can be performed by Fourier transform, angular integration of a correlation function, or the like.

In the feature quantity calculating method according to a sixteenth aspect, in the fifteenth aspect, the fifth feature quantity of a first probe that is formed of one or more (the kind, the number, and the combination may be optional) selected from the first point electric charge, the second point electric charge, the third point electric charge, the fourth point electric charge, the dipole and the fifth point electric charge, and a second probe that is formed of one or more (the kind, the number, and the combination may be optional) selected from the first point electric charge, the second point electric charge, the third point electric charge, the fourth point electric charge, the dipole and the fifth point electric charge, and different from the first probe is calculated in the feature quantity calculating step, and the fifth invariant feature quantity is calculated using the fifth feature quantity of the first probe and the fifth feature quantity of the second probe in the invariant conversion step. According to the sixteenth aspect, since the conversion into an invariant can be performed while information related to the interaction between the probes is maintained using the fifth feature quantity of two different kinds of probes (the first and second probes) in the calculation of the fifth invariant feature quantity, the comparison of compounds (determination of the drug efficacy) can be accurately performed based on the feature quantity (fifth invariant feature quantity). In the sixteenth aspect, a case where at least one of the kind, the number, or the combination of constituent elements (the first point electric charge and the like) of the first and second probes varies corresponds to the case where "the second probe is different from the first probe".

In the feature quantity calculating method according to a seventeenth aspect, in the first aspect, a compound is designated as the target structure in the target structure designating step, a three-dimensional structure of the compound is generated with a plurality of atoms in the three-dimensional structure generating step, and a sixth feature quantity which is a feature quantity obtained by quantifying, in the three-dimensional space, the degree of accumulation of the probes in the periphery of the three-dimensional structure of the compound generated in the three-dimensional structure generating step which is the degree of accumulation using a first probe that is one or more kinds of amino acids and a second probe that is one or more (the kind, the number, and the combination may be optional) selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0, as the probes is calculated in the feature quantity calculating step. In the seventeenth aspect, the feature quantity calculating method for a case where the first probe (one or more kinds of amino acids) and the second probe (the point electric charges and the combination thereof) are used as the probes is defined. However, similar to the second, fifth, eighth, eleventh, and fourteenth aspects, the compounds with similar feature quantities according to the seventeenth aspect exhibit similar drug efficacies. Therefore, even in a case where the first probe and the second probe are used, the feature quantity accurately showing the chemical properties of the target structure can be calculated.

The feature quantity calculating method according to an eighteenth aspect, in the seventeenth aspect, further comprises an invariant conversion step of converting the sixth feature quantity into an invariant with respect to rotation and translation of the compound to calculate a sixth invariant feature quantity. According to the eighteenth aspect, similarly to the third, sixth, ninth, twelfth, and fifteenth aspects, the feature quantity can be easily handled and the data capacity can be reduced. Similar to the third, sixth, ninth, twelfth, and fifteenth aspects, the conversion of the sixth feature quantity into an invariant can be performed by Fourier transform, angular integration of a correlation function, or the like.

In the feature quantity calculating method according to a nineteenth aspect, in the eighteenth aspect, the sixth feature quantity of two kinds of the probes in which at least one of the first probe or the second probe varies is calculated in the feature quantity calculating step, and the sixth invariant feature quantity is calculated using the sixth feature quantity of the two different kinds of the probes in the invariant conversion step. According to the nineteenth aspect, since the conversion into an invariant can be performed while information related to the interaction between the probes is maintained using the sixth feature quantity of two different kinds of probes in the calculation of the sixth invariant feature quantity, the comparison of compounds (determination of the drug efficacy) can be accurately performed based on the feature quantity (sixth invariant feature quantity). In the nineteenth aspect, "two kinds of probes in which at least one of the first probe or the second probe varies" are probes formed of the first probe and the second probe. Among two kinds of probes in which the combination of the first probe and the second probe varies, the above-described "two kinds of probes" include a case where "the first probes are the same as each other and the second probes are different from each other", for example, a case where one set of probes are formed of lysine (one kind of amino acid; an example of the first probe) and a first point electric charge (an example of the second probe) and the other set of probes are formed of lysine (an example of the first probe) and a second point electric charge (another example of the second probe); a case where "the first probes are different from each other and the second probes are the same as each other", for example, a case where one set of probes are formed of lysine (an example of the first probe) and a first point electric charge (an example of the second probe) and the other set of probes are formed of arginine (one kind of amino acid; another example of the first probe) and a first point electric charge (an example of the second probe); and a case where "both the first probes and the second probes are different from each other", for example, a case where one set of probes are formed of lysine (an example of the first probe) and a first point electric charge (an example of the second probe) and the other set of probes are formed of arginine (one kind of amino acid; another example of the first probe) and a second point electric charge (another example of the second probe). Here, a case where at least one of the kind, the number, or the combination of constituent elements of the probes varies corresponds to the case where "one probe is different from the other probe".

In the feature quantity calculating method according to the twentieth aspect, in the first aspect, a compound is designated as the target structure in the target structure designating step, a three-dimensional structure of the compound is generated with a plurality of atoms in the three-dimensional structure generating step, and a seventh feature quantity which is a feature quantity obtained by quantifying, in the three-dimensional space, the degree of accumulation of the probes in the periphery of the three-dimensional structure of the compound generated in the three-dimensional structure generating step which is the degree of accumulation using a first probe that is one or more (the kind, the number, and the combination may be optional) selected from one or more kinds of nucleic acid bases, one or more kinds of lipid molecules, water, one or more kinds of monosaccharide molecules, and one or more kinds of ions and a second probe that is one or more (the kind, the number, and the combination may be optional) selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0, as the probes is calculated in the feature quantity calculating step. In the twentieth aspect, the feature quantity calculating method for a case where the first probe (one or more kinds of nucleic acid bases and the combination thereof) and the second probe (the point electric charges and the combination thereof) are used as the probes is defined. However, similar to the second, fifth, eighth, eleventh, fourteenth, and seventeenth aspects, the compounds with similar feature quantities according to the twentieth aspect exhibit similar drug efficacies. Therefore, even in a case where the first probe and the second probe are used, the feature quantity accurately showing the chemical properties of the target structure can be calculated.

The feature quantity calculating method according to the twenty-first aspect, in the twentieth aspect, further comprises an invariant conversion step of converting the seventh feature quantity into an invariant with respect to rotation and translation of the compound to calculate a seventh invariant feature quantity. According to the twenty-first aspect, similarly to the third, sixth, ninth, twelfth, fifteenth, and eighteenth aspects, the feature quantity can be easily handled and the data capacity can be reduced. Similar to the third, sixth, ninth, twelfth, fifteenth, and eighteenth aspects, the conversion of the seventh feature quantity into an invariant can be performed by Fourier transform, angular integration of a correlation function, or the like.

In the feature quantity calculating method according to the twenty-second aspect, in the twenty-first aspect, the seventh feature quantity of two kinds of the probes in which at least one of the first probe or the second probe varies is calculated in the feature quantity calculating step, and the seventh invariant feature quantity is calculated using the seventh feature quantity of the two different kinds of the probes in the invariant conversion step. According to the twenty-second aspect, since the conversion into an invariant can be performed while information related to the interaction between the probes is maintained using the seventh feature quantity of two different kinds of probes in the calculation of the seventh invariant feature quantity, the comparison of compounds (determination of the drug efficacy) can be accurately performed based on the feature quantity (seventh invariant feature quantity). In the twenty-second aspect, "two kinds of probes in which at least one of the first probe or the second probe varies" are probes formed of the first probe and the second probe. Among two kinds of probes in which the combination of the first probe and the second probe varies, the above-described "two kinds of probes" include a case where "the first probes are the same as each other and the second probes are different from each other", for example, a case where one set of probes are formed of a nucleic acid base (an example of the first probe) and a first point electric charge (an example of the second probe) and the other set of probes are formed of a nucleic acid base (an example of the first probe) and a second point electric charge (another example of the second probe); a case where "the first probes are different from each other and the second probes are the same as each other", for example, a case where one set of probes are formed of a nucleic acid base (an example of the first probe) and a first point electric charge (an example of the second probe) and the other set of probes are formed of a lipid molecule (another example of the first probe) and a first point electric charge (an example of the second probe); and a case where "both the first probes and the second probes are different from each other", for example, a case where one set of probes are formed of a nucleic acid base (an example of the first probe) and a first point electric charge (an example of the second probe) and the other set of probes are formed of a lipid molecule (another example of the first probe) and a second point electric charge (another example of the second probe). Here, a case where at least one of the kind, the number, or the combination of constituent elements of the probes varies corresponds to the case where "one probe is different from the other probe".

In the feature quantity calculating method according to a twenty-third aspect, in the first aspect, a compound is designated as the target structure in the target structure designating step, a three-dimensional structure of the compound is generated with a plurality of atoms in the three-dimensional structure generating step, and an eighth feature quantity which is a feature quantity obtained by quantifying, in the three-dimensional space, the degree of accumulation of the probes in the periphery of the three-dimensional structure of the compound generated in the three-dimensional structure generating step which is the degree of accumulation using a first probe that is one or more kinds of amino acids, a second probe that is one or more (the kind, the number, and the combination may be optional) selected from one or more kinds of nucleic acid bases, one or more kinds of lipid molecules, water, one or more kinds of monosaccharide molecules, and one or more kinds of ions, and a third probe that is one or more (the kind, the number, and the combination may be optional) selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0, as the probes is calculated in the feature quantity calculating step. In the twenty-third aspect, the feature quantity calculating method for a case where the first probe (one or more kinds of amino acids), the second probe (one or more kinds of nucleic acid bases and the combination thereof), and the third probe (point electric charges and the combination thereof) are used as the probes is defined. However, similar to the second, fifth, eighth, eleventh, fourteenth, seventeenth, and twentieth aspects, the compounds with similar feature quantities according to the twenty-third aspect exhibit similar drug efficacies. Therefore, even in a case where the first to third probes are used, the feature quantity accurately showing the chemical properties of the target structure can be calculated.

The feature quantity calculating method according to a twenty-fourth aspect, in the twenty-third aspect, further comprises an invariant conversion step of converting the eighth feature quantity into an invariant with respect to rotation and translation of the compound to calculate an eighth invariant feature quantity. According to the twenty-fourth aspect, similarly to the third, sixth, ninth, twelfth, fifteenth, eighteenth, and twenty-first aspects, the feature quantity can be easily handled and the data capacity can be reduced. Similar to the third, sixth, ninth, twelfth, fifteenth, eighteenth, and twenty-first aspects, the conversion of the eighth feature quantity into an invariant can be performed by Fourier transform, angular integration of a correlation function, or the like.

In the feature quantity calculating method according to a twenty-fifth aspect, in the twenty-fourth aspect, the eighth feature quantity of two kinds of the probes in which at least one of the first probe, the second probe, or the third probe varies is calculated in the feature quantity calculating step, and the eighth invariant feature quantity is calculated using the eighth feature quantity of the two different kinds of the probes in the invariant conversion step. According to the twenty-fifth aspect, since the conversion into an invariant can be performed while information related to the interaction between the probes is maintained using the eighth feature quantity of two different kinds of probes in the calculation of the eighth invariant feature quantity, the comparison of compounds (determination of the drug efficacy) can be accurately performed based on the feature quantity (eighth invariant feature quantity). In the twenty-fifth aspect, "two kinds of probes in which at least one of the first probe, the second probe, or the third probe varies" are probes formed of the first probe, the second probe, and the third probe. Among two different kinds of probes in which the combination of the first probe, the second probe, and the third probe varies, the above-described "two kinds of probes" include a case where "one of the first, second, and third probes is different", a case where "two of the first, second, and third probes are different", and a case where "all the first, second, and third probes are different" between one set of probes and the other set of probes. Here, a case where at least one of the kind, the number, or the combination of constituent elements of the probes varies corresponds to the case where "one probe is different from the other probe".

In order to achieve the above-described object, according to a twenty-sixth aspect of the present invention, there is provided a feature quantity calculating program which causes a computer to execute the feature quantity calculating method according to any one of the first to twenty-fifth aspects. The "computer" in the twenty-sixth aspect can be realized using one or more of various processors such as a Central Processing Unit (CPU). Further, a non-temporary recording medium on which a computer-readable code of the feature quantity calculating program according to the twenty-sixth aspect is recorded can also be exemplified as an aspect of the present invention.

In order to achieve the above-described object, according to a twenty-seventh aspect of the present invention, there is provided a feature quantity calculating device comprising: a target structure designation unit which designates a target structure formed of a plurality of unit structures having chemical properties; a three-dimensional structure generation unit which generates a three-dimensional structure using the plurality of unit structures for the target structure; and a feature quantity calculation unit which calculates a feature quantity obtained by quantifying, in a three-dimensional space, a degree of accumulation of one or more kinds of probes in a periphery of the three-dimensional structure, in which the probe is a structure in which a plurality of points having a real electric charge and generating a van der Waals force are disposed to be separated from each other. According to the twenty-seventh aspect, similarly to the first aspect, the feature quantity accurately showing the chemical properties of the target structure can be calculated. In the twenty-seventh aspect, as the description for the first to twenty-sixth aspects, the first to fifth feature quantities can be calculated using a protein, DNA, and the like as the target compounds and an amino acid, a nucleic acid base, a virtual electric charge, and the like as the probes. In the twenty-seventh aspect, the probe may be "a structure in which a plurality of points having a real electric charge and generating a van der Waals force are disposed to be separated from each other at a certain distance".

In order to achieve the above-described object, according to a twenty-eighth aspect of the present invention, there is provided a screening method of extracting a target compound which is bound to a target protein from a plurality of compounds, the method comprising: a storing step of storing a three-dimensional structure of a compound formed of a plurality of atoms and the first feature quantity of the three-dimensional structure of the compound calculated using the feature quantity calculating method according to the second aspect in association with each other for each of the plurality of compounds; a feature quantity calculating step of calculating the first feature quantity of a ligand that is a compound whose binding to the target protein has been confirmed; a similarity calculating step of calculating a similarity between the first feature quantity of the plurality of compounds and the first feature quantity of the ligand; and a compound extracting step of extracting the target compound from the plurality of compounds based on the similarity. As the description for the second aspect, in a case where the ligand and the target compound have similar first feature quantities (the feature quantities three-dimensionally quantified), the drug efficacies of both the ligand and the target compound are similar. Therefore, according to the twenty-eighth aspect, a target compound having drug efficacy similar to that of the ligand can be extracted based on the first feature quantity so that screening of a pharmaceutical candidate compound can be efficiently performed.

In order to achieve the above-described object, according to a twenty-ninth aspect of the present invention, there is provided a screening method of extracting a target compound which is bound to a target protein from a plurality of compounds, the method comprising: a storing step of storing a three-dimensional structure of a compound formed of a plurality of atoms and the first invariant feature quantity of the three-dimensional structure of the compound in association with each other for each of the plurality of compounds; a feature quantity calculating step of calculating the first invariant feature quantity of a ligand that is a compound whose binding to the target protein has been confirmed using the feature quantity calculating method according to the third aspect; a similarity calculating step of calculating a similarity between the first invariant feature quantity of the plurality of compounds and the first invariant feature quantity of the ligand; and a compound extracting step of extracting the target compound from the plurality of compounds based on the similarity. The twenty-ninth aspect is common to the twenty-eighth aspect in that the feature quantity of the ligand is calculated. However, in the twenty-ninth aspect, a target compound having drug efficacy similar to that of the ligand can be extracted based on the similarity of the first invariant feature quantity so that screening of a pharmaceutical candidate compound can be efficiently performed.

In the screening method according to a thirtieth aspect, in the twenty-eighth or twenty-ninth aspect, a compound having a similarity greater than or equal to a threshold is extracted in the compound extracting step. In the thirtieth aspect, specific standards for extracting the target compound based on the similarity are defined. By extracting a compound having a similarity greater than or equal to a threshold, screening of the pharmaceutical candidate compound can be efficiently performed. The threshold can be set based on conditions, for example, the purpose and the accuracy of the screening and may be set based on a value designated by the user.

In the screening method according to the thirty-first aspect, in any one of the twenty-eighth to thirtieth aspects, compounds are extracted in a descending order of the similarity in the compound extracting step. In the thirty-first aspect, specific standards for extracting the target compound based on the similarity are defined. By extracting compounds in a descending order of the similarity, screening of the pharmaceutical candidate compound can be efficiently performed.

In order to achieve the above-described object, according to a thirty-second aspect of the present invention, there is provided a screening method of extracting a target compound which is bound to a target protein from a plurality of compounds, the method comprising: a storing step of storing a three-dimensional structure of a compound formed of a plurality of atoms and the first feature quantity calculated using the feature quantity calculating method according to the second aspect in association with each other for each of the plurality of compounds; a feature quantity calculating step of calculating the second feature quantity of the pocket structure of the target protein using the feature quantity calculating method according to the fifth aspect; a similarity calculating step of calculating a similarity between the first feature quantity of the plurality of compounds and the second feature quantity of the pocket structure; and a compound extracting step of extracting the target compound from the plurality of compounds based on the similarity. As the description for the fifth aspect, in a case where the pocket structure and the target compound have similar second feature quantities, the chemical properties of both the pocket structure and the target compound are similar. Therefore, according to the thirty-second aspect, a target compound having chemical properties similar to those of the pocket structure is extracted so that screening of a pharmaceutical candidate compound can be efficiently performed. Since the pocket structure corresponds to the compound that is bound to the target protein, the feature quantity (the second feature quantity) of the pocket structure can be compared with the feature quantity (the first feature quantity) of the compound, and the similarity can be calculated.

In order to achieve the above-described object, according to a thirty-third aspect of the present invention, there is provided a screening method of extracting a target compound which is bound to a target protein from a plurality of compounds, the method comprising: a storing step of storing a three-dimensional structure of a compound formed of a plurality of atoms and the first invariant feature quantity calculated using the feature quantity calculating method according to the third aspect in association with each other for each of the plurality of compounds; a feature quantity calculating step of calculating the second invariant feature quantity of the pocket structure of the target protein using the feature quantity calculating method according to the sixth aspect; a similarity calculating step of calculating a similarity between the first invariant feature quantity of the plurality of compounds and the second invariant feature quantity of the pocket structure; and a compound extracting step of extracting the target compound from the plurality of compounds based on the similarity. In the thirty-third aspect, a target compound having chemical properties similar to those of the pocket structure is extracted using the first and second invariant feature quantities so that screening of a pharmaceutical candidate compound can be efficiently performed. As the description for the thirty-second aspect, the feature quantity (the second invariant feature quantity) of the pocket structure can be compared with the feature quantity (the first invariant feature quantity) of the compound, and the similarity can be calculated.

In the screening method according to a thirty-fourth aspect, in the thirty-second or thirty-third aspect, a compound having a similarity greater than or equal to a threshold is extracted in the compound extracting step. In the thirty-fourth aspect, specific standards for extracting the target compound based on the similarity are defined. By extracting a compound having a similarity greater than or equal to the threshold, screening of the pharmaceutical candidate compound can be efficiently performed. The threshold can be set based on conditions, for example, the purpose and the accuracy of the screening and may be set based on a value designated by the user.

In the screening method according to the thirty-fifth aspect, in any one of the thirty-second to thirty-fourth aspects, compounds are extracted in a descending order of the similarity in the compound extracting step. In the thirty-fifth aspect, specific standards for extracting the target compound based on the similarity are defined. By extracting compounds in a descending order of the similarity, screening of the pharmaceutical candidate compound can be efficiently performed.

In order to achieve the above-described object, according to a thirty-sixth aspect of the present invention, there is provided a screening method of extracting a target compound which is bound to a target biopolymer other than a protein from a plurality of compounds, the method comprising: a storing step of storing a three-dimensional structure of a compound formed of a plurality of atoms and the third feature quantity of the three-dimensional structure of the compound calculated using the feature quantity calculating method according to the eighth aspect in association with each other for each of the plurality of compounds; a feature quantity calculating step of calculating the third feature quantity of a binding compound that is a compound whose binding to the target biopolymer other than the protein has been confirmed; a similarity calculating step of calculating a similarity between the third feature quantity of the plurality of compounds and the third feature quantity of the binding compound; and a compound extracting step of extracting the target compound from the plurality of compounds based on the similarity. As the description for the eighth aspect, according to the present invention, DNA or the like, which is a target biopolymer other than a protein, can be handled, and in a case where the target compound and the binding compound that is bound to the target biopolymer have similar third feature quantities, the drug efficacies of both the target compound and the binding compound are similar. Therefore, according to the thirty-sixth aspect, a target compound having drug efficacy similar to that of the binding compound is extracted based on the third feature quantity so that screening of a pharmaceutical candidate compound can be efficiently performed.

In order to achieve the above-described object, according to a thirty-seventh aspect of the present invention, there is provided a screening method of extracting a target compound which is bound to a target biopolymer from a plurality of compounds, the method comprising: a storing step of storing a three-dimensional structure of a compound formed of a plurality of atoms and the fourth feature quantity of the three-dimensional structure of the compound calculated using the feature quantity calculating method according to the eleventh aspect in association with each other for each of the plurality of compounds; a feature quantity calculating step of calculating the fourth feature quantity of a binding compound that is a compound whose binding to the target biopolymer has been confirmed; a similarity calculating step of calculating a similarity between the fourth feature quantity of the plurality of compounds and the fourth feature quantity of the binding compound; and a compound extracting step of extracting the target compound from the plurality of compounds based on the similarity. As the description for the eleventh aspect, compounds having similar fourth feature quantities exhibit similar drug efficacies. Therefore, according to the thirty-seventh aspect, even in a case where a composite structure is used as a target, a target compound having drug efficacy similar to that of the binding compound is extracted based on the fourth feature quantity so that screening of a pharmaceutical candidate compound can be efficiently performed.

In order to achieve the above-described object, according to a thirty-eighth aspect of the present invention, there is provided a screening method of extracting a target compound which is bound to a target biopolymer from a plurality of compounds, the method comprising: a storing step of storing a three-dimensional structure of a compound formed of a plurality of atoms and the fifth feature quantity of the three-dimensional structure of the compound calculated using the feature quantity calculating method according to the fourteenth aspect in association with each other for each of the plurality of compounds; a feature quantity calculating step of calculating the fifth feature quantity of a binding compound that is a compound whose binding to the target biopolymer has been confirmed; a similarity calculating step of calculating a similarity between the fifth feature quantity of the plurality of compounds and the fifth feature quantity of the binding compound; and a compound extracting step of extracting the target compound from the plurality of compounds based on the similarity. As the description for the fourteenth aspect, compounds having similar fifth feature quantities exhibit similar drug efficacies. Therefore, according to the thirty-eighth aspect, even in a case where a virtual probe is used, a target compound having drug efficacy similar to that of the binding compound is extracted based on the fifth feature quantity so that screening of a pharmaceutical candidate compound can be efficiently performed.

In order to achieve the above-described object, according to a thirty-ninth aspect of the present invention, there is provided a screening method of extracting a target compound which is bound to a target biopolymer from a plurality of compounds, the method comprising: a storing step of storing a three-dimensional structure of a compound formed of a plurality of atoms and the sixth feature quantity of the three-dimensional structure of the compound calculated using the feature quantity calculating method according to the seventeenth aspect in association with each other for each of the plurality of compounds; a feature quantity calculating step of calculating the sixth feature quantity of a binding compound that is a compound whose binding to the target biopolymer has been confirmed; a similarity calculating step of calculating a similarity between the sixth feature quantity of the plurality of compounds and the sixth feature quantity of the binding compound; and a compound extracting step of extracting the target compound from the plurality of compounds based on the similarity. As the description for the seventeenth aspect, compounds having similar sixth feature quantities exhibit similar drug efficacies. Therefore, according to the thirty-ninth aspect, even in a case where one or more kinds of amino acids (the first probe) and a virtual electric charge and the like (the second probe) are used, a target compound having drug efficacy similar to that of the binding compound is extracted based on the sixth feature quantity so that screening of a pharmaceutical candidate compound can be efficiently performed.

In order to achieve the above-described object, according to a fortieth aspect of the present invention, there is provided a screening method of extracting a target compound which is bound to a target biopolymer from a plurality of compounds, the method comprising: a storing step of storing a three-dimensional structure of a compound formed of a plurality of atoms and the seventh feature quantity of the three-dimensional structure of the compound calculated using the feature quantity calculating method according to the twentieth aspect in association with each other for each of the plurality of compounds; a feature quantity calculating step of calculating the seventh feature quantity of a binding compound that is a compound whose binding to the target biopolymer has been confirmed; a similarity calculating step of calculating a similarity between the seventh feature quantity of the plurality of compounds and the seventh feature quantity of the binding compound; and a compound extracting step of extracting the target compound from the plurality of compounds based on the similarity. As the description for the twentieth aspect, compounds having similar seventh feature quantities exhibit similar drug efficacies. Therefore, according to the fortieth aspect, even in a case where one or more kinds of nucleic acid bases (the first probe) and a virtual electric charge and the like (the second probe) are used, a target compound having drug efficacy similar to that of the binding compound is extracted based on the seventh feature quantity so that screening of a pharmaceutical candidate compound can be efficiently performed.

In order to achieve the above-described object, according to a forty-first aspect of the present invention, there is provided a screening method of extracting a target compound which is bound to a target biopolymer from a plurality of compounds, the method comprising: a storing step of storing a three-dimensional structure of a compound formed of a plurality of atoms and the eighth feature quantity of the three-dimensional structure of the compound calculated using the feature quantity calculating method according to twenty-third aspect in association with each other for each of the plurality of compounds; a feature quantity calculating step of calculating the eighth feature quantity of a binding compound that is a compound whose binding to the target biopolymer has been confirmed; a similarity calculating step of calculating a similarity between the eighth feature quantity of the plurality of compounds and the eighth feature quantity of the binding compound; and a compound extracting step of extracting the target compound from the plurality of compounds based on the similarity. As the description for the twenty-third aspect, compounds having similar eighth feature quantities exhibit similar drug efficacies. Therefore, according to the forty-first aspect, even in a case where one or more kinds of amino acids (the first probe), one or more kinds of nucleic acid bases and the like (the second probe), and a virtual electric charge and the like (the third probe) are used, a target compound having drug efficacy similar to that of the binding compound is extracted based on the eighth feature quantity so that screening of a pharmaceutical candidate compound can be efficiently performed.

In order to achieve the above-described object, according to a forty-second aspect of the present invention, there is provided a screening program which causes a computer to execute the screening method according to any one of the twenty-eighth to forty-first aspects. The "computer" in the forty-second aspect can be realized using one or more of various processors such as a Central Processing Unit (CPU). Further, a non-temporary recording medium on which a computer-readable code of the screening program according to the forty-second aspect is recorded can also be exemplified as an aspect of the present invention.

In order to achieve the above-described object, according to a forty-third aspect of the present invention, there is provided a screening device which extracts a target compound that is bound to a target protein from a plurality of compounds, the device comprising: a storage unit which stores a three-dimensional structure of a compound formed of a plurality of atoms and the first feature quantity of the three-dimensional structure of the compound calculated using the feature quantity calculating method according to the second aspect in association with each other for each of the plurality of compounds; a feature quantity calculation unit which calculates the first feature quantity of a ligand that is a compound whose binding to the target protein has been confirmed; a similarity calculation unit which calculates a similarity between the first feature quantity of the plurality of compounds and the first feature quantity of the ligand; and a compound extraction unit which extracts the target compound from the plurality of compounds based on the similarity.

As the description for the second aspect, in a case where the ligand and the target compound have similar first feature quantities (the feature quantities three-dimensionally quantified), the drug efficacies of both the ligand and the target compound are similar. Therefore, according to the forty-third aspect, a target compound having drug efficacy similar to that of the ligand can be extracted based on the first feature quantity so that screening of a pharmaceutical candidate compound can be efficiently performed.

In order to achieve the above-described object, according to a forty-fourth aspect of the present invention, there is provided a screening device which extracts a target compound that is bound to a target protein from a plurality of compounds, the device comprising: a storage unit which stores a three-dimensional structure of a compound formed of a plurality of atoms and the first invariant feature quantity of the three-dimensional structure of the compound in association with each other for each of the plurality of compounds; a feature quantity calculation unit which calculates the first invariant feature quantity of a ligand that is a compound whose binding to the target protein has been confirmed using the feature quantity calculating method according to the third aspect; a similarity calculation unit which calculates a similarity between the first invariant feature quantity of the plurality of compounds and the first invariant feature quantity of the ligand; and a compound extraction unit which extracts the target compound from the plurality of compounds based on the similarity.

The forty-fourth aspect is common to the forty-third aspect in that the feature quantity of the ligand is calculated. However, in the forty-fourth aspect, a target compound having drug efficacy similar to that of the ligand can be extracted based on the similarity of the first invariant feature quantity so that screening of a pharmaceutical candidate compound can be efficiently performed.

In the screening device according to a forty-fifth aspect, in the forty-third or forty-fourth aspect, the compound extraction unit extracts a compound having a similarity greater than or equal to a threshold. In the forty-fifth aspect, specific standards for extracting the target compound based on the similarity are defined. By extracting a compound having a similarity greater than or equal to a threshold, screening of the pharmaceutical candidate compound can be efficiently performed. The threshold can be set based on conditions, for example, the purpose and the accuracy of the screening and may be set based on a value designated by the user.

In the screening device according to a forty-sixth aspect, in any one of the forty-third to forty-fifth aspects, the compound extraction unit extracts a compound in a descending order of the similarity. In the forty-sixth aspect, specific standards for extracting the target compound based on the similarity are defined. By extracting compounds in a descending order of the similarity, screening of the pharmaceutical candidate compound can be efficiently performed.

In order to achieve the above-described object, according to a forty-seventh aspect of the present invention, there is provided a screening device which extracts a target compound that is bound to a target protein from a plurality of compounds, the device comprising: a storage unit which stores a three-dimensional structure of a compound formed of a plurality of atoms and the first feature quantity calculated using the feature quantity calculating method according to the second aspect in association with each other for each of the plurality of compounds; a feature quantity calculation unit which calculates the second feature quantity of the pocket structure of the target protein using the feature quantity calculating method according to the fifth aspect; a similarity calculation unit which calculates a similarity between the first feature quantity of the plurality of compounds and the second feature quantity of the pocket structure; and a compound extraction unit which extracts the target compound from the plurality of compounds based on the similarity.

As the description for the fifth aspect, in a case where the pocket structure and the target compound have similar second feature quantities, the chemical properties of both the pocket structure and the target compound are similar. Therefore, according to the forty-seventh aspect, a target compound having chemical properties similar to those of the pocket structure is extracted so that screening of a pharmaceutical candidate compound can be efficiently performed. Since the pocket structure corresponds to the compound that is bound to the target protein, the feature quantity (the second feature quantity) of the pocket structure can be compared with the feature quantity (the first feature quantity) of the compound, and the similarity can be calculated.

In order to achieve the above-described object, according to a forty-eighth aspect of the present invention, there is provided a screening device which extracts a target compound that is bound to a target protein from a plurality of compounds, the device comprising: a storage unit which stores a three-dimensional structure of a compound formed of a plurality of atoms and the first invariant feature quantity calculated using the feature quantity calculating method according to the third aspect in association with each other for each of the plurality of compounds; a feature quantity calculation unit which calculates the second invariant feature quantity of the pocket structure of the target protein using the feature quantity calculating method according to the sixth aspect; a similarity calculation unit which calculates a similarity between the first invariant feature quantity of the plurality of compounds and the second invariant feature quantity of the pocket structure; and a compound extraction unit which extracts the target compound from the plurality of compounds based on the similarity.

In the forty-eighth aspect, a target compound having chemical properties similar to those of the pocket structure is extracted using the first and second invariant feature quantities so that screening of a pharmaceutical candidate compound can be efficiently performed. As the description for the forty-seventh aspect, the feature quantity (the second invariant feature quantity) of the pocket structure can be compared with the feature quantity (the first invariant feature quantity) of the compound, and the similarity can be calculated.

In the screening device according to the forty-ninth aspect, in the forty-seventh or forty-eighth aspect, the compound extraction unit extracts a compound having a similarity greater than or equal to a threshold. In the forty-ninth aspect, specific standards for extracting the target compound based on the similarity are defined. By extracting a compound having a similarity greater than or equal to the threshold, screening of the pharmaceutical candidate compound can be efficiently performed. The threshold can be set based on conditions, for example, the purpose and the accuracy of the screening and may be set based on a value designated by the user.

In the screening device according to the fiftieth aspect, in any one of the forty-seventh to forty-ninth aspects, the compound extraction unit extracts a compound in a descending order of the similarity. In the fiftieth aspect, specific standards for extracting the target compound based on the similarity are defined. By extracting compounds in a descending order of the similarity, screening of the pharmaceutical candidate compound can be efficiently performed.

In order to achieve the above-described object, according to a fifty-first aspect of the present invention, there is provided a screening device which extracts a target compound that is bound to a target biopolymer other than a protein from a plurality of compounds, the device comprising: a storage unit which stores a three-dimensional structure of a compound formed of a plurality of atoms and the third feature quantity of the three-dimensional structure of the compound calculated using the feature quantity calculating method according to the eighth aspect in association with each other for each of the plurality of compounds; a feature quantity calculation unit which calculates the third feature quantity of a binding compound that is a compound whose binding to the target biopolymer other than the protein has been confirmed; a similarity calculation unit which calculates a similarity between the third feature quantity of the plurality of compounds and the third feature quantity of the binding compound; and a compound extraction unit which extracts the target compound from the plurality of compounds based on the similarity.

As the description for the eighth aspect, in a case where the target compound and the target biopolymer other than the protein have similar third feature quantities, the drug efficacies of both the target compound and the target biopolymer are similar. Therefore, according to the fifty-first aspect, a target compound having drug efficacy similar to that of the target biopolymer other than the protein is extracted based on the third feature quantity so that screening of a pharmaceutical candidate compound can be efficiently performed.

In order to achieve the above-described object, according to a fifty-second aspect of the present invention, there is provided a screening device which extracts a target compound that is bound to a target biopolymer from a plurality of compounds, the device comprising: a storage unit which stores a three-dimensional structure of a compound formed of a plurality of atoms and the fourth feature quantity of the three-dimensional structure of the compound calculated using the feature quantity calculating method according to the eleventh aspect in association with each other for each of the plurality of compounds; a feature quantity calculation unit which calculates the fourth feature quantity of a binding compound that is a compound whose binding to the target biopolymer has been confirmed; a similarity calculation unit which calculates a similarity between the fourth feature quantity of the plurality of compounds and the fourth feature quantity of the binding compound; and a compound extraction unit which extracts the target compound from the plurality of compounds based on the similarity.

As the description for the eleventh aspect, compounds having similar fourth feature quantities exhibit similar drug efficacies. Therefore, according to the fifty-second aspect, even in a case where a composite structure is used as a target, a target compound having drug efficacy similar to that of the binding compound is extracted based on the fourth feature quantity so that screening of a pharmaceutical candidate compound can be efficiently performed.

In order to achieve the above-described object, according to a fifty-third aspect of the present invention, there is provided a screening device which extracts a target compound that is bound to a target biopolymer from a plurality of compounds, the device comprising: a storage unit which stores a three-dimensional structure of a compound formed of a plurality of atoms and the fifth feature quantity of the three-dimensional structure of the compound calculated using the feature quantity calculating method according to the fourteenth aspect in association with each other for each of the plurality of compounds; a feature quantity calculation unit which calculates the fifth feature quantity of a binding compound that is a compound whose binding to the target biopolymer has been confirmed; a similarity calculation unit which calculates a similarity between the fifth feature quantity of the plurality of compounds and the fifth feature quantity of the binding compound; and a compound extraction unit which extracts the target compound from the plurality of compounds based on the similarity.

As the description for the fourteenth aspect, compounds having similar fifth feature quantities exhibit similar drug efficacies. Therefore, according to the fifty-third aspect, even in a case where a virtual probe is used, a target compound having drug efficacy similar to that of the binding compound is extracted based on the fifth feature quantity so that screening of a pharmaceutical candidate compound can be efficiently performed.

In order to achieve the above-described object, according to a fifty-fourth aspect of the present invention, there is provided a screening device which extracts a target compound that is bound to a target biopolymer from a plurality of compounds, the device comprising: a storage unit which stores a three-dimensional structure of a compound formed of a plurality of atoms and the sixth feature quantity of the three-dimensional structure of the compound calculated using the feature quantity calculating method according to the seventeenth aspect in association with each other for each of the plurality of compounds; a feature quantity calculation unit which calculates the sixth feature quantity of a binding compound that is a compound whose binding to the target biopolymer has been confirmed; a similarity calculation unit which calculates a similarity between the sixth feature quantity of the plurality of compounds and the sixth feature quantity of the binding compound; and a compound extraction unit which extracts the target compound from the plurality of compounds based on the similarity.

As the description for the seventeenth aspect, compounds having similar sixth feature quantities exhibit similar drug efficacies. Therefore, according to the fifty-fourth aspect, even in a case where the first probe that is one or more kinds of amino acids and the second probe that is a virtual point electric charge and the like and the combination thereof are used, a target compound having drug efficacy similar to that of the binding compound is extracted based on the sixth feature quantity so that screening of a pharmaceutical candidate compound can be efficiently performed.

In order to achieve the above-described object, according to a fifty-fifth aspect of the present invention, there is provided a screening device which extracts a target compound that is bound to a target biopolymer from a plurality of compounds, the device comprising: a storage unit which stores a three-dimensional structure of a compound formed of a plurality of atoms and the seventh feature quantity of the three-dimensional structure of the compound calculated using the feature quantity calculating method according to the twentieth aspect in association with each other for each of the plurality of compounds; a feature quantity calculation unit which calculates the seventh feature quantity of a binding compound that is a compound whose binding to the target biopolymer has been confirmed; a similarity calculation unit which calculates a similarity between the seventh feature quantity of the plurality of compounds and the seventh feature quantity of the binding compound; and a compound extraction unit which extracts the target compound from the plurality of compounds based on the similarity.

As the description for the twentieth aspect, compounds having similar seventh feature quantities exhibit similar drug efficacies. Therefore, according to the fifty-fifth aspect, even in a case where the first probe (one or more kinds of nucleic acid bases and the like and the combination thereof) and the second probe that is a virtual point electric charge and the like are used, a target compound having drug efficacy similar to that of the binding compound is extracted based on the seventh feature quantity so that screening of a pharmaceutical candidate compound can be efficiently performed.

In order to achieve the above-described object, according to a fifty-sixth aspect of the present invention, there is provided a screening device which extracts a target compound that is bound to a target biopolymer from a plurality of compounds, the device comprising: a storage unit which stores a three-dimensional structure of a compound formed of a plurality of atoms and the eighth feature quantity of the three-dimensional structure of the compound calculated using the feature quantity calculating method according to the twenty-third aspect in association with each other for each of the plurality of compounds; a feature quantity calculation unit which calculates the eighth feature quantity of a binding compound that is a compound whose binding to the target biopolymer has been confirmed; a similarity calculation unit which calculates a similarity between the eighth feature quantity of the plurality of compounds and the eighth feature quantity of the binding compound; and a compound extraction unit which extracts the target compound from the plurality of compounds based on the similarity.

As the description for the twenty-third aspect, compounds having similar eighth feature quantities exhibit similar drug efficacies. Therefore, according to the fifty-sixth aspect, even in a case where the first probe (one or more kinds of amino acids), the second probe that is a virtual probe (one or more kinds of nucleic acid bases and the like and the combination thereof), and the third probe (a virtual point electric charge and the like and the combination thereof) are used, a target compound having drug efficacy similar to that of the binding compound is extracted based on the eighth feature quantity so that screening of a pharmaceutical candidate compound can be efficiently performed.

In order to achieve the above-described object, according to a fifty-seventh aspect of the present invention, there is provided a compound creating method of creating a three-dimensional structure of a target compound that is bound to a target protein from a plurality of compounds, the method comprising: a storing step of storing a three-dimensional structure of a compound formed of a plurality of atoms and the first feature quantity in association with each other for each of the plurality of compounds; a feature quantity calculating step of calculating the first feature quantity of a ligand that is a compound whose binding to the target protein has been confirmed using the feature quantity calculating method according to the second aspect; a generator constructing step of constructing a generator through machine learning using the three-dimensional structure of the plurality of compounds as teacher data and the first feature quantity as an explanatory variable; and a compound three-dimensional structure generating step of generating a three-dimensional structure of the target compound from the first feature quantity of the ligand using the generator.

In the screening methods according to the twenty-eighth to forty-first aspects described above, a compound that is compatible with a ligand or a target protein is found among a plurality of compounds whose structural formulae have already been determined (written down). Accordingly, after the feature quantity of the compound is calculated, a method of extracting the compound based on the similarity with the feature quantity of the separately calculated ligand or the pocket structure of the target protein, that is, a search method is employed. Therefore, in a case where the correspondence between the structural formula of the compound and the feature quantity thereof is recorded, a structural formula having a high similarity (greater than or equal to the threshold) can be found. Meanwhile, in the fifty-seventh aspect, a structural formula of a compound having a feature quantity similar to the feature quantity (the first feature quantity) of the ligand (accordingly, the drug efficacies are similar) is generated without performing search.

The generation of the structural formula in a case where the feature quantity has been provided can be performed using a generator constructed through machine learning. Specifically, in the fifty-seventh aspect, a generator is constructed through machine learning (the learning method is not particularly limited) using the three-dimensional structure of the compounds as teacher data and the first feature quantity as an explanatory variable, and a three-dimensional structure of the target compound is generated from the first feature quantity of the ligand using the generator. In the fifty-seventh aspect, since search is not performed, the three-dimensional structure of the compound can be generated even in a case of "no solution was found as the result of screening search", and thus the three-dimensional structure of the pharmaceutical candidate compound can be efficiently created.

In the fifty-seventh aspect, the three-dimensional structure to be generated is affected by the features of the compound provided as teacher data. Therefore, by selecting the features of the compound to be provided as teacher data, a compound having a three-dimensional structure with different features can be generated. For example, a compound having a three-dimensional structure that is easily synthesized can be generated by providing a compound that is easily synthesized as teacher data.

In order to achieve the above-described object, according to a fifty-eighth aspect of the present invention, there is provided a compound creating method of creating a three-dimensional structure of a target compound that is bound to a target protein from a plurality of compounds, the method comprising: a storing step of storing a three-dimensional structure of a compound formed of a plurality of atoms and the first invariant feature quantity calculated using the feature quantity calculating method according to the third aspect in association with each other for each of the plurality of compounds; a feature quantity calculating step of calculating the first invariant feature quantity of a ligand that is a compound whose binding to the target protein has been confirmed; a generator constructing step of constructing a generator through machine learning using the three-dimensional structure of the plurality of compounds as teacher data and the first invariant feature quantity as an explanatory variable; and a compound three-dimensional structure generating step of generating a three-dimensional structure of the target compound from the first invariant feature quantity of the ligand using the generator. In the fifty-eighth aspect, similar to the fifty-seventh aspect, a structural formula of a compound having a feature quantity similar to the feature quantity (the first invariant feature quantity) of the ligand (accordingly, the drug efficacies are similar) is generated without performing search, and thus the three-dimensional structure of the pharmaceutical candidate compound can be efficiently created. Therefore, similar to the fifty-seventh aspect, by selecting the features of the compound to be provided as teacher data, a compound having a three-dimensional structure with different features can be generated.

In order to achieve the object described above, according to a fifty-ninth aspect of the present invention, there is provided a compound creating method of creating a three-dimensional structure of a target compound that is bound to a target protein from a plurality of compounds, the method comprising: a storing step of storing a three-dimensional structure of a compound formed of a plurality of atoms and the first feature quantity calculated using the feature quantity calculating method according to the second aspect in association with each other for each of the plurality of compounds; a feature quantity calculating step of calculating the second feature quantity of the pocket structure of the target protein using the feature quantity calculating method according to the fifth aspect; a generator constructing step of constructing a generator through machine learning using the three-dimensional structure of the plurality of compounds as teacher data and the first feature quantity as an explanatory variable; and a compound three-dimensional structure generating step of generating a three-dimensional structure of the target compound from the second feature quantity of the pocket structure using the generator. According to the fifty-ninth aspect, similar to the fifty-seventh and fifty-eighth aspects, a structural formula of a compound having a feature quantity similar to the feature quantity (the second feature quantity) of the pocket structure (accordingly, the drug efficacies are similar) is generated without performing search, and thus the three-dimensional structure of the pharmaceutical candidate compound can be efficiently created. Similar to the fifty-seventh and fifty-eighth aspects, by selecting the features of the compound to be provided as teacher data, a compound having a three-dimensional structure with different features can be generated.

In order to achieve the object described above, according to a sixtieth aspect of the present invention, there is provided a compound creating method of creating a three-dimensional structure of a target compound that is bound to a target protein from a plurality of compounds, the method comprising: a storing step of storing a three-dimensional structure of a compound formed of a plurality of atoms and the first invariant feature quantity calculated using the feature quantity calculating method according to the third aspect in association with each other for each of the plurality of compounds; a feature quantity calculating step of calculating the second invariant feature quantity of the pocket structure of the target protein using the feature quantity calculating method according to the sixth aspect; a generator constructing step of constructing a generator through machine learning using the three-dimensional structure of the plurality of compounds as teacher data and the first invariant feature quantity as an explanatory variable; and a compound three-dimensional structure generating step of generating a three-dimensional structure of the target compound from the second invariant feature quantity of the pocket structure using the generator. According to the sixtieth aspect, similar to the fifty-seventh to fifty-ninth aspects, a structural formula of a compound having a feature quantity similar to the feature quantity (the second invariant feature quantity) of the pocket structure (accordingly, the drug efficacies are similar) is generated without performing search, and thus the three-dimensional structure of the pharmaceutical candidate compound can be efficiently created. Similar to the fifty-seventh to fifty-ninth aspects, by selecting the features of the compound to be provided as teacher data, a compound having a three-dimensional structure with different features can be generated.

In order to achieve the above-described object, according to a sixty-first aspect of the present invention, there is provided a compound creating method of creating a three-dimensional structure of a target compound that is bound to a target biopolymer other than a protein from a plurality of compounds, the method comprising: a storing step of storing a three-dimensional structure of a compound formed of a plurality of atoms and the third feature quantity in association with each other for each of the plurality of compounds; a feature quantity calculating step of calculating the third feature quantity of a binding compound that is a compound whose binding to the target biopolymer other than the protein has been confirmed using the feature quantity calculating method according to the eighth aspect; a generator constructing step of constructing a generator through machine learning using the three-dimensional structure of the plurality of compounds as teacher data and the third feature quantity as an explanatory variable; and a compound three-dimensional structure generating step of generating a three-dimensional structure of the target compound from the third feature quantity of the binding compound using the generator.

According to the sixty-first aspect, similar to the fifty-seventh to sixtieth aspects, a structural formula of a compound having a feature quantity similar to the feature quantity (the third feature quantity) of the binding compound (accordingly, the drug efficacies are similar) is generated without performing search, and thus the three-dimensional structure of the pharmaceutical candidate compound can be efficiently created. Similar to the fifty-seventh to sixtieth aspects, by selecting the features of the compound to be provided as teacher data, a compound having a three-dimensional structure with different features can be generated.

In order to achieve the above-described object, according to a sixty-second aspect of the present invention, there is provided a compound creating method of creating a three-dimensional structure of a target compound that is bound to a target biopolymer from a plurality of compounds, the method comprising: a storing step of storing a three-dimensional structure of a compound formed of a plurality of atoms and the fourth feature quantity in association with each other for each of the plurality of compounds; a feature quantity calculating step of calculating the fourth feature quantity of a binding compound that is a compound whose binding to the target biopolymer has been confirmed using the feature quantity calculating method according to the eleventh aspect; a generator constructing step of constructing a generator through machine learning using the three-dimensional structure of the plurality of compounds as teacher data and the fourth feature quantity as an explanatory variable; and a compound three-dimensional structure generating step of generating a three-dimensional structure of the target compound from the fourth feature quantity of the binding compound using the generator.

According to the sixty-second aspect, similar to the fifty-seventh to sixty-first aspects, a structural formula of a compound having a feature quantity similar to the feature quantity (the fourth feature quantity) of the binding compound (accordingly, the drug efficacies are similar) is generated without performing search, and thus the three-dimensional structure of the pharmaceutical candidate compound can be efficiently created. Similar to the fifty-seventh to sixty-first aspects, by selecting the features of the compound to be provided as teacher data, a compound having a three-dimensional structure with different features can be generated.

In order to achieve the above-described object, according to a sixty-third aspect of the present invention, there is provided a compound creating method of creating a three-dimensional structure of a target compound that is bound to a target biopolymer from a plurality of compounds, the method comprising: a storing step of storing a three-dimensional structure of a compound formed of a plurality of atoms and the fifth feature quantity in association with each other for each of the plurality of compounds; a feature quantity calculating step of calculating the fifth feature quantity of a binding compound that is a compound whose binding to the target biopolymer has been confirmed using the feature quantity calculating method according to the fourteenth aspect; a generator constructing step of constructing a generator through machine learning using the three-dimensional structure of the plurality of compounds as teacher data and the fifth feature quantity as an explanatory variable; and a compound three-dimensional structure generating step of generating a three-dimensional structure of the target compound from the fifth feature quantity of the binding compound using the generator.

According to the sixty-third aspect, similar to the fifty-seventh to sixty-second aspects, a structural formula of a compound having a feature quantity similar to the feature quantity (the fifth feature quantity) of the binding compound (accordingly, the drug efficacies are similar) is generated without performing search, and thus the three-dimensional structure of the pharmaceutical candidate compound can be efficiently created. Similar to the fifty-seventh to sixty-second aspects, by selecting the features of the compound to be provided as teacher data, a compound having a three-dimensional structure with different features can be generated.

In order to achieve the object described above, according to a sixty-fourth aspect of the present invention, there is provided a compound creating method of creating a three-dimensional structure of a target compound that is bound to a target biopolymer from a plurality of compounds, the method comprising: a storing step of storing a three-dimensional structure of a compound formed of a plurality of atoms and the sixth feature quantity in association with each other for each of the plurality of compounds; a feature quantity calculating step of calculating the sixth feature quantity of a binding compound that is a compound whose binding to the target biopolymer has been confirmed using the feature quantity calculating method according to the seventeenth aspect; a generator constructing step of constructing a generator through machine learning using the three-dimensional structure of the plurality of compounds as teacher data and the sixth feature quantity as an explanatory variable; and a compound three-dimensional structure generating step of generating a three-dimensional structure of the target compound from the sixth feature quantity of the binding compound using the generator.

According to the sixty-fourth aspect, similar to the fifty-seventh to sixty-third aspects, a structural formula of a compound having a feature quantity similar to the feature quantity (the sixth feature quantity) of the binding compound (accordingly, the drug efficacies are similar) is generated without performing search, and thus the three-dimensional structure of the pharmaceutical candidate compound can be efficiently created. Similar to the fifty-seventh to sixty-third aspects, by selecting the features of the compound to be provided as teacher data, a compound having a three-dimensional structure with different features can be generated.

In order to achieve the object described above, according to a sixty-fifth aspect of the present invention, there is provided a compound creating method of creating a three-dimensional structure of a target compound that is bound to a target biopolymer from a plurality of compounds, the method comprising: a storing step of storing a three-dimensional structure of a compound formed of a plurality of atoms and the seventh feature quantity in association with each other for each of the plurality of compounds; a feature quantity calculating step of calculating the seventh feature quantity of a binding compound that is a compound whose binding to the target biopolymer has been confirmed using the feature quantity calculating method according to the twentieth aspect; a generator constructing step of constructing a generator through machine learning using the three-dimensional structure of the plurality of compounds as teacher data and the seventh feature quantity as an explanatory variable; and a compound three-dimensional structure generating step of generating a three-dimensional structure of the target compound from the seventh feature quantity of the binding compound using the generator.

According to the sixty-fifth aspect, similar to the fifty-seventh to sixty-fourth aspects, a structural formula of a compound having a feature quantity similar to the feature quantity (the seventh feature quantity) of the binding compound (accordingly, the drug efficacies are similar) is generated without performing search, and thus the three-dimensional structure of the pharmaceutical candidate compound can be efficiently created. Similar to the fifty-seventh to sixty-fourth aspects, by selecting the features of the compound to be provided as teacher data, a compound having a three-dimensional structure with different features can be generated.

In order to achieve the above-described object, according to a sixty-sixth aspect of the present invention, there is provided a compound creating method of creating a three-dimensional structure of a target compound that is bound to a target biopolymer from a plurality of compounds, the method comprising: a storing step of storing a three-dimensional structure of a compound formed of a plurality of atoms and the eighth feature quantity in association with each other for each of the plurality of compounds; a feature quantity calculating step of calculating the eighth feature quantity of a binding compound that is a compound whose binding to the target biopolymer has been confirmed using the feature quantity calculating method according to the twenty-third aspect; a generator constructing step of constructing a generator through machine learning using the three-dimensional structure of the plurality of compounds as teacher data and the eighth feature quantity as an explanatory variable; and a compound three-dimensional structure generating step of generating a three-dimensional structure of the target compound from the eighth feature quantity of the binding compound using the generator.

According to the sixty-sixth aspect, similar to the fifty-seventh to sixty-fifth aspects, a structural formula of a compound having a feature quantity similar to the feature quantity (the eighth feature quantity) of the binding compound (accordingly, the drug efficacies are similar) is generated without performing search, and thus the three-dimensional structure of the pharmaceutical candidate compound can be efficiently created. Similar to the fifty-seventh to sixty-fifth aspects, by selecting the features of the compound to be provided as teacher data, a compound having a three-dimensional structure with different features can be generated.

In order to achieve the above-described object, according to a sixty-seventh aspect of the present invention, there is provided a compound creating program which causes a computer to execute the compound creating method according to any one of the fifty-seventh to sixty-sixth aspects. The "computer" in the sixty-seventh aspect can be realized using one or more of various processors such as a Central Processing Unit (CPU). Further, a non-temporary recording medium on which a computer-readable code of the compound creating program according to the sixty-seventh aspect is recorded can also be exemplified as an aspect of the present invention.

In order to achieve the above-described object, according to a sixty-eighth aspect of the present invention, there is provided a compound creating device which creates a three-dimensional structure of a target compound that is bound to a target protein from a plurality of compounds, the device comprising: a storage unit which stores a three-dimensional structure of a compound formed of a plurality of atoms and the first feature quantity in association with each other for each of the plurality of compounds; a feature quantity calculation unit which calculates the first feature quantity of a ligand that is a compound whose binding to the target protein has been confirmed using the feature quantity calculating method according to the second aspect; a generator construction unit which constructs a generator through machine learning using the three-dimensional structure of the plurality of compounds as teacher data and the first feature quantity as an explanatory variable; and a compound three-dimensional structure generation unit which generates a three-dimensional structure of the target compound from the first feature quantity of the ligand using the generator.

In the screening device according to the forty-third to fifty-sixth aspects described above, a compound that is compatible with a ligand or a target protein is found among a plurality of compounds whose structural formulae have already been determined (written down). Accordingly, after the feature quantity of the compound is calculated, a method of extracting the compound based on the similarity with the feature quantity of the separately calculated ligand or the pocket structure of the target protein, that is, a search method is employed. Therefore, in a case where the correspondence between the structural formula of the compound and the feature quantity thereof is recorded, a structural formula having a high similarity (greater than or equal to the threshold) can be found. Meanwhile, in the sixty-eighth aspect, a structural formula of a compound having a feature quantity similar to the feature quantity (the first feature quantity) of the ligand (accordingly, the drug efficacies are similar) is generated without performing search.

The generation of the structural formula in a case where the feature quantity has been provided can be performed using a generator constructed through machine learning. Specifically, in the sixty-eighth aspect, a generator is constructed through machine learning (the learning method is not particularly limited) using the three-dimensional structure of the compounds as teacher data and the first feature quantity as an explanatory variable, and a three-dimensional structure of the target compound is generated from the first feature quantity of the ligand using the generator. In the sixty-eighth aspect, since search is not performed, the three-dimensional structure of the compound can be generated even in a case of "no solution was found as the result of screening search", and thus the three-dimensional structure of the pharmaceutical candidate compound can be efficiently created.

In the sixty-eighth aspect, the three-dimensional structure to be generated is affected by the features of the compound provided as teacher data. Therefore, by selecting the features of the compound to be provided as teacher data, a compound having a three-dimensional structure with different features can be generated. For example, a compound having a three-dimensional structure that is easily synthesized can be generated by providing a compound that is easily synthesized as teacher data.

In order to achieve the above-described object, according to a sixty-ninth aspect of the present invention, there is provided a compound creating device which creates a three-dimensional structure of a target compound that is bound to a target protein from a plurality of compounds, the device comprising: a storage unit which stores a three-dimensional structure of a compound formed of a plurality of atoms and the first invariant feature quantity calculated using the feature quantity calculating method according to the third aspect in association with each other for each of the plurality of compounds; a feature quantity calculation unit which calculates the first invariant feature quantity of a ligand that is a compound whose binding to the target protein has been confirmed; a generator construction unit which constructs a generator through machine learning using the three-dimensional structure of the plurality of compounds as teacher data and the first invariant feature quantity as an explanatory variable; and a compound three-dimensional structure generation unit which generates a three-dimensional structure of the target compound from the first invariant feature quantity of the ligand using the generator. In the sixty-ninth aspect, similar to the sixty-eighth aspect, a structural formula of a compound having a feature quantity similar to the feature quantity (the first invariant feature quantity) of the ligand (accordingly, the drug efficacies are similar) is generated without performing search, and thus the three-dimensional structure of the pharmaceutical candidate compound can be efficiently created. Therefore, similar to the sixty-eighth aspect, by selecting the features of the compound to be provided as teacher data, a compound having a three-dimensional structure with different features can be generated.

In order to achieve the object described above, according to a seventieth aspect of the present invention, there is provided a compound creating device which creates a three-dimensional structure of a target compound that is bound to a target protein from a plurality of compounds, the device comprising: a storage unit which stores a three-dimensional structure of a compound formed of a plurality of atoms and the first feature quantity calculated using the feature quantity calculating method according to the second aspect in association with each other for each of the plurality of compounds; a feature quantity calculation unit which calculates the second feature quantity of the pocket structure of the target protein using the feature quantity calculating method according to the fifth aspect; a generator construction unit which constructs a generator through machine learning using the three-dimensional structure of the plurality of compounds as teacher data and the first feature quantity as an explanatory variable; and a compound three-dimensional structure generation unit which generates a three-dimensional structure of the target compound from the second feature quantity of the pocket structure using the generator. According to the seventieth aspect, similar to the sixty-eighth and sixty-ninth aspects, a structural formula of a compound having a feature quantity similar to the feature quantity (the second feature quantity) of the pocket structure (accordingly, the drug efficacies are similar) is generated without performing search, and thus the three-dimensional structure of the pharmaceutical candidate compound can be efficiently created. Similar to the sixty-eighth and sixty-ninth aspects, by selecting the features of the compound to be provided as teacher data, a compound having a three-dimensional structure with different features can be generated.

In order to achieve the object described above, according to a seventy-first aspect of the present invention, there is provided a compound creating device which creates a three-dimensional structure of a target compound that is bound to a target protein from a plurality of compounds, the device comprising: a storage unit which stores a three-dimensional structure of a compound formed of a plurality of atoms and the first invariant feature quantity calculated using the feature quantity calculating method according to the third aspect in association with each other for each of the plurality of compounds; a feature quantity calculation unit which calculates the second invariant feature quantity of the pocket structure of the target protein using the feature quantity calculating method according to the sixth aspect; a generator construction unit which constructs a generator through machine learning using the three-dimensional structure of the plurality of compounds as teacher data and the first invariant feature quantity as an explanatory variable; and a compound three-dimensional structure generation unit which generates a three-dimensional structure of the target compound from the second invariant feature quantity of the pocket structure using the generator. According to the seventy-first aspect, similar to the sixty-eighth to seventieth aspects, a structural formula of a compound having a feature quantity similar to the feature quantity (the second invariant feature quantity) of the pocket structure (accordingly, the drug efficacies are similar) is generated without performing search, and thus the three-dimensional structure of the pharmaceutical candidate compound can be efficiently created. Similar to the sixty-eighth to seventieth aspects, by selecting the features of the compound to be provided as teacher data, a compound having a three-dimensional structure with different features can be generated.

In order to achieve the object described above, according to a seventy-second aspect of the present invention, there is provided a compound creating device which creates a three-dimensional structure of a target compound that is bound to a target biopolymer other than a protein from a plurality of compounds, the device comprising: a storage unit which stores a three-dimensional structure of a compound formed of a plurality of atoms and the third feature quantity in association with each other for each of the plurality of compounds; a feature quantity calculation unit which calculates the third feature quantity of a binding compound that is a compound whose binding to the target biopolymer other than the protein has been confirmed using the feature quantity calculating method according to the eighth aspect; a generator construction unit which constructs a generator through machine learning using the three-dimensional structure of the plurality of compounds as teacher data and the third feature quantity as an explanatory variable; and a compound three-dimensional structure generation unit which generates a three-dimensional structure of the target compound from the third feature quantity of the binding compound using the generator.

According to the seventy-second aspect, similar to the sixty-eighth to seventy-first aspects, a structural formula of a compound having a feature quantity similar to the feature quantity (the third feature quantity) of the binding compound (accordingly, the drug efficacies are similar) is generated without performing search, and thus the three-dimensional structure of the pharmaceutical candidate compound can be efficiently created. Similar to the sixty-eighth to seventy-first aspects, by selecting the features of the compound to be provided as teacher data, a compound having a three-dimensional structure with different features can be generated.

In order to achieve the above-described object, according to a seventy-third aspect of the present invention, there is provided a compound creating device which creates a three-dimensional structure of a target compound that is bound to a target biopolymer from a plurality of compounds, the device comprising: a storage unit which stores a three-dimensional structure of a compound formed of a plurality of atoms and the fourth feature quantity in association with each other for each of the plurality of compounds; a feature quantity calculation unit which calculates the fourth feature quantity of the binding compound that is a compound whose binding to the target biopolymer has been confirmed using the feature quantity calculating method according to the eleventh aspect; a generator construction unit which constructs a generator through machine learning using the three-dimensional structure of the plurality of compounds as teacher data and the fourth feature quantity as an explanatory variable; and a compound three-dimensional structure generation unit which generates a three-dimensional structure of the target compound from the fourth feature quantity of the binding compound using the generator.

According to the seventy-third aspect, similar to the sixty-eighth to seventy-second aspects, a structural formula of a compound having a feature quantity similar to the feature quantity (the fourth feature quantity) of the binding compound (accordingly, the drug efficacies are similar) is generated without performing search, and thus the three-dimensional structure of the pharmaceutical candidate compound can be efficiently created. Similar to the sixty-eighth to seventy-second aspects, by selecting the features of the compound to be provided as teacher data, a compound having a three-dimensional structure with different features can be generated.

In order to achieve the above-described object, according to a seventy-fourth aspect of the present invention, there is provided a compound creating device which creates a three-dimensional structure of a target compound that is bound to a target biopolymer from a plurality of compounds, the device comprising: a storage unit which stores a three-dimensional structure of a compound formed of a plurality of atoms and the fifth feature quantity in association with each other for each of the plurality of compounds; a feature quantity calculation unit which calculates the fifth feature quantity of the binding compound that is a compound whose binding to the target biopolymer has been confirmed using the feature quantity calculating method according to the fourteenth aspect; a generator construction unit which constructs a generator through machine learning using the three-dimensional structure of the plurality of compounds as teacher data and the fifth feature quantity as an explanatory variable; and a compound three-dimensional structure generation unit which generates a three-dimensional structure of the target compound from the fifth feature quantity of the binding compound using the generator.

According to the seventy-fourth aspect, similar to the sixty-eighth to seventy-third aspects, a structural formula of a compound having a feature quantity similar to the feature quantity (the fifth feature quantity) of the binding compound (accordingly, the drug efficacies are similar) is generated without performing search, and thus the three-dimensional structure of the pharmaceutical candidate compound can be efficiently created. Similar to the sixty-eighth to seventy-third aspects, by selecting the features of the compound to be provided as teacher data, a compound having a three-dimensional structure with different features can be generated.

In order to achieve the above-described object, according to a seventy-fifth aspect of the present invention, there is provided a compound creating device which creates a three-dimensional structure of a target compound that is bound to a target biopolymer from a plurality of compounds, the device comprising: a storage unit which stores a three-dimensional structure of a compound formed of a plurality of atoms and the sixth feature quantity in association with each other for each of the plurality of compounds; a feature quantity calculation unit which calculates the sixth feature quantity of the binding compound that is a compound whose binding to the target biopolymer has been confirmed using the feature quantity calculating method according to the seventeenth aspect; a generator construction unit which constructs a generator through machine learning using the three-dimensional structure of the plurality of compounds as teacher data and the sixth feature quantity as an explanatory variable; and a compound three-dimensional structure generation unit which generates a three-dimensional structure of the target compound from the sixth feature quantity of the binding compound using the generator.

According to the seventy-fifth aspect, similar to the sixty-eighth to seventy-fourth aspects, a structural formula of a compound having a feature quantity similar to the feature quantity (the sixth feature quantity) of the binding compound (accordingly, the drug efficacies are similar) is generated without performing search, and thus the three-dimensional structure of the pharmaceutical candidate compound can be efficiently created. Similar to the sixty-eighth to seventy-fourth aspects, by selecting the features of the compound to be provided as teacher data, a compound having a three-dimensional structure with different features can be generated.

In order to achieve the above-described object, according to a seventy-sixth aspect of the present invention, there is provided a compound creating device which creates a three-dimensional structure of a target compound that is bound to a target biopolymer from a plurality of compounds, the device comprising: a storage unit which stores a three-dimensional structure of a compound formed of a plurality of atoms and the seventh feature quantity in association with each other for each of the plurality of compounds; a feature quantity calculation unit which calculates the seventh feature quantity of the binding compound that is a compound whose binding to the target biopolymer has been confirmed using the feature quantity calculating method according to the twentieth aspect; a generator construction unit which constructs a generator through machine learning using the three-dimensional structure of the plurality of compounds as teacher data and the seventh feature quantity as an explanatory variable; and a compound three-dimensional structure generation unit which generates a three-dimensional structure of the target compound from the seventh feature quantity of the binding compound using the generator.

According to the seventy-sixth aspect, similar to the sixty-eighth to seventy-fifth aspects, a structural formula of a compound having a feature quantity similar to the feature quantity (the seventh feature quantity) of the binding compound (accordingly, the drug efficacies are similar) is generated without performing search, and thus the three-dimensional structure of the pharmaceutical candidate compound can be efficiently created. Similar to the sixty-eighth to seventy-fifth aspects, by selecting the features of the compound to be provided as teacher data, a compound having a three-dimensional structure with different features can be generated.

In order to achieve the object described above, according to a seventy-seventh aspect of the present invention, there is provided a compound creating device which creates a three-dimensional structure of a target compound that is bound to a target biopolymer from a plurality of compounds, the device comprising: a storage unit which stores a three-dimensional structure of a compound formed of a plurality of atoms and the eighth feature quantity in association with each other for each of the plurality of compounds; a feature quantity calculation unit which calculates the eighth feature quantity of the binding compound that is a compound whose binding to the target biopolymer has been confirmed using the feature quantity calculating method according to the twenty-third aspect; a generator construction unit which constructs a generator through machine learning using the three-dimensional structure of the plurality of compounds as teacher data and the eighth feature quantity as an explanatory variable; and a compound three-dimensional structure generation unit which generates a three-dimensional structure of the target compound from the eighth feature quantity of the binding compound using the generator.

According to the seventy-seventh aspect, similar to the sixty-eighth to seventy-sixth aspects, a structural formula of a compound having a feature quantity similar to the feature quantity (the eighth feature quantity) of the binding compound (accordingly, the drug efficacies are similar) is generated without performing search, and thus the three-dimensional structure of the pharmaceutical candidate compound can be efficiently created. Similar to the sixty-eighth to seventy-sixth aspects, by selecting the features of the compound to be provided as teacher data, a compound having a three-dimensional structure with different features can be generated.

As described above, according to the feature quantity calculating method, the feature quantity calculating program, and the feature quantity calculating device of the present invention, it is possible to calculate a feature quantity that accurately shows chemical properties of a target structure. Further, according to the screening method, the screening program, and the screening device of the present invention, screening of a pharmaceutical candidate compound can be efficiently performed. Further, according to the compound creating method, compound creating program, and compound creating device of the present invention, it is possible to efficiently create a three-dimensional structure of a pharmaceutical candidate compound.

## BRIEF DESCRIPTION OF THE DRAWINGS

Fig. 1 is a block diagram showing a configuration of a screening device according to a first embodiment.
Fig. 2 is a block diagram showing a configuration of a processing unit.
Fig. 3 is a diagram illustrating information stored in a storage unit.
Fig. 4 is a diagram showing a state in which structure information of a compound and a feature quantity thereof are stored in association with each other.
Fig. 5 is a flowchart showing a procedure for calculating a three-dimensional AAM feature quantity of a compound.
Figs. 6A and 6B are diagrams showing an example of three-dimensionalization of a structural formula.
Figs. 7A and 7B are diagrams showing an example of a three-dimensional AAM feature quantity.
Fig. 8 is a table showing an example of a three-dimensional AAM feature quantity.
Fig. 9 is a flowchart showing a procedure for a three-dimensional AAM descriptor for a pocket structure.
Figs. 10A to 10C are conceptual views showing a state of a three-dimensional AAM descriptor for a pocket structure.
Figs. 11A and 11B are graphs showing an example of an invariant AAM descriptor.
Figs. 12A and 12B show an example of compounds with similar invariant AAM descriptors.
Fig. 13 is a graph showing easiness of finding a hit in a case where an invariant AAM descriptor is used.
Fig. 14 is a graph showing the time of search for a hit in a case where an invariant AAM descriptor is used.
Fig. 15 is another graph showing the time of search a hit in a case where an invariant AAM descriptor is used.
Fig. 16 is a flowchart showing a procedure for extracting a target compound based on the similarity between descriptors.
Figs. 17A and 17B are tables showing an example of a result of extracting a target compound based on the similarity of descriptors.
Fig. 18 is another flowchart showing a procedure for extracting a target compound based on the similarity between descriptors.

Figs. 19A and 19B are other tables showing an example of a result of extracting a target compound based on the similarity of the descriptor.

Fig. 20 is a block diagram showing a configuration of a compound creating device according to a second embodiment.

Fig. 21 is a diagram showing a configuration of a processing unit.

Fig. 22 is a diagram showing information stored in a storage unit.

Fig. 23 is a flowchart showing a procedure for generating a three-dimensional structure in the case where a ligand is input.

Fig. 24 is a diagram showing a state of generating a three-dimensional structure using a result of machine learning.

Fig. 25 is a graph showing a relationship between the number of interlayers and the cos similarity.

Figs. 26A and 26B are diagrams showing an example of generating a three-dimensional structure.

Fig. 27 is a flowchart showing a procedure for generating a three-dimensional structure in a case where a target protein is input.

Fig. 28 is a block diagram showing a configuration of a pharmaceutical candidate compound search device according to a third embodiment.

Fig. 29 is a diagram showing a configuration of a processing unit.

Fig. 30 is a diagram showing information stored in a storage unit.

Fig. 31 is a diagram showing a comparison result of easiness of finding a hit.

## DESCRIPTION OF THE PREFERRED EMBODIMENTS

Hereinafter, embodiments of a feature quantity calculating method, a screening device, and a compound creating device of the present invention will be described in detail with reference to the accompanying drawings.

<First embodiment

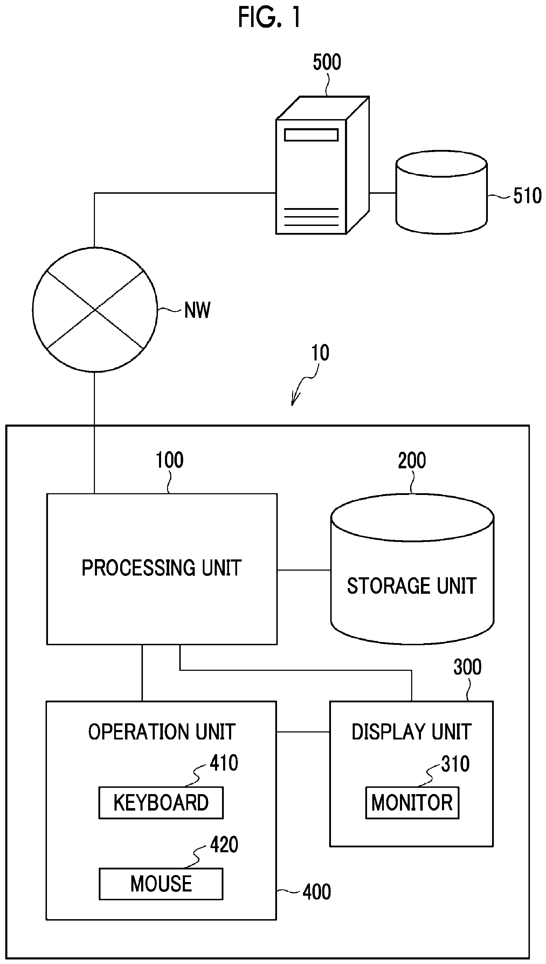

Fig. 1 is a block diagram showing a configuration of a screening device 10 (a feature quantity calculating device or a screening device) according to a first embodiment. The screening device 10 is a device that calculates a feature quantity of a compound (target structure) and/or a pocket structure (target structure) and extracts (screens) a target compound, and can be realized using a computer. As shown in Fig. 1, the screening device 10 includes a processing unit 100, a storage unit 200, a display unit 300, and an operation unit 400, and these units are connected to one another to transmit and receive necessary information. These constituent elements may be installed by employing various installation forms. Respective constituent elements may be installed in one site (in one housing, one room, or the like) or may be installed in places separated from each other and connected via a network. Further, the screening device 10 is connected to an external server 500 and an external database 510 such as a Protein Data Bank (PDB) via a network NW such as the Internet, and information related to structural formulae of compounds and crystal structures of proteins can be obtained as necessary.

<Configuration of processing unit>

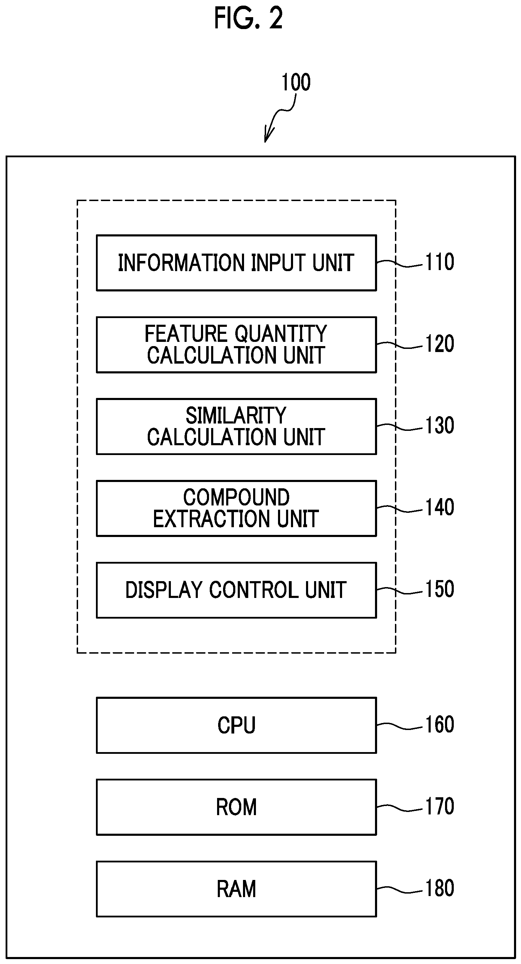

Fig. 2 is a diagram showing a configuration of the processing unit 100. The processing unit 100 includes an information input unit 110, a feature quantity calculation unit 120, a similarity calculation unit 130, a compound extraction unit 140, a display control unit 150, a CPU 160 (CPU: Central Processing Unit), a ROM 170 (ROM: Read Only Memory), and a RAM 180 (Random Access Memory).

The information input unit 110 inputs information related to a structural formula of a compound, an X crystal structure of a target protein, and a pocket position via a recording medium interface such as a DVD drive (not shown) or a semiconductor memory terminal and/or a network NW. The feature quantity calculation unit 120 (the target structure designation unit, the three-dimensional structure generation unit, or the feature quantity calculation unit) calculates feature quantities (a first feature quantity, a first invariant feature quantity, and a second feature quantity, a second invariant feature quantity) according to the present invention. The similarity calculation unit 130 (the similarity calculation unit) calculates the similarity between the calculated feature quantities. The compound extraction unit 140 (the compound extraction unit) extracts a target compound from a plurality of compounds based on the similarity. The display control unit 150 controls input information and display of the process result on the monitor 310. The process of calculation of the feature quantity and screening of the target compound using these functions of the processing unit 100 will be described below in detail. Further, the process using these functions is performed under the control of the CPU 160.

The function of each unit of the processing unit 100 described above can be realized using various processors. Various processors include a CPU that is a general-purpose processor that executes software (program) to realize various functions. Further, the various processors described above include a graphics processing unit (GPU) serving as a processor specialized in image processing and a programmable logic device (PLD) serving as a processor that can change the circuit configuration after manufacture of a field programmable gate array (FPGA). Further, the above-described various processors include an exclusive electric circuit serving as a processor having a circuit configuration designed exclusively for executing a specific process such as an application specific integrated circuit (ASIC).

The functions of each unit may be realized by one processor, or may be realized by a plurality of same or different processors (for example, a plurality of FPGAs, a combination of a CPU and an FPGA, or a combination of a CPU and a GPU). Further, a plurality of functions may be realized by one processor. As an example in which a plurality of functions are configured by one processor, first, as represented by a computer such as a client or a server, a form of one processor which is configured by a combination of one or more CPUs and software and can be realized as a plurality of functions is exemplified. Second, as represented by a system-on-chip (SoC) or the like, there is a form in which a processor that realizes the functions of the entire system by one integrated circuit (IC) chip is used. As described above, various functions are configured using one or more of the above-described various processors as a hardware structure. Further, the hardware structure of these various processors is more specifically an electric circuit (circuitry) in which circuit elements such as semiconductor elements are combined.

In a case where the above-described processor or electric circuit executes software (program), a processor (computer)-readable code of the software to be executed is stored in a non-temporary recording medium such as a ROM 170 (see Fig. 2). The processor refers to the software. The software stored in the non-temporary recording medium includes the feature quantity calculating method and a program (a feature quantity calculating program and a screening program) for executing a target compound extraction process according to the present invention. The code may be recorded on non-temporary recording media such as various magneto-optical recording devices and semiconductor memories instead of the ROM 170. During the process using software, for example, the RAM 180 is used as a temporary storage area, and the data stored in, for example, an electronically erasable and programmable read only memory (EEPROM) (not shown) can also be referred to.

<Configuration of storage unit>

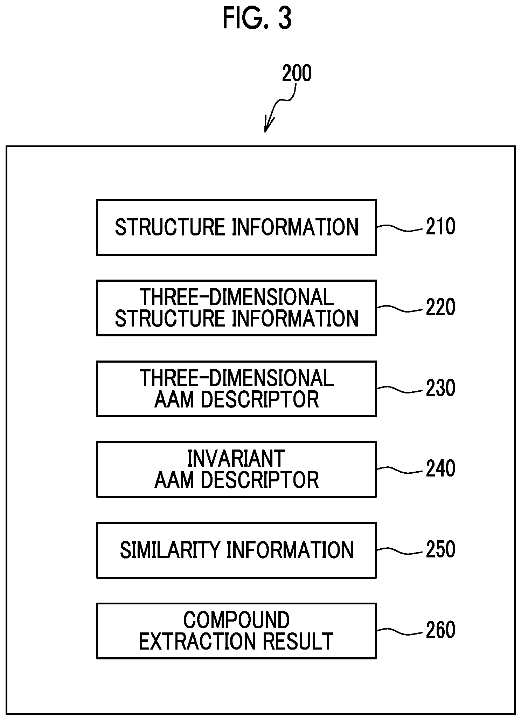

The storage unit 200 is formed of non-temporary recording media such as a digital versatile disk (DVD), a hard disk, and various semiconductor memories and control units thereof and stores images and information shown in Fig. 3. Structure information 210 includes the structural formula of the compound, the three-dimensional structure of the target protein, and the position of the pocket. Three-dimensional structure information 220 is information related to the three-dimensional structure of the compound and/or the pocket structure generated from the structure information 210. A three-dimensional AAM descriptor 230 (the first feature quantity or the second feature quantity) is a feature quantity obtained by quantifying, in a three-dimensional space, the degree of accumulation of one or more kinds of amino acids in the periphery of the three-dimensional structure of the compound or the pocket structure and is calculated according to the feature quantity calculating method described below. Further, "AAM" stands for "Amino Acid Mapping". An invariant AAM descriptor 240 (the first invariant feature quantity or the second invariant feature quantity) is a feature quantity obtained by converting the three-dimensional AAM descriptor 230 into an invariant with respect to rotation and translation of the compound or the pocket structure. Similarity information 250 is information related to the similarity between the feature quantities, and a compound extraction result 260 is information related to the target compound extracted based on the similarity.

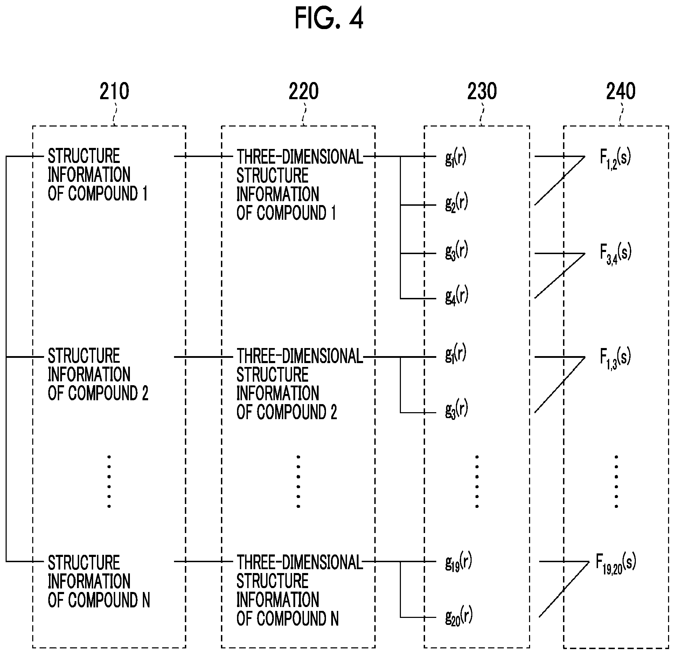

Fig. 4 shows a state in which the structure information 210, the three-dimensional structure information 220, the three-dimensional AAM descriptor 230, and the invariant AAM descriptor 240 are associated with one another and stored in the storage unit 200 for N compounds (N represents an integer of 2 or greater). In Fig. 4, for example, a structural formula can be used as the structure information 210, and a three-dimensionalized structural formula (described below) can be used as the three-dimensional structure information 220. In Fig. 4, the three-dimensional AAM descriptor 230 (described as "$g_a(r)$"; a is a subscript indicating the kind of amino acid) and the invariant AAM descriptor 240 (described as "$F_{ab}(s)$"; a and b are subscripts indicating the kinds of amino acids) corresponding to the three-dimensional AAM descriptor 230 for each of twenty kinds of amino acids for each compound are stored in association with each other. The three-dimensional AAM descriptor 230 and the invariant AAM descriptor 240 may be stored not for all twenty kinds of amino acids but for some amino acids according to the number of descriptors used for screening.

A plurality of sets (libraries) of information as shown in Fig. 4 may be stored in the storage unit 200. Fig. 4 shows a state in which information related to compounds is stored, and information related to target proteins can be stored with the same configuration. Further, a method for calculating a three-dimensional AAM descriptor and/or an invariant AAM descriptor using such structure information and three-dimensional structure information will be described below.

<Configuration of display unit and operation unit>

The display unit 300 includes the monitor 310 (display device) and can display input images, images and information stored in the storage unit 200, results of the process carried out by the processing unit 100, and the like. The operation unit 400 includes a keyboard 410 and a mouse 420 as an input device and/or a pointing device, and execution of the feature quantity calculating method according to the present invention and the operation required for extraction of the target compounds can be performed by a user through these devices and the screen of the monitor 310 (described later). The operations that can be performed by the user include, for example, a processing mode, the kind of descriptor to be calculated, a descriptor used for screening, and designation of a threshold with respect to the similarity.

<Process in screening device>

In the screening device 10 with the above-described configuration, calculation of a feature quantity (descriptor) and/or extraction of a target compound can be performed according to the user's instruction via the operation unit 400. Hereinafter, the details of each process will be described.

<Calculation of feature quantity>

The screening device 10 is capable of calculating a three-dimensional AAM descriptor and/or an invariant AAM descriptor according to the user's instruction via the operation unit 400.

<Calculation of three-dimensional AAM descriptor for compound>

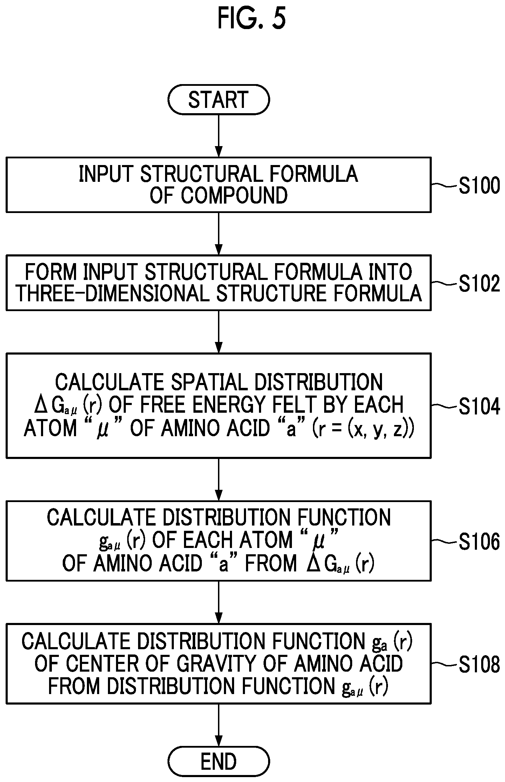

Fig. 5 is a flowchart showing a procedure for calculating a three-dimensional AAM descriptor for a compound (target structure). The ligand is a compound whose binding to the target protein has been confirmed, and the three-dimensional AAM descriptor can be calculated according to the procedure of Fig. 5. In Step S100, the information input unit 110 inputs a structural formula of a compound according to an operation by the user. In this manner, the compound represented by the input chemical formula is designated as the target structure (target structure designating step).

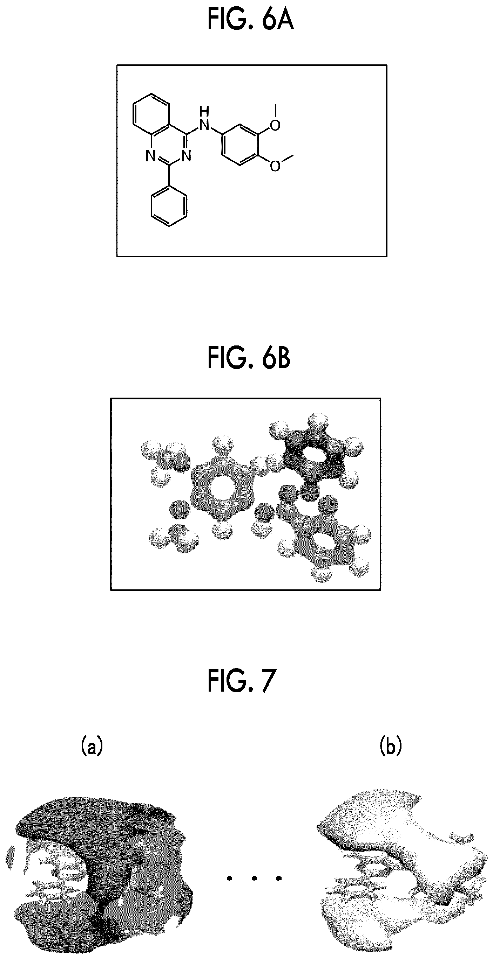

The feature quantity calculation unit 120 three-dimensionalizes the input structural formula to generate a three-dimensional structure of a compound formed of a plurality of atoms (a plurality of unit structures having chemical properties) (Step S102: a three-dimensional structure generating step). Various techniques are known for three-dimensionalization of a structural formula, and the present invention is not particularly limited to the technique used in Step S102. Figs. 6A and 6B show an example of a three-dimensional structuralized formula. Fig. 6A shows an input structural formula and Fig. 6B shows a three-dimensionalized structural formula.

The feature quantity calculation unit 120 calculates a spatial distribution $\Delta G_{a\mu}(r)$ of free energy felt by each atom "$\mu$" of an amino acid "a" (a represents a number representing the kind of amino acid; 1 to 20) (Step S104; feature quantity calculating step). As a method of calculating $\Delta G_{a\mu}(r)$, a molecular dynamics (MD) method can be employed, but the present invention is not limited thereto. The amino acid for calculating the feature quantity may be a predetermined kind of amino acid or may be determined according to the user's instruction (one or more kinds of amino acids may be used, and a plurality of kinds of amino acids may also be used).

The feature quantity calculation unit 120 calculates a distribution function $g_{a\mu}(r)$ of each atom "$\mu$" of the amino acid "a" from $\Delta G_{a\mu}(r)$ (Step S106: feature quantity calculating step). $g_{a\mu}(r)$ is represented by Equation (1) in a case where T is set as room temperature and $K_B$ is set as a Boltzmann constant.

$$g_{a\mu}(\mathbf{r}) = \exp\left(-\Delta G_{a\mu}(\mathbf{r})/K_B T\right) \qquad \cdots (1)$$

The feature quantity calculation unit 120 calculates a distribution function $g_{a\mu}(r)$ of the center of gravity of an amino acid from the distribution function $g_{a\mu}(r)$ (Step S108: feature quantity calculating step). For the calculation, $g_{a\mu}(r)$ is geometrically averaged for each atom "$\mu$". This distribution function $g_a(r)$ is a three-dimensional AAM descriptor (first feature quantity) obtained by quantifying, in a three-dimensional space, the degree of accumulation of one or more kinds of amino acids "a" in the periphery of the three-dimensional structure of the compound. The feature quantity calculation unit 120 stores the calculated three-dimensional AAM descriptor in the storage unit 200 as the three-dimensional AAM descriptor 230 in association with the structure information (structure information 210) and the three-dimensional structure information (three-dimensional structure information 220) of the compound. (see Fig. 4).

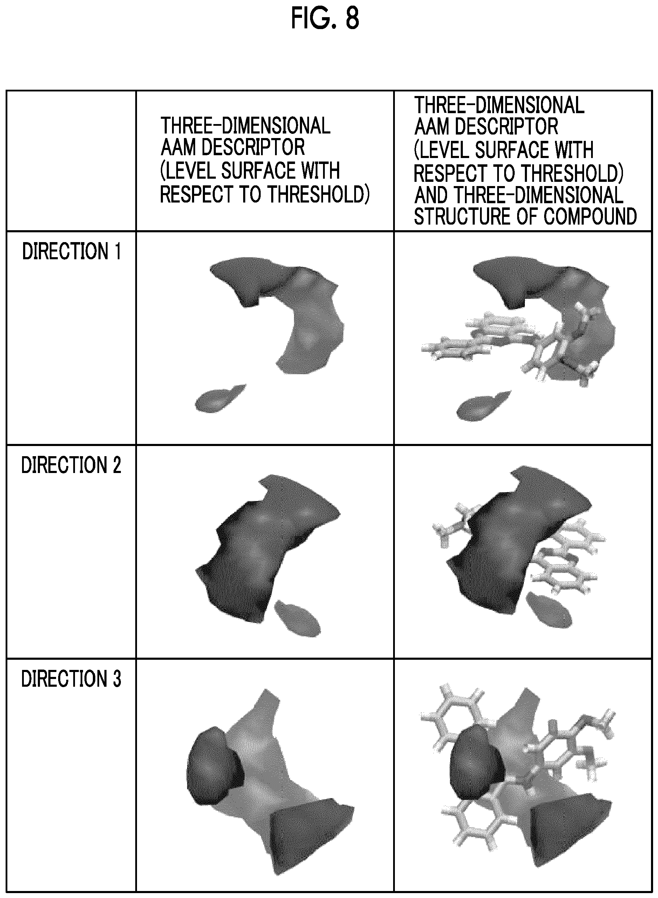

Figs. 7A and 7B are an example of a three-dimensional AAM descriptor of the compound shown in Figs. 6A and 6B. Fig. 7A shows a three-dimensional AAM descriptor of alanine, and Fig. 7B shows a three-dimensional AAM descriptor of valine. In Figs. 7A and 7B, the dark region is a region where the degree of accumulation of amino acids (existence probability) is high. Fig. 8 is a table showing another example of the three-dimensional AAM descriptor of the compound shown in Figs. 6A and 6B, and the three-dimensional AAM descriptor is shown in a direction 1, a direction 2, and a direction 3 (the first, second, and third rows of the table, respectively) which are different from one another. The left columns of the table show the three-dimensional AAM descriptor (the level surface with respect to the threshold), and the right columns of the table show the three-dimensional AAM descriptor (the level surface with respect to the threshold) and the three-dimensional structure of the compound.

<Calculation of 3D AAM descriptor for pocket structure>

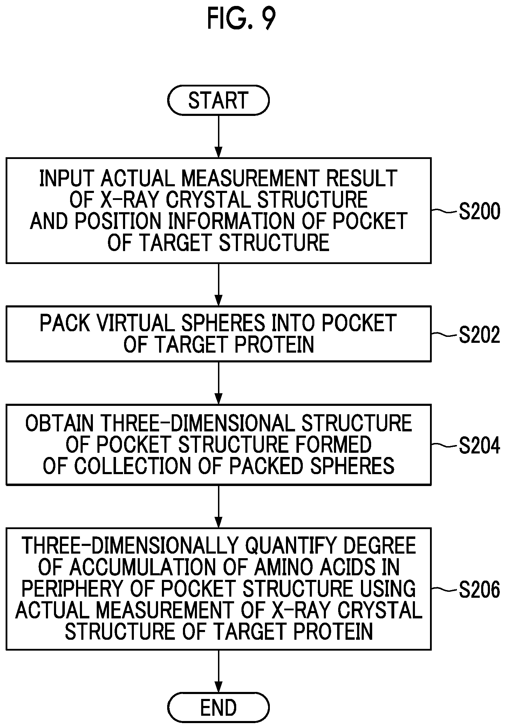

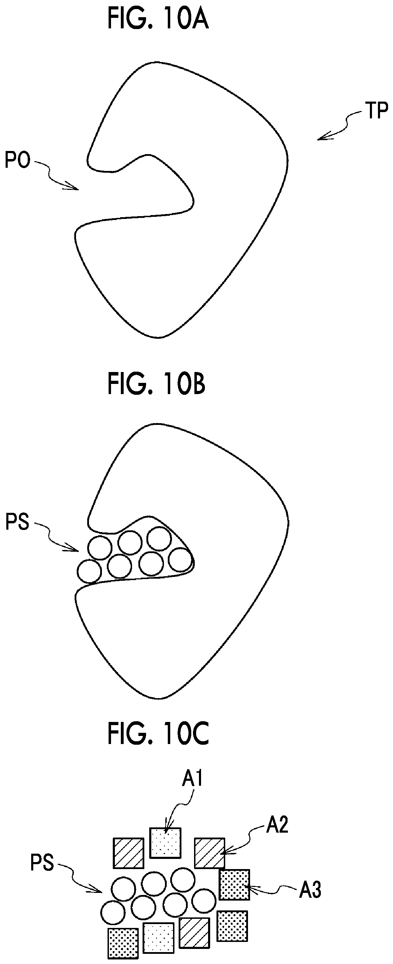

In the screening device 10, a pocket structure that is bound to a target protein instead of a compound is designated as a target structure, and the feature quantity (the three-dimensional AAM descriptor; the second feature quantity) of this pocket structure can be calculated. The pocket structure is a target structure that is bound to a pocket, which is an active site of the target protein, and the "active site" indicates a site where the activity of the target protein is promoted or suppressed by the binding of the pocket structure. Fig. 9 is a flowchart showing a procedure for calculating the three-dimensional AAM descriptor for the pocket structure. Figs. 10A to 10C are conceptual diagrams showing a state of the three-dimensional AAM descriptor for the pocket structure.

In the flowchart of Fig. 9, the information input unit 110 inputs the actual measurement result of the three-dimensional structure of the target protein and the position information of the pocket (Step S200: target structure designating step). Fig. 10A shows a pocket PO in a target protein TP. The pocket structure is designated as the target structure by the process of Step S200.

The feature quantity calculation unit 120 packs a plurality of virtual spheres (a plurality of unit structures having chemical properties) into the pocket of the target protein (Step S202: the target structure designating step or the three-dimensional structure generating step). The "virtual sphere" can be considered to have chemical properties such as a van der Waals radius and an electric charge, and "packing the virtual spheres" can be performed by simulation (for example, a molecular dynamics method). A collection of the packed virtual spheres (three-dimensional structure) can be obtained as a three-dimensional structure of the pocket structure (target structure) in Step S202 (Step S204: three-dimensional structure generating step). Fig. 10B shows an example of a pocket structure PS of the target protein TP.

The feature quantity calculation unit 120 three-dimensionally quantifies the degree of accumulation of one or more kinds of amino acids in the periphery of the pocket structure using actual measurement of the three-dimensional structure of the target protein (Step S206: feature quantity calculating step). Practically, it is possible to read out what kinds of amino acids are accumulated in the periphery of the pocket structure. Fig. 10C shows that three kinds of amino acids A1, A2, and A3 are accumulated in the periphery of the pocket structure PS. Further, the number of amino acids for quantifying the degree of accumulation may be one or more (a plurality of kinds of amino acids may be used). In addition, a predetermined kind of amino acid may be quantified or an amino acid which has been set according to the operation of the user may be quantified. The feature quantity calculation unit 120 stores the calculated three-dimensional AAM descriptor in the storage unit 200 as the three-dimensional AAM descriptor 230 in association with the structure information (structure information 210) and the three-dimensional structure information (three-dimensional structure information 220) of the compound (see Figs. 3 and 4; the storing step). In a case where the invariant AAM descriptor described below has been calculated, the feature quantity calculation unit 120 associates the three-dimensional AAM descriptor with the invariant AAM descriptor.

<Conversion of three-dimensional AAM descriptor into invariant>

The above-described three-dimensional AAM descriptor indicates the degree of three-dimensional accumulation of amino acids. However, in a case where shift or rotation of the center of gravity occurs even though the compounds are the same as each other, the value changes and the data capacity is large because the information is three-dimensional information. Therefore, in the screening device 10 according to the first embodiment, "an invariant AAM descriptor obtained by converting the three-dimensional AAM descriptor into an invariant with respect to rotation and translation of the compound" (the first invariant feature quantity or the second invariant feature quantity) can be calculated in addition to or instead of the three-dimensional AAM descriptor. Further, conversion into an invariant can be performed according to the same procedures in both cases of a compound and a pocket structure. In a case where a three-dimensional AAM descriptor (first feature quantity) of a compound is used, an invariant AAM descriptor (first invariant feature quantity) of the compound is obtained. Further, in a case where a three-dimensional AAM descriptor (second feature quantity) of the pocket structure is used, an invariant AAM descriptor (second invariant feature quantity) of the pocket structure is obtained.

The feature quantity calculation unit 120 calculates $f_a(k)$ using Fourier transform as shown in Equation (2) (invariant conversion step). As described above, "a" is a subscript (1 to 20) indicating the kind of amino acid. Further, "i" is an imaginary unit.

$$f_a(\mathbf{k}) = \int d^3\mathbf{r}(g_a(\mathbf{r}) - 1)e^{i\mathbf{k}\mathbf{r}} \qquad \cdots (2)$$

The feature quantity calculation unit 120 calculates $F_{ab}(s)$ (the first invariant feature quantity or the second invariant feature quantity) that is an invariant AAM descriptor using the $f_a(k)$ according to the following equation (3) (invariant conversion step). In Equation (3), an invariant AAM descriptor is calculated by angular integration of a correlation function using three-dimensional AAM descriptors ($g_a(r)$ and $g_b(r)$) of two different kinds of amino acids (denoted by "a" and "b"). Further, the combination of the two kinds of amino acids used for calculation of the invariant AAM descriptors among twenty kinds of amino acids is not particularly limited.

$$F_{ab}(s) = \int d^3\mathbf{k} f_a(-\mathbf{k}) f_b(+\mathbf{k}) \delta(\mathbf{k}^2 - s) \qquad \cdots (3)$$

In Equation (3), a delta function is used for conversion into an invariant. However, as shown in Equation (4), conversion into an invariant can be performed using an optional function ($h(k^2\text{-}s)$).

$$F_{ab}(s) = \int d^3\mathbf{k} f_a(-\mathbf{k}) f_b(+\mathbf{k}) h(\mathbf{k}^2 - s) \qquad \cdots (4)$$

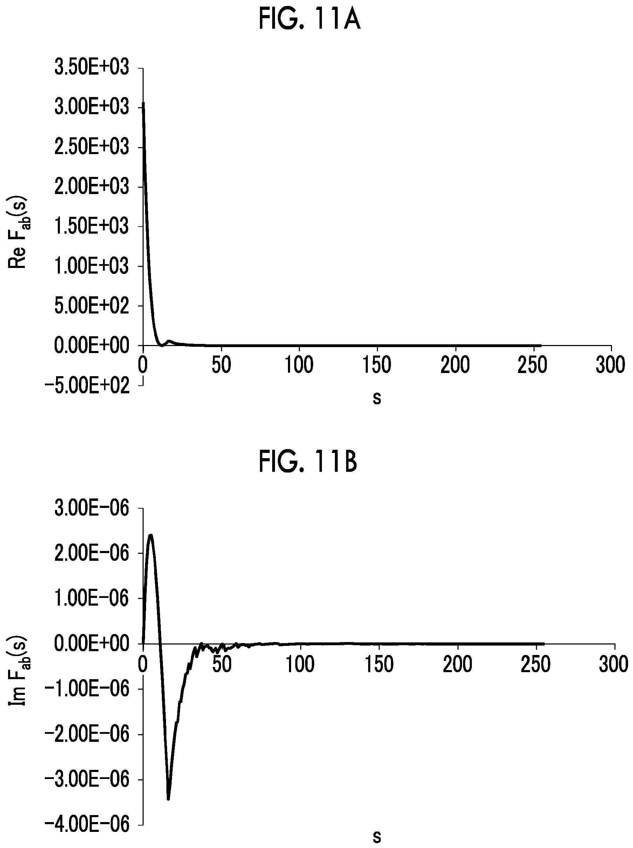

Figs. 11A and 11B show an example of the invariant AAM descriptor calculated in the above-described manner. Fig. 11A shows a real part of $F_{12}(s)$ (an invariant AAM descriptor of amino acids 1 and 2), which is an invariant AAM descriptor, and Fig. 11B shows an imaginary part. In this manner, the conversion into an invariant can be performed while information related to the interaction between amino acids is maintained by performing conversion into an invariant using a three-dimensional AAM descriptor of two different kinds of amino acids so that compound comparison (drug efficacy determination) based on the feature quantities (the first invariant feature quantity and the second invariant feature quantity) can be accurately performed.

The feature quantity calculation unit 120 stores the calculated invariant AAM descriptor in the storage unit 200 as the invariant AAM descriptor 240 in association with the structure information (structure information 210), the three-dimensional structure information (three-dimensional structure information 220), and the original three-dimensional AAM descriptor 230 of the compound (see Figs. 3 and 4; the storing step). In the first embodiment, since the invariant AAM descriptor is calculated using the three-dimensional AAM descriptor of two different kinds of amino acids, a plurality of associations between the three-dimensional AAM descriptor and the invariant AAM descriptor may be present.

<Evaluation of effectiveness of invariant AAM descriptor>

The effectiveness of the invariant AAM descriptor calculated by the above-described process will be described.

<Example of activity of compound having similar invariant AAM descriptor>

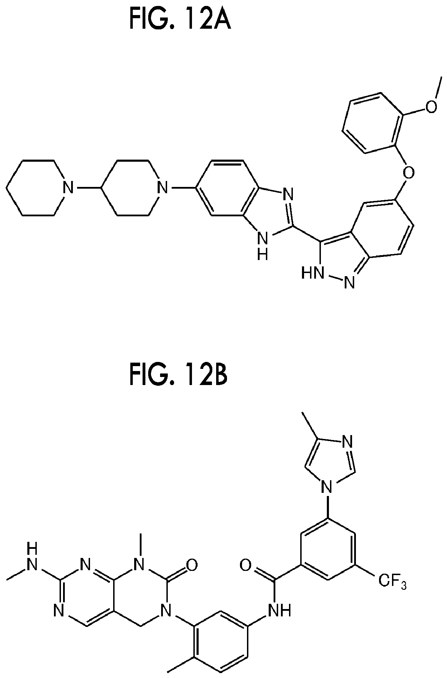

Fig. 12A shows a structural formula of a ligand for a protein ABL1 (an example of a target protein). The binding force of this ligand is at a level of 1 $\mu$M with an IC50 (50% inhibitory concentration). Meanwhile, Fig. 12B shows a structural formula of a compound having almost the same invariant AAM descriptor as that of the ligand. In a case where the activity of this compound is actually measured, the activity is at the same level as that of the ligand. That is, Figs. 11A and 11B are an example showing that compounds having similar invariant AAM descriptors have similar drug efficacies. As described above, according to the first embodiment, a feature quantity (invariant AAM descriptor) accurately showing the chemical properties of the target structure can be obtained.

<Easiness of finding hits>

The easiness of finding hits using invariant AAM descriptors is evaluated according to the following procedures 1 to 5.

(Procedure 1) X hit compounds and Y non-hit compounds are mixed with a certain protein (target protein).
(Procedure 2) Invariant AAM descriptors of all (X + Y) compounds are calculated.
(Procedure 3) The similarity of each descriptor is calculated.
(Procedure 4) The (X + Y) compounds are divided into teams based on the similarities of the invariant AAM descriptors.

(Procedure 5) It is checked whether the teams in which hits are collected are mechanically generated.

As a result of division of 10,933 compounds having 183 hits (a hit content of 1.6%) for the protein ABL1 (kinase) into teams by according to the above-described procedures, the number of teams is 221. A certain team contains 16 hits and 14 other compounds, and the hit content is 53.3%. Further, this team also contains the compound shown in Fig. 12A and the compound shown in Fig. 12B. In a case where the fingerprint, which is a descriptor of the related art, is used, the similarity of these compounds is 25%. It is recognized as not being a hit even though it is a hit originally. As described above, in a case where the invariant AAM descriptor according to the first embodiment of the present invention is used in the above-described team, it was found that hits which are not collected in a case of using the fingerprint belong to the same team.

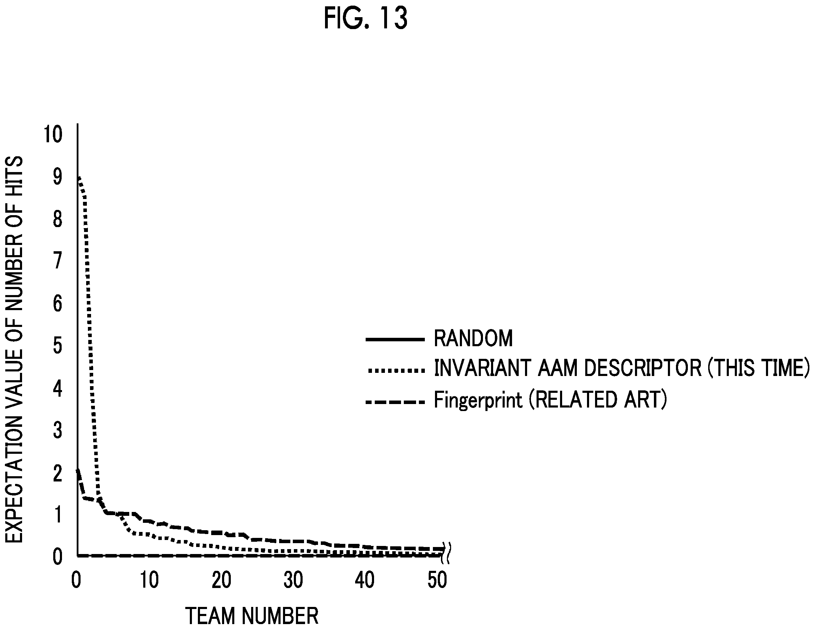

Fig. 13 shows the results of acquiring the easiness of finding hits (= expectation value; number of hits $\times$ hit content) for each of the 221 teams described above. For comparison, the results obtained in a case where the teams are randomly divided using the fingerprint are shown. Based on these results, it was found that in a case where the invariant AAM descriptor is used for the above-described compound group, teams having hits more than the hits of the randomly divided teams or the teams divided using the fingerprint are generated. In Fig. 13, the team numbers vary depending on the team division method (random, the invariant AAM descriptor, the fingerprint), and thus the superiority of the team division is determined not by comparing the expectation values with the same team number but by verifying "whether teams with high expectation values (having more hits) are included or not".

<Hit search time (part 1)>

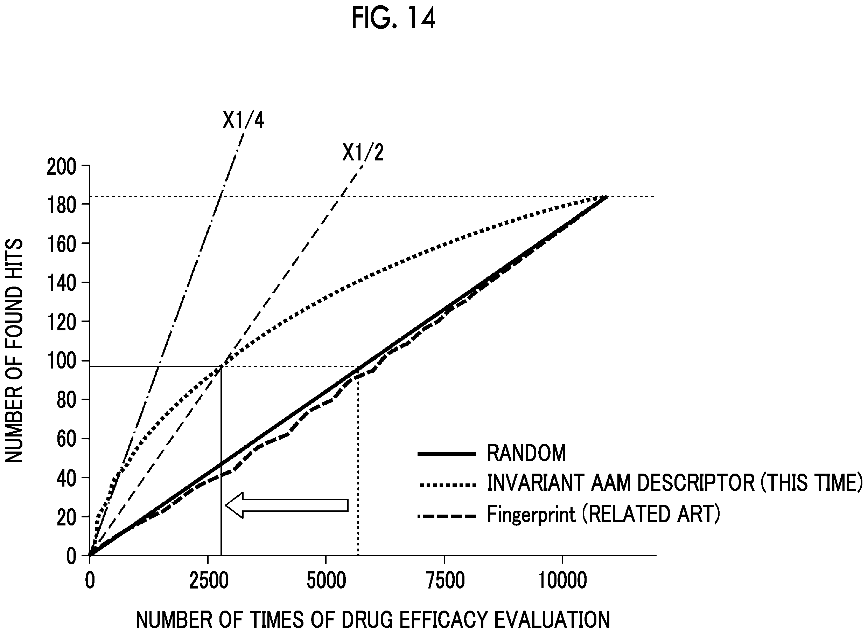

Fig. 14 is a graph showing simulation results of Importance Sampling for the above-described compound group. In a case where the invariant AAM descriptor according to the first embodiment is used, the hit search time (the number of times of drug efficacy evaluations for finding the number of same hits) compared with a case of the random team division is reduced to approximately one-half in a case of 50% search and reduced to one-fourth in a case of 25% search. Meanwhile, in a case of the team division using the fingerprint, the hit search time is not shortened. Further, the simulation of the Importance Sampling here indicates a method of providing variables showing the priority for each team and collecting more hits with a smaller number of times of measurements while updating the variables such that the priority of the team from which hits have found is raised and the priority of the team that does not have hits is lowered for each measurement. Fig. 14 shows an example in which an NB method (NB: Naive Bayes), which is a kind of machine learning method is used for controlling the priority, but the present invention is not limited thereto.

<Hit search time (part 2)>

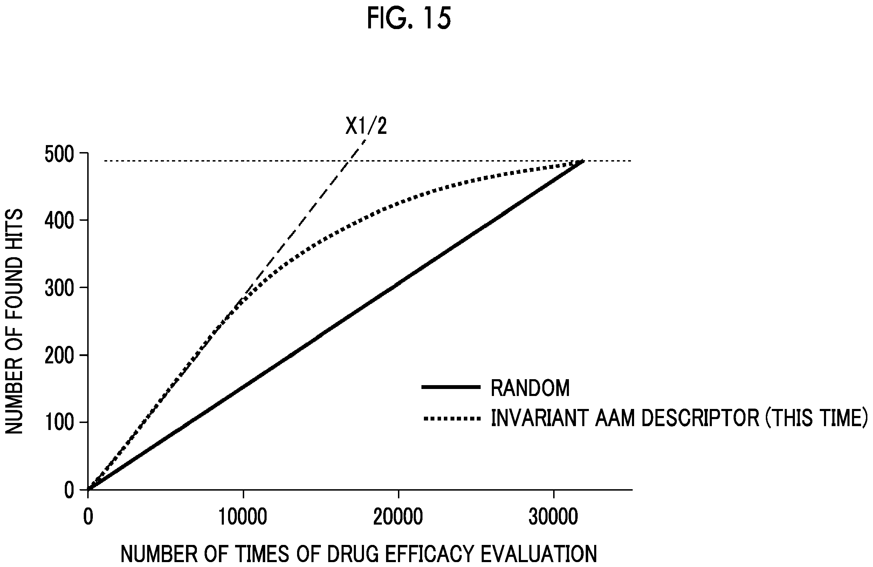

Fig. 15 shows a simulation result of Importance Sampling for 32,464 compounds (having 483 hits) for a protein AA2AR, similar to the case of (part 1). The protein AA2AR is a membrane protein from which an X-ray crystal structure is difficult to obtain, but the hit search time is reduced to approximately 50% in a case of 50% search even with such a membrane protein.

According to the invariant AAM descriptor described above, since compounds having similar descriptors exhibit similar drug efficacies (binding to a target protein), the chemical properties of the target structure (a compound or a pocket structure) are accurately exhibited. According to the invariant AAM descriptor in which the three-dimensional AAM descriptor is converted into an invariant, the data capacity can be easily reduced while comparison (determination of the drug efficacy) of compounds based on the descriptor is accurately performed by performing conversion into an invariant using the three-dimensional AAM descriptor of two kinds of different amino acids. Further, according to the invariant AAM descriptor, hits are easily found, and the search can be sped up.

<Effects of feature quantity calculating method and feature quantity calculating program>

As described above, the screening device 10 according to the first embodiment is capable of calculating the feature quantity (the three-dimensional AAM descriptor or the invariant AAM descriptor) accurately showing the chemical property of the target structure using the feature quantity calculating method and the feature quantity calculating program according to the embodiment of the present invention.

<Extraction of target compound (screening)>

Extraction of a target compound (pharmaceutical candidate compound) from a plurality of compounds using the above-described three-dimensional AAM descriptor and invariant AAM descriptor will be described. A target compound is extracted in a mode (first mode) in which the extraction is carried out based on the descriptor (the three-dimensional AAM descriptor or the invariant AAM descriptor) of the ligand or in a mode (second mode) in which the extraction is carried out based on the descriptor (the three-dimensional AAM descriptor or the invariant AAM descriptor) of the pocket structure of the target protein. The mode for extraction can be selected from the above-described modes according to the operation of the user via the operation unit 400.

<Screening of ligand input>

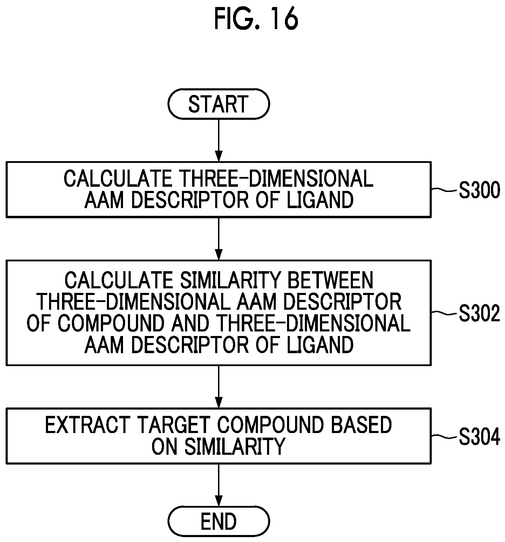

Fig. 16 is a flowchart showing a procedure for screening using a three-dimensional AAM descriptor of a ligand. After the start of the process, the feature quantity calculation unit 120 calculates a three-dimensional AAM descriptor of the ligand (Step S300: the feature quantity calculating step). Since the ligand is a compound whose binding to the target protein has been confirmed, the three-dimensional AAM descriptor in Step S300 can be calculated according to the procedure shown in the flowchart of Fig. 5.

As described above with reference to Fig. 4, in the screening device 10, the three-dimensional structure of the compound formed of a plurality of atoms and the three-dimensional AAM descriptor (the first feature quantity) corresponding to the three-dimensional structure are stored in the storage unit 200 in association with each other for each of the plurality of compounds. The similarity calculation unit 130 calculates the similarity between the three-dimensional AAM descriptor of the compound and the three-dimensional AAM descriptor of the ligand calculated in Step S300 (Step S302: the similarity calculating step). After the calculation of the similarity, the compound extraction unit 140 extracts the target compound based on the similarity (Step S304: the target compound extracting step). As described above, in a case where three-dimensional AAM descriptors are similar, since similar drug efficacies (binding to the target protein) are exhibited, a compound having drug efficacy similar to that of the ligand (that is, a target compound serving as a pharmaceutical candidate) can be extracted by using the similarity of the three-dimensional AAM descriptor. Further, the extraction of the target compound based on the similarity (Step S304) can be specifically performed by "extracting a compound having a similarity greater than or equal to the threshold" or "extracting a compound in a descending order of the similarity".

Fig. 16 shows the procedure for screening using a three-dimensional AAM descriptor, but the screening using an invariant AAM descriptor can also be performed in the same manner as described above. Specifically, the feature quantity calculation unit 120 calculates the invariant AAM descriptor (the first invariant feature quantity) of the ligand according to the procedure of Fig. 5 and Equations (2) and (3), and the similarity calculation unit 130 calculates the similarity between the compound stored in the storage unit 200 and the invariant AAM descriptor. After the calculation of the similarity, the compound extraction unit 140 extracts the target compound based on the similarity. Specifically, the target compound can be extracted based on the similarity in the same manner as the extraction of the three-dimensional AAM descriptor.

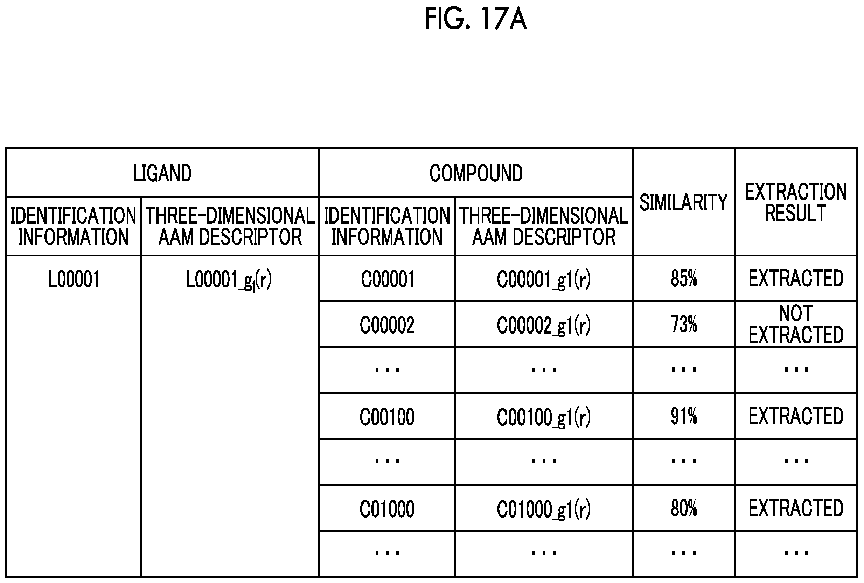

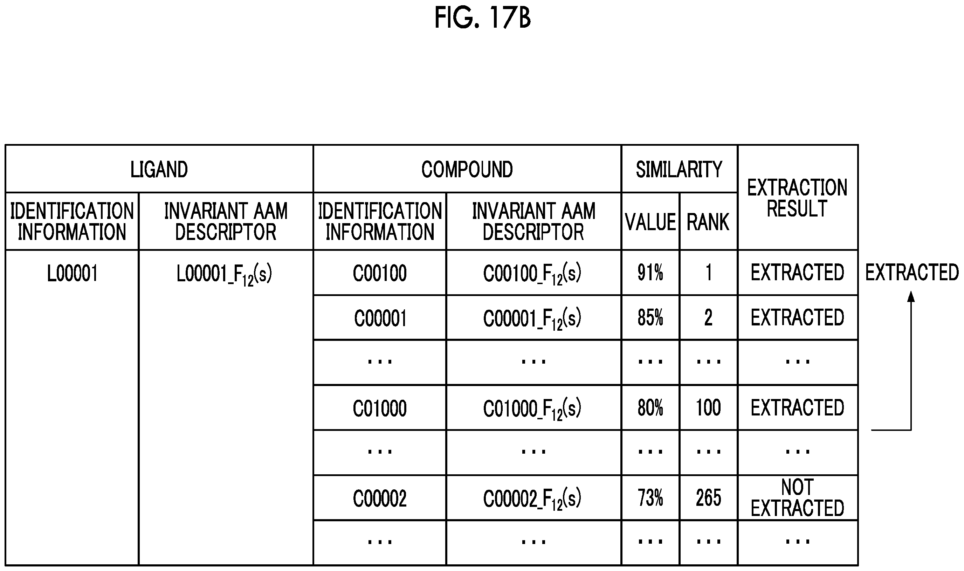

Figs. 17A and 17B are tables showing an example of a screening result of ligand input. Fig. 17A shows the result in a case of "extraction of a compound having a similarity greater than or equal to the threshold" using a three-dimensional AAM descriptor, and Fig. 17B shows the result in a case of "extraction of a compound in a descending order of the similarity" using an invariant AAM descriptor. Further, in Fig. 17A, the compound is extracted based on a three-dimensional AAM descriptor ($g_1(r)$) of the amino acid 1, but the compound may be extracted based on the three-dimensional AAM descriptor (for example, $g_2(r)$) of other amino acids (amino acids 2 to 20). In addition, the similarities (the similarity between the values of $g_1(r)$ and the similarity between the values of $g_2(r)$) of a plurality of three-dimensional AAM descriptors (for example, $g_1(r)$ and $g_2(r)$) of different amino acids are respectively calculated, and compounds may be extracted based on the results. The number of kinds of the three-dimensional AAM descriptors used for extraction of a compound may be one, but extraction of a compound based on the similarity can be accurately performed using a plurality of kinds of three-dimensional AAM descriptors. Further, in a case where a plurality of kinds of three-dimensional AAM descriptors are used, the combination of amino acids among the descriptors is not particularly limited (for example, a combination of $g_1(r)$ and $g_2(r)$ or a combination of $g_3(r)$ and $g_4(r)$ may be used).

Similarly, in Fig. 17B, a compound is extracted based on an invariant AAM descriptor ($F_{12}(s)$) of the amino acids 1 and 2, but the combination of amino acids used for calculation of the invariant AAM descriptor may vary (for example, $F_{34}(s)$ with amino acids 3 and 4). Further, a compound may be extracted based on a plurality of invariant AAM descriptors (for example, $F_{12}(s)$ and $F_{34}(s)$) with different combinations of amino acids (for example, the similarity between the values of $F_{12}(s)$ and the similarity between the values of $F_{34}(s)$ are used). The number of kinds of the invariant AAM descriptors used for extraction of a compound may be one, but extraction of a compound based on the similarity can be accurately performed using a plurality of kinds of invariant AAM descriptors. Further, in a case where a plurality of kinds of invariant AAM descriptors are used, the combination of amino acids among the descriptors is not particularly limited (for example, a combination of $F_{12}(s)$ and $F_{34}(s)$ or a combination of $F_{12}(s)$ and $F_{13}(s)$ may be used). The processing unit 100 (the feature quantity calculation unit 120, the similarity calculation unit 130, or the compound extraction unit 140) may determine which amino acid is to be used for calculation of the descriptor and the similarity according to the user's instruction via the operation unit 400, but the determination may be made by the processing unit 100 regardless of the user's instruction.

Further, the threshold of the similarity is set to 80% in Fig. 17A, and the number of times of extraction is set to 100 in Fig. 17B, but these values are merely examples. The threshold and the number of times of extraction can be set according to the conditions, for example, the accuracy of screening. The setting can be performed in response to a user input via the operation unit 400. Further, "a compound may be extracted in a descending order of the similarity" in a case where a three-dimensional AAM descriptor is used in contrast to Figs. 17A and 17B, and "a compound having a similarity greater than or equal to the threshold may be extracted" in a case where an invariant AAM descriptor is used. The compound extraction unit 140 stores the extraction result as shown in Figs. 17A and 17B in the storage unit 200 as the compound extraction result 260 (see Fig. 3).

<Screening of target protein input>

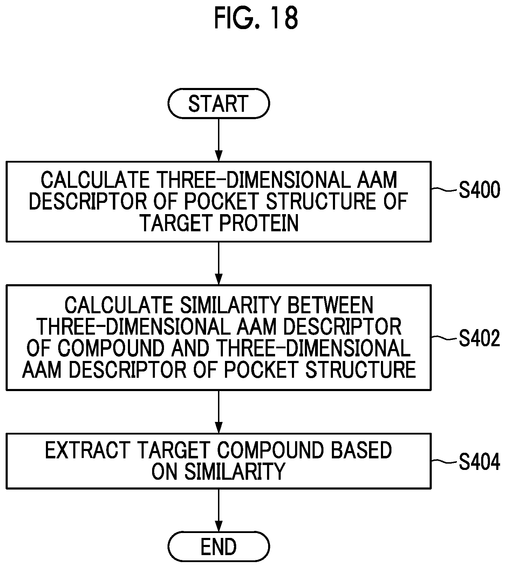

Fig. 18 is a flowchart showing a procedure for screening of a pocket structure of a target protein using a three-dimensional AAM descriptor. After the start of the process, the feature quantity calculation unit 120 calculates a three-dimensional AAM descriptor of the pocket structure of the target protein (Step S400: the feature quantity calculating step). The three-dimensional AAM descriptor in Step S400 can be calculated according to the procedure shown in the flowchart of Fig. 9. The similarity calculation unit 130 calculates the similarity between the three-dimensional AAM descriptor of the compound and the three-dimensional AAM descriptor of the pocket structure calculated in Step S400 (Step S402: the similarity calculating step). After the calculation of the similarity, the compound extraction unit 140 extracts the target compound based on the similarity (Step S404: the target compound extracting step). Similar to the case of the ligand input described above, the extraction of the target compound based on the similarity (Step S404) can be specifically performed by "extracting a compound having a similarity greater than or equal to the threshold" or "extracting a compound in a descending order of the similarity".

Even in a case of using the invariant AAM descriptor, a target compound can be extracted according to the same procedure as in the flowchart of Fig. 18.

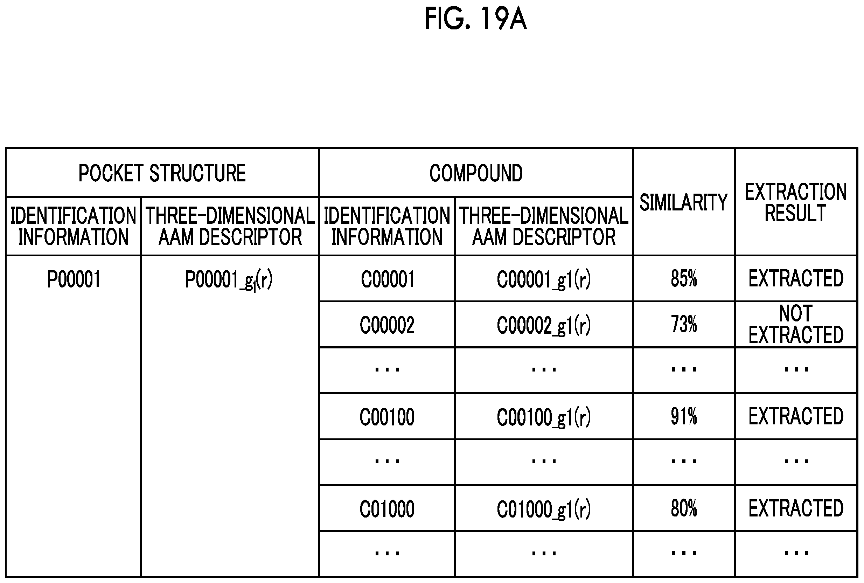

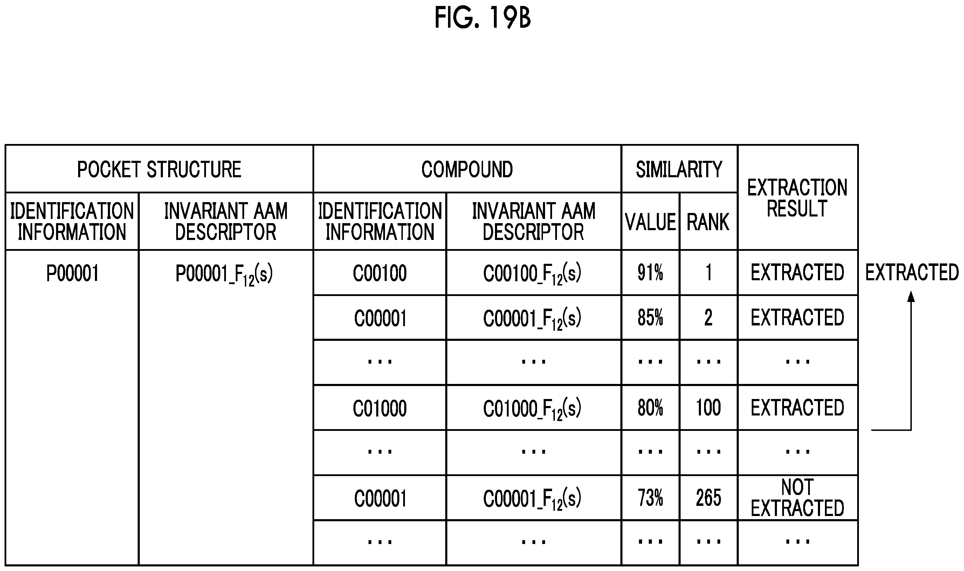

Figs. 19A and 19B are tables showing an example of a screening result of target protein input. Fig. 19A shows the result in a case of "extraction of a compound having a similarity greater than or equal to the threshold" using a three-dimensional AAM descriptor, and Fig. 19B shows the result in a case of "extraction of a compound in a descending order of the similarity" using an invariant AAM descriptor. The threshold of the similarity and the number of times of extraction can be set according to the conditions, for example, the accuracy of screening. The setting can be performed in response to a user input via the operation unit 400. Further, "a compound may be extracted in a descending order of the similarity" in a case where a three-dimensional AAM descriptor is used in contrast to Figs. 19A and 19B, and "a compound having a similarity greater than or equal to the threshold may be extracted" in a case where an invariant AAM descriptor is used.

In a case of screening for the target protein input, the kind of amino acid may be changed in the same manner as in the case of screening for the ligand input (see Figs. 17A and 17B and the description), or a plurality of descriptors of different amino acids (the three-dimensional AAM descriptor and the invariant AAM descriptor) may be used. The number of kinds of the descriptors used for extraction of a compound may be one, but extraction of a compound based on the similarity can be accurately performed using a plurality of kinds of descriptors. Further, in a case where a plurality of kinds of descriptors are used, the combination of amino acids among the descriptors is not particularly limited. The processing unit 100 (the feature quantity calculation unit 120, the similarity calculation unit 130, or the compound extraction unit 140) may determine which amino acid is to be used for calculation of the descriptor and the similarity according to the user's instruction via the operation unit 400, but the determination may be made by the processing unit 100 regardless of the user's instruction.

The compound extraction unit 140 stores the extraction result as shown in Figs. 19A and 19B in the storage unit 200 as the compound extraction result 260 (see Fig. 3).

<Effect of screening device>

As described above, the screening device 10 according to the first embodiment is capable of efficient screening of a pharmaceutical candidate compound according to the screening method and the screening program according to the embodiment of the present invention using the feature quantity (the three-dimensional AAM descriptor or the invariant AAM descriptor) calculated by the feature quantity calculating method and the feature quantity calculating program according to the embodiment of the present invention.

<Second Embodiment

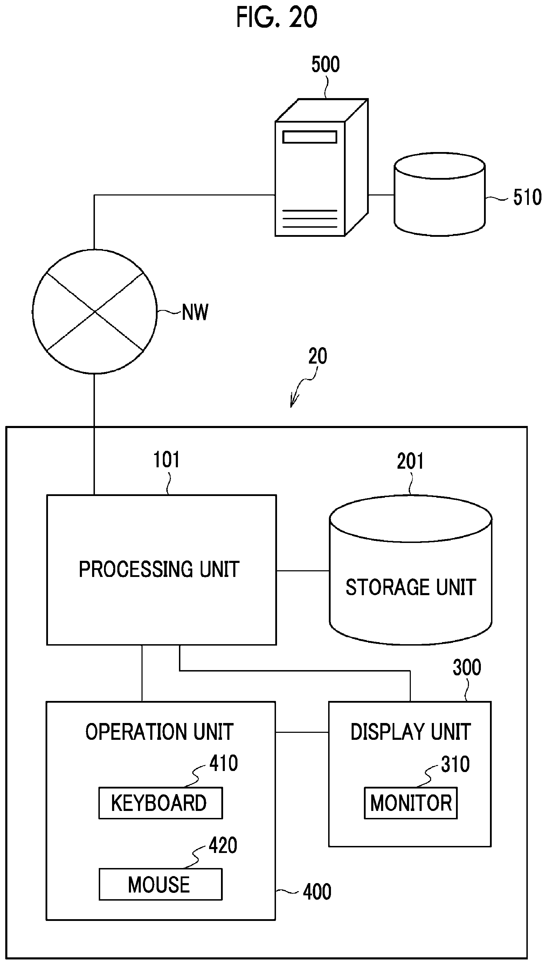

A compound creating device according to a second embodiment of the present invention will be described. Fig. 20 is a block diagram showing a configuration of a compound creating device 20 (a feature quantity calculating device or a compound creating device). Further, the same elements as those in the first embodiment are denoted by the same reference numerals, and detailed description thereof will not be provided.

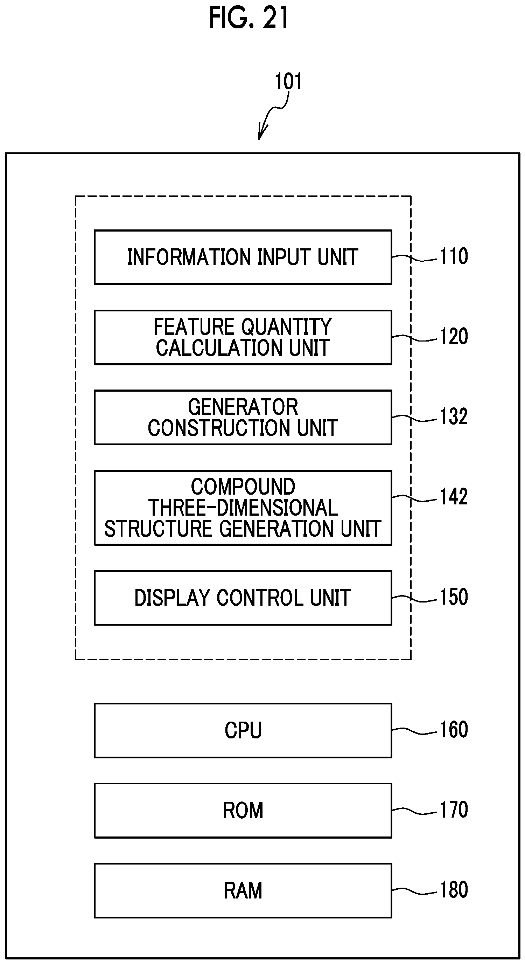

The compound creating device 20 includes a processing unit 101. The processing unit 101 is formed as shown in Fig. 21 and includes an information input unit 110, a feature quantity calculation unit 120 (feature quantity calculation unit), a generator construction unit 132 (generator construction unit), a compound three-dimensional structure generation unit 142 (compound three-dimensional structure generation unit), and a display control unit 150. The functions of the information input unit 110, the feature quantity calculation unit 120, and the display control unit 150 are respectively the same as the information input unit 110, the feature quantity calculation unit 120, and the display control unit 150 in the above-described screening device 10. The functions of these units can be realized using various processors in the same manner as described above in the section of the screening device 10.

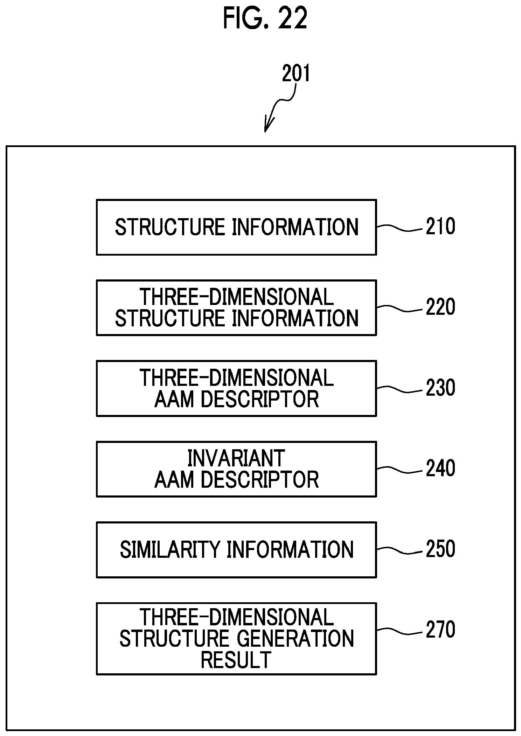

Fig. 22 is a diagram showing information stored in the storage unit 201. The storage unit 201 stores a three-dimensional structure generation result 270 instead of the compound extraction result 260 in the screening device 10. The information stored in the storage unit 201 is stored in association as described above with reference to Fig. 4.

<Generation of three-dimensional structure of target compound>

Generation of a three-dimensional structure of a target compound (pharmaceutical candidate compound) using the above-described three-dimensional AAM descriptor and invariant AAM descriptor will be described. Since search is not performed in the generation of a three-dimensional structure of a target compound using the compound creating device 20, the three-dimensional structure of the compound can be generated even in a case of "no solution was found as the result of screening search", and thus the three-dimensional structure of the pharmaceutical candidate compound can be efficiently created. A three-dimensional structure is generated in a mode in which the generation is carried out based on the descriptor (the three-dimensional AAM descriptor or the invariant AAM descriptor) of the ligand or in a mode in which the generation is carried out based on the descriptor (the three-dimensional AAM descriptor or the invariant AAM descriptor) of the pocket structure of the target protein. The mode for generation of a three-dimensional structure can be selected from the above-described modes according to the operation of the user via the operation unit 400.

<Generation of three-dimensional structure in case of ligand input>

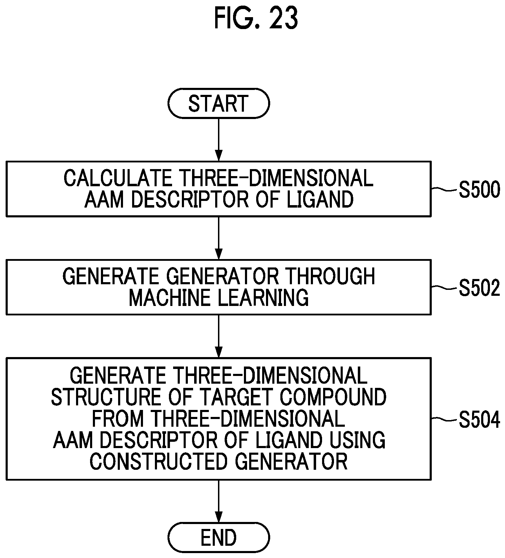

Fig. 23 is a flowchart showing a procedure for generating a three-dimensional structure in a case of ligand input. After the start of the process, the feature quantity calculation unit 120 calculates a descriptor (a three-dimensional AAM descriptor) of the ligand (Step S500: the target structure designating step, the three-dimensional structure generating step, or the feature quantity calculating step). The process of Step S500 can be performed using the feature quantity calculating method and the feature quantity calculating program according to the embodiment of the present invention in the same manner as in the first embodiment (see Figs. 5 to 8 and the description of these drawings).

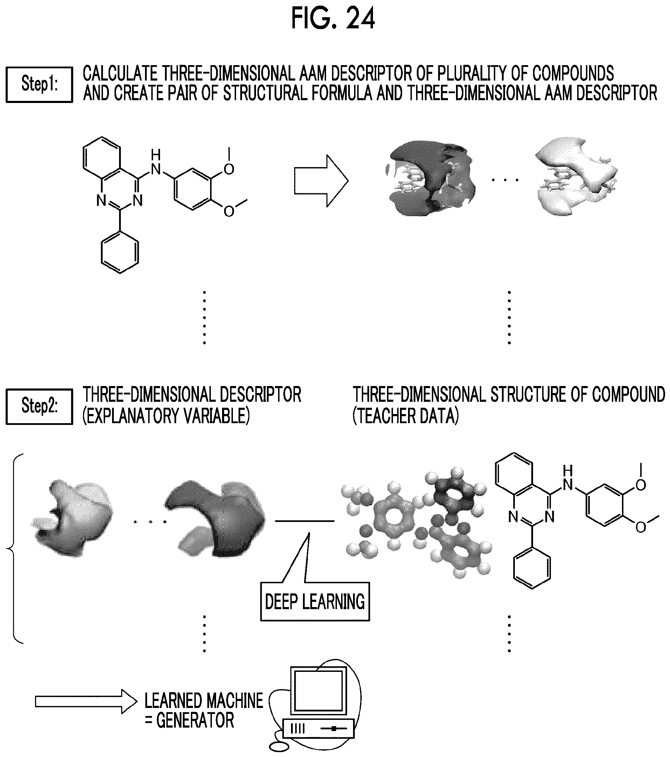

In Step S502, the generator construction unit 132 constructs a generator through machine learning (a generator constructing step). Hereinafter, the process of Step S502 will be described with reference to Fig. 24. (Step 1) The feature quantity calculation unit 120 calculates three-dimensional AAM descriptors of a plurality of compounds and creates a pair (three-dimensional data) of a structural formula (a three-dimensionalized structural formula) and a three-dimensional AAM descriptor. (Step 2) The generator construction unit 132 constructs a generator through machine learning (deep learning) using a three-dimensional structure of a compound as teacher data and a three-dimensional AAM descriptor (first feature quantity) as an explanatory variable. The method of deep learning is not limited to a specific method, and a simple fully-coupled neural network or a convolutional neural network (CNN) may be employed. However, since the generation accuracy of the three-dimensional structure depends on the learning method to be used, it is preferable to select a learning method according to the condition for generating the three-dimensional structure and the condition such as the required accuracy.

After the completion of the processes of Steps 1 and 2 described above, the process returns to the flowchart of Fig. 23. The compound three-dimensional structure generation unit 142 generates a three-dimensional structure (three-dimensionalized structural formula) of the target compound (hit) from the three-dimensional AAM descriptor of the ligand using the constructed generator (Step S504: the compound three-dimensional structure generating step). In this manner, the three-dimensional structure of a compound having drug efficacy (binding to a target protein) similar to that of a ligand, that is, a pharmaceutical candidate compound can be obtained. Further, a plurality of three-dimensional structures that provide the same three-dimensional AAM descriptor may be present. The compound three-dimensional structure generation unit 142 stores the generated three-dimensional structure in the storage unit 201 in association with the three-dimensional AAM descriptor (the three-dimensional AAM descriptor 230) as the three-dimensional structure generation result 270 (see Fig. 22). The display control unit 150 may display the generated three-dimensional structure on the monitor 310 in response to the user's instruction via the operation unit 400.

Further, in the procedure described above, the number of kinds of amino acids used for calculation of the three-dimensional AAM descriptor for machine learning may be one or plural. However, the accuracy of the generated three-dimensional structure can be improved by calculating the three-dimensional AAM descriptor of a plurality of kinds of amino acids and providing the result for learning. Further, in a case where a plurality of three-dimensional AAM descriptors having different kinds of amino acids are used, the combination of amino acids among the descriptors is not particularly limited. The processing unit 100 (the feature quantity calculation unit 120, the similarity calculation unit 130, or the compound extraction unit 140) may determine which amino acid is to be used for calculation of the three-dimensional AAM descriptor and for provision for learning according to the user's instruction via the operation unit 400, but the determination may be made by the processing unit 100 regardless of the user's instruction.

<Example of generation of three-dimensional structure>

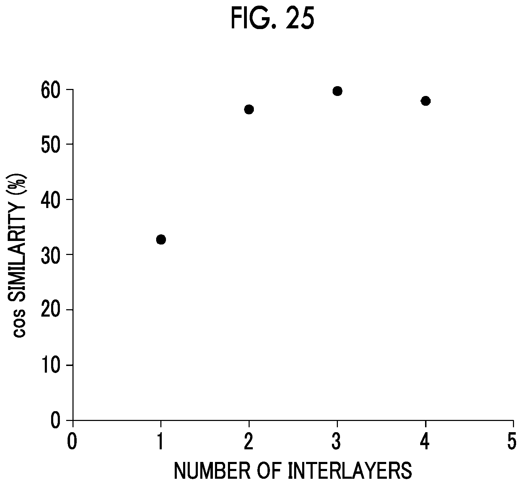

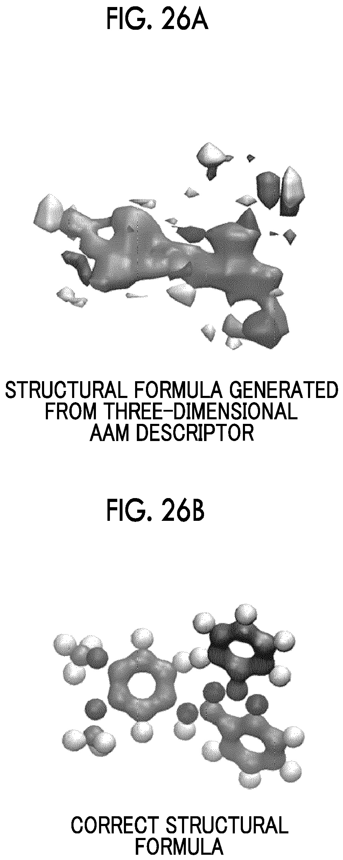

An example of a three-dimensional structure generated using a generator constructed through machine learning will be described. In this example, 1,800 compounds among the library compounds are learned according to the above-described method using a simple fully-coupled neural network, and the extent to which the three-dimensional structures of the remaining 200 compounds can be reproduced is examined. The results are shown in Fig. 25. In a case where the number of interlayers in the neural network is increased, the average cos similarity is 59%. Figs. 26A and 26B are diagrams showing a three-dimensional structure (structural formula) and a correct structural formula (Figs. 26A and 26B, respectively) generated from a three-dimensional AAM descriptor as an example showing such similarity.

<Relationship between features of teacher data and generated three-dimensional structure>

The three-dimensional structure generated according to the above-described procedure is affected by the features of the compound provided as teacher data. Therefore, by selecting the features of the compound to be provided as teacher data, a compound having a three-dimensional structure with different features can be generated. For example, a compound having drug efficacy similar to that of a ligand and having a three-dimensional structure that is easy to synthesize can be generated by providing, as teacher data, a three-dimensional AAM descriptor of a compound having a three-dimensional structure that is easy to synthesize. It is possible to select which compound to be provided for the three-dimensional AAM descriptor as the teacher data according to the features of the compound intended to be generated.

<Generation of three-dimensional structure using invariant AAM descriptor>

In Figs. 23 to 26, the generation of the three-dimensional structure using the three-dimensional AAM descriptor has been described. Meanwhile, similarly to the case of using the three-dimensional AAM descriptor, the three-dimensional structure of the target compound can be generated through machine learning (deep learning) using the invariant AAM descriptor as teacher data and the three-dimensional structure (three-dimensionalized structural formula) as an explanatory variable even in a case of using the invariant AAM descriptor (first invariant feature quantity).

<Generation of three-dimensional structure in case of target protein input>

The compound creating device 20 is capable of generating a three-dimensional structure of a target compound by setting a target protein as an input, in addition to the generation of the three-dimensional structure by ligand input. Even in this case, similarly to the case of ligand input, generation of a three-dimensional structure can be performed using a three-dimensional AAM descriptor (second feature quantity) and generation of a three-dimensional structure can be performed using an invariant AAM descriptor (second invariant feature quantity).

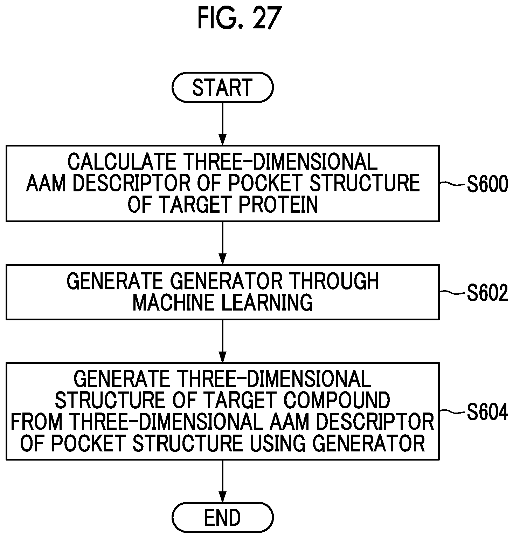

Fig. 27 is a flowchart showing a procedure for generating a three-dimensional structure in a case of setting a target protein as an input (a three-dimensional AAM descriptor is set to be used). After the start of the process, the feature quantity calculation unit 120 calculates the three-dimensional AAM descriptor (second feature quantity) of the pocket structure of the target protein (Step S600: the target structure designating step, the three-dimensional structure generating step, or the feature quantity calculating step). The process of Step S600 can be performed using the feature quantity calculating method according to the embodiment of the present invention, similarly to the first embodiment (see Figs. 9 and 10 and the description of these drawings).

In Step S602, the generator construction unit 132 constructs a generator through machine learning (deep learning) similar to the case of ligand input (the generator constructing step). The construction of the generator can be performed in the same manner as in Steps 1 and 2 described above. The compound three-dimensional structure generation unit 142 generates a three-dimensional structure (three-dimensionalized structural formula) of the target compound (hit) from the three-dimensional AAM descriptor of the pocket structure using the constructed generator (Step S604: the compound three-dimensional structure generating step). In this manner, the three-dimensional structure of a compound having drug efficacy (binding to a target protein) similar to that of the pocket structure, that is, a pharmaceutical candidate compound can be obtained. Further, a plurality of three-dimensional structures that provide the same three-dimensional AAM descriptor may be present. The compound three-dimensional structure generation unit 142 stores the generated three-dimensional structure in the storage unit 201 in association with the three-dimensional AAM descriptor (the three-dimensional AAM descriptor 230) as the three-dimensional structure generation result 270 (see Fig. 22). The display control unit 150 may display the generated three-dimensional structure on the monitor 310 in response to the user's instruction via the operation unit 400.

<Effects of compound creating device>

As described above, the compound creating device 20 according to the second embodiment efficiently creates a three-dimensional structure of a pharmaceutical candidate compound according to the feature quantity calculating method and the compound creating program according to the embodiment of the present invention using the feature quantity (the three-dimensional AAM descriptor or the invariant AAM descriptor) calculated using the compound creating method and the feature quantity calculating program according to the embodiment of the present invention.

<Third embodiment

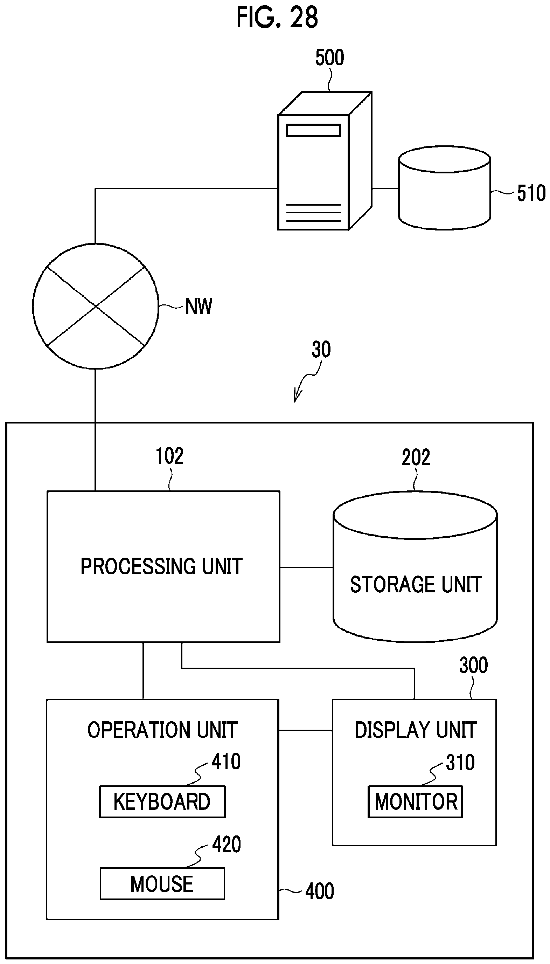

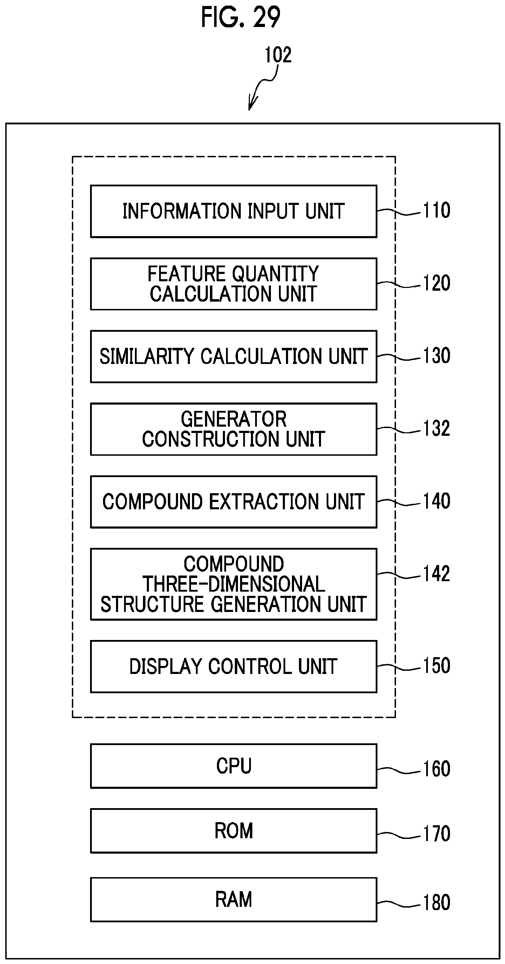

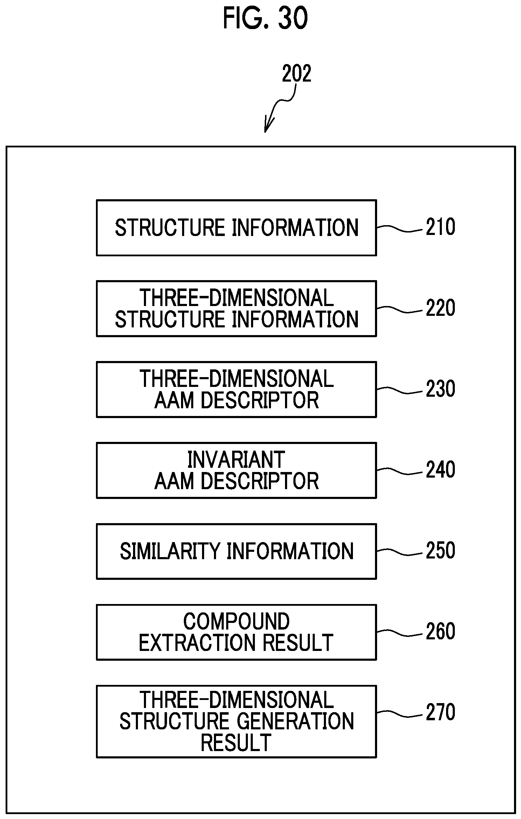

The first embodiment described above is an aspect in which the calculation of the feature quantity and screening based on the calculation are performed, and the second embodiment is an aspect in which the calculation of the feature quantity and creation of the three-dimensional structure of the target compound based on the calculation are performed. In addition to the calculation of the feature quantity, both the screening and the creation of a three-dimensional structure of the target compound may be performed. Therefore, a pharmaceutical candidate compound search device 30 (the feature quantity calculating device, the screening device, or the compound creating device; see Fig. 28) according to the third embodiment includes a processing unit 102 shown in Fig. 28 in place of the processing unit 100 of the screening device 10 shown in Fig. 1 or the processing unit 101 of the compound creating device 20 shown in Fig. 20. As shown in Fig. 29, the processing unit 102 includes a feature quantity calculation unit 120 (feature quantity calculation unit), a similarity calculation unit 130 (similarity calculation unit), a generator construction unit 132 (generator construction unit), a compound extraction unit. 140 (compound extraction unit), and a compound three-dimensional structure generation unit 142 (compound three-dimensional structure generation unit) and can perform calculation of a feature quantity, screening, and creation of a three-dimensional structure of a compound. In addition, the pharmaceutical candidate compound search device 30 stores information related to the above-described performance in the storage unit 202. Specifically, as shown in Fig. 30, information (see Figs. 3 and 22) stored in the storage unit 200 and the storage unit 201 is stored in the storage unit 202 together.

Since other elements are the same as those of the screening device 10 shown in Fig. 1 and the compound creating device 20 shown in Fig. 20, the elements are denoted by the same reference numerals and the detailed description thereof will not be provided.

With the above-described configuration, also in the pharmaceutical candidate compound search device 30 according to the third embodiment, the feature quantity accurately showing the chemical properties of the target structure is calculated, screening of a pharmaceutical candidate compound is efficiently performed, and a three-dimensional structure of the pharmaceutical candidate compound can be efficiently created, similarly to the screening device 10 and the compound creating device 20.

The embodiments of the present invention have been described above, but the present invention is not limited to the above-described embodiments, and various modifications can be made without departing from the spirit of the present invention as exemplified below.

<Target of drug that can be treated>

In the present invention, deoxyribonucleic acid (DNA), ribonucleic acid (RNA), cell membranes, and polysaccharides can be treated as the targets of drugs. However, in the first to third embodiments, it is necessary to change the amino acid to another one. Specifically, an amino acid is changed to a nucleic acid base in a case of DNA, an amino acid is changed to a nucleic acid base in a case of RNA, an amino acid is changed to a lipid molecule in a case of cell membranes, and an amino acid is changed to a monosaccharide molecule in a case of polysaccharides. In the description below, the reason why DNA, RNA, cell membranes, and polysaccharides can be treated with this change in the present invention will be described. Proteins, DNA, RNA, cell membranes, and polysaccharides are collectively referred to as biopolymers and are made up of unique building blocks. Specifically, the building block of proteins is an amino acid, the building block of DNA is a nucleic acid base, the building block of RNA is similarly a nucleic acid base, the building block of cell membranes is a lipid molecule, and the building block of polysaccharides is a monosaccharide molecule. Since DNA, RNA, cell membranes, and polysaccharides, which are biopolymers other than proteins also have pockets that are active sites similarly to the proteins, even in a case where a drug target (target biopolymer) is DNA, RNA, cell membranes and polysaccharides, the present invention can deal with this by changing amino acids to the building blocks of the targets in the first to third embodiments shown in the case of proteins. Further, water can be considered at the time of quantifying the degree of accumulation of amino acids, nucleic acid bases, lipid molecules, and monosaccharide molecules in the periphery of a compound or a pocket structure.

<Activities that can be treated>

In the present invention, in addition to the typical activity which is the "activity of a target biomolecule alone by a compound", the "activity of a cell, which is a composite formed of other biomolecules in addition to the target biomolecule by a compound" can also be treated.

<(Modification Example 1) feature quantity of biopolymer other than amino acid and use thereof>

<Target and probe>

In a case of treating DNA, RNA, cell membranes, and polysaccharides, which are biopolymers (compounds) other than proteins as drug targets (target biopolymers), the probe used for calculation of the feature quantity is not an amino acid but a different substance (the building block of each target). Specifically, in a case where the targets are "DNA, RNA, cell membranes, and polysaccharides", the probes are respectively set as "one or more kinds of nucleic acid bases, one or more kinds of nucleic acid bases, one or more kinds of lipid molecules, and one or more kinds of monosaccharide molecules". Further, water and one or more kinds of ions may be considered during the quantification of the degree of accumulation using these as probes. Further, in a case where the targets are formed of a plurality of kinds of biopolymers among "DNA, RNA, cell membranes, and polysaccharides", the probe can also be set as one or more of "one or more kinds of nucleic acid bases, one or more kinds of nucleic acid bases, one or more kinds of lipid molecules, one or more kinds of monosaccharide molecules, water, and one or more kinds of ions" (the kind, the number, and the combination thereof may be optional according to the configurations of the targets) in accordance of the configurations of the targets. Further, all the probes are assumed to generate van der Waals forces.

<Calculation of feature quantity and screening>

The configurations of devices for calculating the feature quantity (third feature quantity) and performing screening (the feature quantity calculating device and the screening device) are the same as those in the first embodiment (see Figs. 1 to 3). However, a third descriptor (third feature quantity) is calculated and stored instead of the three-dimensional AAM descriptor 230 in Fig. 3, and a third invariant feature quantity is calculated and stored instead of the invariant AAM descriptor 240. The procedures for calculation of the feature quantity and screening are the same as those in the first embodiment, and the feature quantity calculating method, the feature quantity calculating program, the screening method, and the screening program according to the embodiment of the present invention can be used. Specifically, the third invariant feature quantity is calculated (see Equation (2)) by calculating the distribution function (see Equation (1)) using "one or more (the kind, the number, and the combination thereof may be optional) selected from one or more kinds of nucleic acid bases, one or more kinds of lipid molecules, one or more kinds of monosaccharide molecules, water, and one or more kinds of ions" instead of the "amino acid" as the probe in the calculation of the first feature quantity (see Fig. 5), calculating the third feature quantity from the distribution function, and performing Fourier transform on the third feature quantity. Further, the third invariant feature quantity may be calculated by angular integration of the correlation function (see Equations (3) and (4)) using the third feature quantity of two different kinds of probes (the first probe formed of one or more selected from one or more kinds of nucleic acid bases, one or more kinds of lipid molecules, one or more kinds of monosaccharide molecules, water, and one or more kinds of ions and the second probe that is formed of one or more selected from one or more kinds of nucleic acid bases, one or more kinds of lipid molecules, one or more kinds of monosaccharide molecules, water, and one or more kinds of ions and different from the first probe).

In addition, the target compound can be extracted based on the similarity between the third feature quantity of a plurality of compounds and the third feature quantity of the binding compound using the third feature quantity instead of the three-dimensional AAM descriptor in the first embodiment. A compound having a similarity greater than or equal to the threshold may be extracted, or a compound may be extracted in a descending order of the similarity.

<Calculation of feature quantity and creation of compound>

The configurations of devices for calculating a feature quantity (third feature quantity) and creating a compound (the feature quantity calculating device and the compound creating device) are the same as those in the second embodiment (see Figs. 20 to 22). However, a third descriptor (third feature quantity) is calculated and stored instead of the three-dimensional AAM descriptor 230 in Fig. 22, and a third invariant feature quantity is calculated and stored instead of the invariant AAM descriptor 240. The procedures for calculation of the feature quantity and creation of a compound are the same as those in the second embodiment, and the feature quantity calculating method, the feature quantity calculating program, the compound creating method, and the compound creating program according to the embodiment of the present invention can be used. In Modification Example 1, a generator is constructed through machine learning (deep learning) using the three-dimensional structure of the compound as teacher data and the third feature quantity as an explanatory variable, and the three-dimensional structure of the target biopolymer can be generated from the third feature quantity of the binding compound whose binding to the target biopolymer has been confirmed using the constructed generator. Further, similar to the first to third embodiments, a compound having a three-dimensional structure with different features can be generated by selecting features of a compound to be provided as teacher data.

In the same manner as in the first to third embodiments described above, since the drug efficacy of a compound (the binding force with respect to the target such as DNA) is locally exhibited as the result of an interaction between a compound and a nucleic acid base (probe), in a case where the degree of accumulation of nucleic acid bases and the like is similar between compounds, the compounds have similar binding forces with respect to the targets. That is, compounds having similar third feature quantities exhibit similar drug efficacies. Therefore, in Modification Example 1, the chemical properties of the compound can be accurately exhibited by the third feature quantity. In addition, in a case where the third feature quantities are similar between the target compound and the binding compound that is bound to the target biopolymer, the drug efficacies of both the binding compound and the target compound are similar. Therefore, according to Modification Example 1, a target compound having drug efficacy similar to that of the binding compound is extracted based on the third feature quantity so that screening of a pharmaceutical candidate compound can be efficiently performed. Further, according to Modification Example 1, similarly to the above-described embodiments, a structural formula of a compound having a feature quantity similar to the feature quantity (the third feature quantity) of the binding compound (accordingly, the drug efficacies are similar) is generated without performing search, and thus the three-dimensional structure of the pharmaceutical candidate compound can be efficiently created.

In addition, in a case where the calculation of a feature quantity, the screening, and the creation of a compound are performed (corresponding to the third embodiment), the calculation, the screening, and the creation can be performed using the same configurations as in Figs. 28 to 30.

<(Modification Example 2) feature quantity of amino acid and others and use thereof>

<Target and probe>

In Modification Example 2, "composite of proteins and biopolymers other than the proteins (DNA, RNA, cell membranes, and polysaccharides)" are set as targets. Further, "one or more kinds of amino acids" (first probe) and "one or more selected from one or more kinds of nucleic acid bases, one or more kinds of nucleic acid bases, one or more kinds of lipid molecules, one or more kinds of monosaccharide molecules, water, and one or more kinds of ions" (second probe; the kind, the number, and the combination thereof may be optional) are set as probes. The configurations of the first and second probes (the kind, the number, and the combination thereof) can be set according to the configurations of the targets. Further, all the probes are assumed to generate van der Waals forces.

<Calculation of feature quantity and screening>

The configurations of devices for calculating the feature quantity (fourth feature quantity) and performing screening (the feature quantity calculating device and the screening device) are the same as those in the first embodiment (see Figs. 1 to 3). However, a fourth descriptor (fourth feature quantity) is calculated and stored instead of the three-dimensional AAM descriptor 230 in Fig. 3, and a fourth invariant feature quantity is calculated and stored instead of the invariant AAM descriptor 240. The procedures for calculation of the feature quantity and screening are the same as those in the first embodiment, and the feature quantity calculating method, the feature quantity calculating program, the screening method, and the screening program according to the embodiment of the present invention can be used. Specifically, the fourth invariant feature quantity is calculated (see Equation (2)) by calculating the distribution function (see Equation (1)) using "one or more kinds of amino acids" (the first probe) and "one or more selected from one or more kinds of nucleic acid bases, one or more kinds of lipid molecules, water, one or more kinds of monosaccharide molecules, and one or more kinds of ions" (the second probe; the kind, the number, and the combination thereof may be optional) instead of the "amino acid" as the probe in the calculation of the first feature quantity (see Fig. 5), calculating the fourth feature quantity from the distribution function, and performing Fourier transform on the fourth feature quantity. Further, the fourth invariant feature quantity may be calculated by angular integration of the correlation function (see Equations (3) and (4)) using the fourth feature quantity of two kinds of probes in which at least one of "one or more kinds of amino acids" (first probe) or "one or more selected from one or more kinds of nucleic acid bases, one or more kinds of lipid molecules, one or more kinds of monosaccharide molecules, water, and one or more kinds of ions" (second probe; the kind, the number, and the combination thereof may be optional) varies.

In addition, the target compound can be extracted based on the similarity between the fourth feature quantity of a plurality of compounds and the fourth feature quantity of the binding compound using the fourth feature quantity instead of the three-dimensional AAM descriptor in the first embodiment. A compound having a similarity greater than or equal to the threshold may be extracted, or a compound may be extracted in a descending order of the similarity.

<Calculation of feature quantity and creation of compound>

The configurations of devices for calculating a feature quantity (fourth feature quantity) and creating a compound (the feature quantity calculating device and the compound creating device) are the same as those in the second embodiment (see Figs. 20 to 22). However, a fourth descriptor (fourth feature quantity) is calculated and stored instead of the three-dimensional AAM descriptor 230 in Fig. 22, and a fourth invariant feature quantity is calculated and stored instead of the invariant AAM descriptor 240. The procedures for calculation of the feature quantity and creation of a compound are the same as those in the second embodiment, and the feature quantity calculating method, the feature quantity calculating program, the compound creating method, and the compound creating program according to the embodiment of the present invention can be used. In Modification Example 2, a generator is constructed through machine learning (deep learning) using the three-dimensional structure of the compound as teacher data and the fourth feature quantity as an explanatory variable, and the three-dimensional structure of the target biopolymer can be generated from the third feature quantity of the binding compound whose binding to the target biopolymer has been confirmed using the constructed generator. Further, similar to the first to third embodiments and Modification Example 1, a compound having a three-dimensional structure with different features can be generated by selecting features of a compound to be provided as teacher data.

In the same manner as in the first to third embodiments and Modification Example 1 described above, since the drug efficacy of a compound (the binding force with respect to the target) is locally exhibited as the result of an interaction between a compound and a probe, in a case where the degree of accumulation of probes is similar between compounds, the compounds have similar binding forces with respect to the targets. That is, compounds having similar fourth feature quantities exhibit similar drug efficacies. Therefore, in Modification Example 2, the chemical properties of the compound can be accurately exhibited by the fourth feature quantity. In addition, in a case where the fourth feature quantities are similar between the target compound and the binding compound that is bound to the target biopolymer, the drug efficacies of both the binding compound and the target compound are similar. Therefore, according to Modification Example 2, a target compound having drug efficacy similar to that of the binding compound is extracted based on the fourth feature quantity so that screening of a pharmaceutical candidate compound can be efficiently performed. Further, according to Modification Example 2, similarly to the above-described embodiments and Modification Example 1, a structural formula of a compound having a feature quantity similar to the feature quantity (the fourth feature quantity) of the binding compound (accordingly, the drug efficacies are similar) is generated without performing search, and thus the three-dimensional structure of the pharmaceutical candidate compound can be efficiently created.

In addition, in a case where the calculation of a feature quantity, the screening, and the creation of a compound are performed (corresponding to the third embodiment), the calculation, the screening, and the creation can be performed using the same configurations as in Figs. 28 to 30.

<(Modification Example 3) feature quantity of virtual point electric charge and the like and use thereof>

<Target and probe>

In Modification Example 3, a biopolymer is used as the target, and "one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0 (the kind, the number, and the combination thereof may be optional)" (virtual point electric charges and the like having a real electric charge and generating a van der Waals force) are used as the probes.

<Calculation of feature quantity and screening>

The configurations of devices for calculating the feature quantity (fifth feature quantity) and performing screening (the feature quantity calculating device and the screening device) are the same as those in the first embodiment (see Figs. 1 to 3). However, a fifth descriptor (fifth feature quantity) is calculated and stored instead of the three-dimensional AAM descriptor 230 in Fig. 3, and a fifth invariant feature quantity is calculated and stored instead of the invariant AAM descriptor 240. The procedures for calculation of the feature quantity and screening are the same as those in the first embodiment, and the feature quantity calculating method, the feature quantity calculating program, the screening method, and the screening program according to the embodiment of the present invention can be used. Specifically, the fifth invariant feature quantity is calculated (see Equation (2)) by calculating the distribution function (see Equation (1)) using "one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0 (the kind, the number, and the combination thereof may be optional)" instead of the "amino acid" as the probe in the calculation of the first feature quantity (see Fig. 5), calculating the fifth feature quantity from the distribution function, and performing Fourier transform on the fifth feature quantity. Further, the fifth invariant feature quantity may be calculated by angular integration of the correlation function (see Equations (3) and (4)) using the fifth feature quantity of two different kinds of probes (the first probe formed of one or more selected from the first point electric charge, the second point electric charge, the third point electric charge, the fourth point electric charge, the dipole, and the fifth point electric charge and the second probe that is formed of one or more selected from the first point electric charge, the second point electric charge, the third point electric charge, the fourth point electric charge, the dipole, and the fifth point electric charge and different from the first probe).

In addition, the target compound can be extracted based on the similarity between the fifth feature quantity of a plurality of compounds and the fifth feature quantity of the binding compound using the fifth feature quantity instead of the three-dimensional AAM descriptor in the first embodiment. A compound having a similarity greater than or equal to the threshold may be extracted, or a compound may be extracted in a descending order of the similarity.

<Calculation of feature quantity and creation of compound>

The configurations of devices for calculating a feature quantity (fifth feature quantity) and creating a compound (the feature quantity calculating device and the compound creating device) are the same as those in the second embodiment (see Figs. 20 to 22). However, a fifth descriptor (fifth feature quantity) is calculated and stored instead of the three-dimensional AAM descriptor 230 in Fig. 22, and a fifth invariant feature quantity is calculated and stored instead of the invariant AAM descriptor 240. The procedures for calculation of the feature quantity and creation of a compound are the same as those in the second embodiment, and the feature quantity calculating method, the feature quantity calculating program, the compound creating method, and the compound creating program according to the embodiment of the present invention can be used. In Modification Example 3, a generator is constructed through machine learning (deep learning) using the three-dimensional structure of the compound as teacher data and the fifth feature quantity as an explanatory variable, and the three-dimensional structure of the target biopolymer can be generated from the third feature quantity of the binding compound whose binding to the target biopolymer has been confirmed using the constructed generator. Further, similar to the first to third embodiments and Modification Examples 1 and 2, a compound having a three-dimensional structure with different features can be generated by selecting features of a compound to be provided as teacher data.

In the same manner as in the first to third embodiments and Modification Examples 1 and 2 described above, since the drug efficacy of a compound (the binding force with respect to the target) is locally exhibited as the result of an interaction between a compound and a probe, in a case where the degree of accumulation of probes is similar between compounds, the compounds have similar binding forces with respect to the targets. That is, compounds having similar fifth feature quantities exhibit similar drug efficacies. Therefore, in Modification Example 3, the chemical properties of the compound can be accurately exhibited by the fifth feature quantity. In addition, in a case where the fifth feature quantities are similar between the target compound and the binding compound that is bound to the target biopolymer, the drug efficacies of both the binding compound and the target compound are similar. Therefore, according to Modification Example 3, a target compound having drug efficacy similar to that of the binding compound is extracted based on the fifth feature quantity so that screening of a pharmaceutical candidate compound can be efficiently performed. Further, according to Modification Example 3, similarly to the above-described first to third embodiments and Modification Examples 1 and 2, a structural formula of a compound having a feature quantity similar to the feature quantity (the fifth feature quantity) of the binding compound (accordingly, the drug efficacies are similar) is generated without performing search, and thus the three-dimensional structure of the pharmaceutical candidate compound can be efficiently created.

In addition, in a case where the calculation of a feature quantity, the screening, and the creation of a compound are performed (corresponding to the third embodiment), the calculation, the screening, and the creation can be performed using the same configurations as in Figs. 28 to 30.

<(Modification Example 4) feature quantity of amino acid and virtual point electric charge and use thereof>

<Target and probe>

In Modification Example 4, a biopolymer (compound) is used as the target, and "a first probe that is one or more kinds of amino acids and a second probe that is one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0 (the kind, the number, and the combination thereof may be optional)" are used as the probes.

<Calculation of feature quantity and screening>

The configurations of devices for calculating the feature quantity (sixth feature quantity) and performing screening (the feature quantity calculating device and the screening device) are the same as those in the first embodiment (see Figs. 1 to 3). However, a sixth descriptor (sixth feature quantity) is calculated and stored instead of the three-dimensional AAM descriptor 230 in Fig. 3, and a sixth invariant feature quantity is calculated and stored instead of the invariant AAM descriptor 240. The procedures for calculation of the feature quantity and screening are the same as those in the first embodiment, and the feature quantity calculating method, the feature quantity calculating program, the screening method, and the screening program according to the embodiment of the present invention can be used. Specifically, the sixth invariant feature quantity is calculated (see Equation (2)) by calculating the distribution function (see Equation (1)) using "a first probe that is one or more kinds of amino acids and a second probe that is one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0 (the kind, the number, and the combination thereof may be optional)" instead of the "amino acid" as the probe in the calculation of the first feature quantity (see Fig. 5), calculating the sixth feature quantity from the distribution function, and performing Fourier transform on the sixth feature quantity. Further, the sixth invariant feature quantity may be calculated by angular integration of the correlation function (see Equations (3) and (4)) using the sixth feature quantity of two kinds of probes in which at least one of the first probe or the second probe varies.
In addition, the target compound can be extracted based on the similarity between the sixth feature quantity of a plurality of compounds and the sixth feature quantity of the binding compound using the sixth feature quantity instead of the three-dimensional AAM descriptor in the first embodiment. A compound having a similarity greater than or equal to the threshold may be extracted, or a compound may be extracted in a descending order of the similarity.

<Calculation of feature quantity and creation of compound>

The configurations of devices for calculating a feature quantity (sixth feature quantity) and creating a compound (the feature quantity calculating device and the compound creating device) are the same as those in the second embodiment (see Figs. 20 to 22). However, a sixth descriptor (sixth feature quantity) is calculated and stored instead of the three-dimensional AAM descriptor 230 in Fig. 22, and a sixth invariant feature quantity is calculated and stored instead of the invariant AAM descriptor 240. The procedures for calculation of the feature quantity and creation of a compound are the same as those in the second embodiment, and the feature quantity calculating method, the feature quantity calculating program, the compound creating method, and the compound creating program according to the embodiment of the present invention can be used. In Modification Example 4, a generator is constructed through machine learning (deep learning) using the three-dimensional structure of the compound as teacher data and the sixth feature quantity as an explanatory variable, and the three-dimensional structure of the target biopolymer can be generated from the sixth feature quantity of the binding compound whose binding to the target biopolymer has been confirmed using the constructed generator. Further, similar to the first to third embodiments, a compound having a three-dimensional structure with different features can be generated by selecting features of a compound to be provided as teacher data.
In the same manner as in the first to third embodiments, since the drug efficacy of a compound (the binding force with respect to the target) is locally exhibited as the result of an interaction between a compound and a probe, in a case where the degree of accumulation of probes is similar between compounds, the compounds have similar binding forces with respect to the targets. That is, compounds having similar sixth feature quantities exhibit similar drug efficacies. Therefore, in Modification Example 4, the chemical properties of the compound can be accurately exhibited by the sixth feature quantity. In addition, in a case where the sixth feature quantities are similar between the target compound and the binding compound that is bound to the target biopolymer, the drug efficacies of both the binding compound and the target compound are similar. Therefore, according to Modification Example 4, a target compound having drug efficacy similar to that of the binding compound is extracted based on the sixth feature quantity so that screening of a pharmaceutical candidate compound can be efficiently performed. Further, according to Modification Example 4, similarly to the above-described embodiments, a structural formula of a compound having a feature quantity similar to the feature quantity (the sixth feature quantity) of the binding compound (accordingly, the drug efficacies are similar) is generated without performing search, and thus the three-dimensional structure of the pharmaceutical candidate compound can be efficiently created.

In addition, in a case where the calculation of a feature quantity, the screening, and the creation of a compound are performed (corresponding to the third embodiment), the calculation, the screening, and the creation can be performed using the same configurations as in Figs. 28 to 30.

<(Modification Example 5) feature quantity of nucleic acid base and virtual point electric charge and use thereof>

<Target and probe>

In Modification Example 5, a biopolymer (compound) is used as the target, and a first probe "that is one or more selected from one or more kinds of nucleic acid bases, one or more kinds of lipid molecules, water, one or more kinds of monosaccharide molecules, and one or more kinds of ions (the kind, the number, and the combination thereof may be optional)" and a second probe "that is one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0 (the kind, the number, and the combination thereof may be optional)" are used as the probes.

<Calculation of feature quantity and screening>

The configurations of devices for calculating the feature quantity (seventh feature quantity) and performing screening (the feature quantity calculating device and the screening device) are the same as those in the first embodiment (see Figs. 1 to 3). However, a seventh descriptor (seventh feature quantity) is calculated and stored instead of the three-dimensional AAM descriptor 230 in Fig. 3, and a seventh invariant feature quantity is calculated and stored instead of the invariant AAM descriptor 240. The procedures for calculation of the feature quantity and screening are the same as those in the first embodiment, and the feature quantity calculating method, the feature quantity calculating program, the screening method, and the screening program according to the embodiment of the present invention can be used. Specifically, the seventh invariant feature quantity is calculated (see Equation (2)) by calculating the distribution function (see Equation (1)) using the first probe "that is one or more selected from one or more kinds of nucleic acid bases, one or more kinds of lipid molecules, water, one or more kinds of monosaccharide molecules, and one or more kinds of ions (the kind, the number, and the combination thereof may be optional)" and the second probe "that is one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0 (the kind, the number, and the combination thereof may be optional)"instead of the "amino acid" as the probe in the calculation of the first feature quantity (see Fig. 5), calculating the seventh feature quantity from the distribution function, and performing Fourier transform on the seventh feature quantity. Further, the seventh invariant feature quantity may be calculated by angular integration of the correlation function (see Equations (3) and (4)) using the seventh feature quantity of two kinds of probes in which at least one of the first probe or the second probe varies.

In addition, the target compound can be extracted based on the similarity between the seventh feature quantity of a plurality of compounds and the seventh feature quantity of the binding compound using the seventh feature quantity instead of the three-dimensional AAM descriptor in the first embodiment. A compound having a similarity greater than or equal to the threshold may be extracted, or a compound may be extracted in a descending order of the similarity.

<Calculation of feature quantity and creation of compound>

The configurations of devices for calculating a feature quantity (seventh feature quantity) and creating a compound (the feature quantity calculating device and the compound creating device) are the same as those in the second embodiment (see Figs. 20 to 22). However, a seventh descriptor (seventh feature quantity) is calculated and stored instead of the three-dimensional AAM descriptor 230 in Fig. 22, and a seventh invariant feature quantity is calculated and stored instead of the invariant AAM descriptor 240. The procedures for calculation of the feature quantity and creation of a compound are the same as those in the second embodiment, and the feature quantity calculating method, the feature quantity calculating program, the compound creating method, and the compound creating program according to the embodiment of the present invention can be used. In Modification Example 5, a generator is constructed through machine learning (deep learning) using the three-dimensional structure of the compound as teacher data and the seventh feature quantity as an explanatory variable, and the three-dimensional structure of the target biopolymer can be generated from the seventh feature quantity of the binding compound whose binding to the target biopolymer has been confirmed using the constructed generator. Further, similar to the first to third embodiments, a compound having a three-dimensional structure with different features can be generated by selecting features of a compound to be provided as teacher data.

In the same manner as in the first to third embodiments, since the drug efficacy of a compound (the binding force with respect to the target) is locally exhibited as the result of an interaction between a compound and a probe, in a case where the degree of accumulation of probes is similar between compounds, the compounds have similar binding forces with respect to the targets. That is, compounds having similar seventh feature quantities exhibit similar drug efficacies. Therefore, in Modification Example 5, the chemical properties of the compound can be accurately exhibited by the seventh feature quantity. In addition, in a case where the seventh feature quantities are similar between the target compound and the binding compound that is bound to the target biopolymer, the drug efficacies of both the binding compound and the target compound are similar. Therefore, according to Modification Example 5, a target compound having drug efficacy similar to that of the binding compound is extracted based on the seventh feature quantity so that screening of a pharmaceutical candidate compound can be efficiently performed. Further, according to Modification Example 5, similarly to the above-described embodiments, a structural formula of a compound having a feature quantity similar to the feature quantity (the seventh feature quantity) of the binding compound (accordingly, the drug efficacies are similar) is generated without performing search, and thus the three-dimensional structure of the pharmaceutical candidate compound can be efficiently created.

In addition, in a case where the calculation of a feature quantity, the screening, and the creation of a compound are performed (corresponding to the third embodiment), the calculation, the screening, and the creation can be performed using the same configurations as in Figs. 28 to 30.

<(Modification Example 6) feature quantity of nucleic acid base and virtual point electric charge and use thereof>

<Target and probe>

In Modification Example 6, a biopolymer (compound) is used as the target, and a first probe "that is one or more kinds of amino acids", a second probe "that is one or more selected from one or more kinds of nucleic acid bases, one or more kinds of lipid molecules, water, one or more kinds of monosaccharide molecules, and one or more kinds of ions (the kind, the number, and the combination thereof may be optional)", and a third probe "that is one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0 (the kind, the number, and the combination thereof may be optional)" are used as the probes.

<Calculation of feature quantity and screening>

The configurations of devices for calculating the feature quantity (eighth feature quantity) and performing screening (the feature quantity calculating device and the screening device) are the same as those in the first embodiment (see Figs. 1 to 3). However, an eighth descriptor (eighth feature quantity) is calculated and stored instead of the three-dimensional AAM descriptor 230 in Fig. 3, and an eighth invariant feature quantity is calculated and stored instead of the invariant AAM descriptor 240. The procedures for calculation of the feature quantity and screening are the same as those in the first embodiment, and the feature quantity calculating method, the feature quantity calculating program, the screening method, and the screening program according to the embodiment of the present invention can be used. Specifically, the eighth invariant feature quantity is calculated (see Equation (2)) by calculating the distribution function (see Equation (1)) using the first probe "that is one or more kinds of amino acids", the second probe "that is one or more selected from one or more kinds of nucleic acid bases, one or more kinds of lipid molecules, water, one or more kinds of monosaccharide molecules, and one or more kinds of ions (the kind, the number, and the combination thereof may be optional)", and the third probe "that is one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0 (the kind, the number, and the combination thereof may be optional)" instead of the "amino acid" as the probe in the calculation of the first feature quantity (see Fig. 5), calculating the eighth feature quantity from the distribution function, and performing Fourier transform on the eighth feature quantity. Further, the eighth invariant feature quantity may be calculated by angular integration of the correlation function (see Equations (3) and (4)) using the eighth feature quantity of two kinds of probes in which at least one of the first probe, the second probe, or the third probe varies.

In addition, the target compound can be extracted based on the similarity between the eighth feature quantity of a plurality of compounds and the eighth feature quantity of the binding compound using the eighth feature quantity instead of the three-dimensional AAM descriptor in the first embodiment. A compound having a similarity greater than or equal to the threshold may be extracted, or a compound may be extracted in a descending order of the similarity.

<Calculation of feature quantity and creation of compound>

The configurations of devices for calculating a feature quantity (eighth feature quantity) and creating a compound (the feature quantity calculating device and the compound creating device) are the same as those in the second embodiment (see Figs. 20 to 22). However, an eighth descriptor (eighth feature quantity) is calculated and stored instead of the three-dimensional AAM descriptor 230 in Fig. 22, and an eighth invariant feature quantity is calculated and stored instead of the invariant AAM descriptor 240. The procedures for calculation of the feature quantity and creation of a compound are the same as those in the second embodiment, and the feature quantity calculating method, the feature quantity calculating program, the compound creating method, and the compound creating program according to the embodiment of the present invention can be used. In Modification Example 6, a generator is constructed through machine learning (deep learning) using the three-dimensional structure of the compound as teacher data and the eighth feature quantity as an explanatory variable, and the three-dimensional structure of the target biopolymer can be generated from the eighth feature quantity of the binding compound whose binding to the target biopolymer has been confirmed using the constructed generator. Further, similar to the first to third embodiments, a compound having a three-dimensional structure with different features can be generated by selecting features of a compound to be provided as teacher data.

In the same manner as in the first to third embodiments, since the drug efficacy of a compound (the binding force with respect to the target) is locally exhibited as the result of an interaction between a compound and a probe, in a case where the degree of accumulation of probes is similar between compounds, the compounds have similar binding forces with respect to the targets. That is, compounds having similar eighth feature quantities exhibit similar drug efficacies. Therefore, in Modification Example 6, the chemical properties of the compound can be accurately exhibited by the eighth feature quantity. In addition, in a case where the eighth feature quantities are similar between the target compound and the binding compound that is bound to the target biopolymer, the drug efficacies of both the binding compound and the target compound are similar. Therefore, according to Modification Example 6, a target compound having drug efficacy similar to that of the binding compound is extracted based on the eighth feature quantity so that screening of a pharmaceutical candidate compound can be efficiently performed. Further, according to Modification Example 6, similarly to the above-described embodiments, a structural formula of a compound having a feature quantity similar to the feature quantity (the eighth feature quantity) of the binding compound (accordingly, the drug efficacies are similar) is generated without performing search, and thus the three-dimensional structure of the pharmaceutical candidate compound can be efficiently created.

In addition, in a case where the calculation of a feature quantity, the screening, and the creation of a compound are performed (corresponding to the third embodiment), the calculation, the screening, and the creation can be performed using the same configurations as in Figs. 28 to 30.

<comparison of number of hits based on each feature quantity>

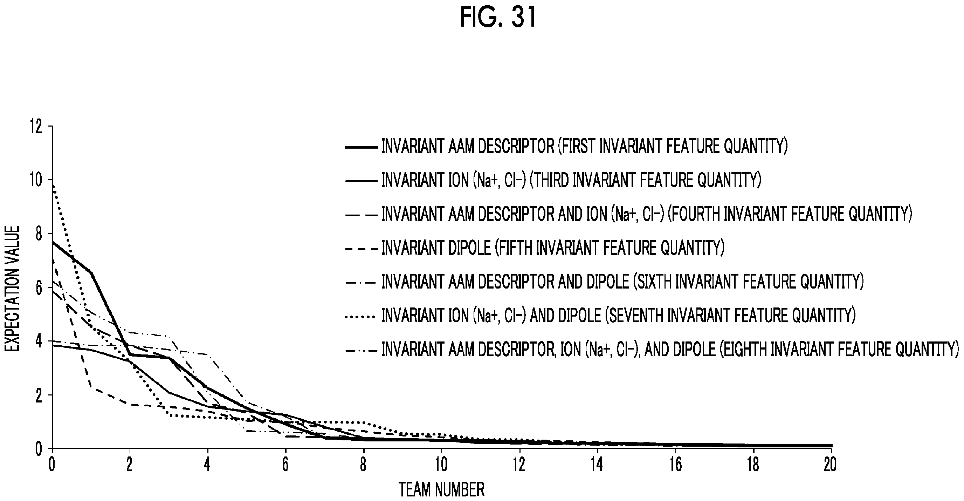

Fig. 31 is a diagram showing an example of a result of comparative evaluation of the easiness of finding hits based on an invariant feature quantity (a first invariant feature quantity using an amino acid as a probe and third to eighth invariant feature quantities using others as probes) with a compound as a target structure for the same system (protein ABL1) as in Fig. 13 described above. According to Fig. 31, although there is a difference in the effect (the expectation value of the number of hits) depending on the kind of descriptor (feature quantity), it was found that the expectation value is improved as compared with the random case (see Fig. 13). Further, Fig. 31 shows a result of clustering with (the number of teams = 183), and thus the result (clustering with the number of teams = 221) is different from the above-described result in a case where the number of hits for "AAM" is as shown in Fig. 13. As described above, even in a case where the third to eighth invariant feature quantities are used, screening of a pharmaceutical candidate compound can be efficiently performed.

Explanation of References

| | |
|---|---|
| 10: | screening device |
| 20: | compound creating device |
| 30: | pharmaceutical candidate compound search device |
| 100: | processing unit |
| 101: | processing unit |
| 102: | processing unit |
| 110: | information input unit |
| 120: | feature quantity calculation unit |
| 130: | similarity calculation unit |
| 132: | generator construction unit |
| 140: | compound extraction unit |
| 142: | compound three-dimensional structure generation unit |
| 150: | display control unit |
| 160: | CPU |
| 170: | ROM |
| 180: | RAM |
| 200: | storage unit |
| 201: | storage unit |
| 202: | storage unit |
| 210: | structure information |
| 220: | three-dimensional structure information |
| 230: | three-dimensional AAM descriptor |
| 240: | invariant AAM descriptor |
| 250: | similarity information |
| 260: | compound extraction result |
| 270: | three-dimensional structure generation result |
| 300: | display unit |
| 310: | monitor |
| 400: | operation unit |
| 410: | keyboard |
| 420: | mouse |
| 500: | external server |
| 510: | external database |
| A1: | amino acid |
| A2: | amino acid |
| A3: | amino acid |
| AA2AR: | protein |
| ABL1: | protein |
| NW: | network |
| PO: | pocket |
| PS: | pocket structure |
| S100 to S108: | each step of feature quantity calculating method |
| S200 to S206: | each step of feature quantity calculating method |
| S300 to S304: | each step of target compound extracting method |
| S400 to S404: | each step of target compound extracting method |
| S500 to S504: | each step of three-dimensional structure creating method |
| S600 to S604: | each step of three-dimensional structure creating method |
| TP: | target protein |

**Claims**

1. A feature quantity calculating method comprising:

a target structure designating step of designating a target structure formed of a plurality of unit structures having chemical properties;

a three-dimensional structure generating step of generating a three-dimensional structure using the plurality of unit structures for the target structure; and

a feature quantity calculating step of calculating a feature quantity obtained by quantifying, in a three-dimensional space, a degree of accumulation of one or more kinds of probes in a periphery of the three-dimensional structure, wherein the probe is a structure in which a plurality of points having a real electric charge and generating a van der Waals force are disposed to be separated from each other.

2. The feature quantity calculating method according to claim 1,

wherein a compound is designated as the target structure in the target structure designating step,

a three-dimensional structure of the compound is generated with a plurality of atoms in the three-dimensional structure generating step, and

a first feature quantity which is a feature quantity obtained by quantifying, in the three-dimensional space, a degree of accumulation of amino acids as the probes in the periphery of the three-dimensional structure of the compound generated in the three-dimensional structure generating step is calculated in the feature quantity calculating step.

3. The feature quantity calculating method according to claim 2, further comprising:

an invariant conversion step of converting the first feature quantity into an invariant with respect to rotation and translation of the compound to calculate a first invariant feature quantity.

4. The feature quantity calculating method according to claim 3,

wherein the first feature quantity of two different kinds of amino acids is calculated in the feature quantity calculating step, and

the first invariant feature quantity is calculated using the first feature quantity of the two different kinds of amino acids in the invariant conversion step.

5. The feature quantity calculating method according to claim 1,

wherein a pocket structure bound to a pocket that is an active site of a target protein is designated as the target structure in the target structure designating step,

a three-dimensional structure of the pocket structure is generated with a plurality of virtual spheres in the three-dimensional structure generating step, and

a second feature quantity which is a feature quantity obtained by quantifying, in the three-dimensional space, a degree of accumulation of amino acids as the probes in the periphery of the three-dimensional structure of the pocket structure generated in the three-dimensional structure generating step is calculated in the feature quantity calculating step.

6. The feature quantity calculating method according to claim 5, further comprising:

an invariant conversion step of converting the second feature quantity into an invariant with respect to rotation and translation of the pocket structure to calculate a second invariant feature quantity.

7. The feature quantity calculating method according to claim 6,

wherein the second feature quantity of two different kinds of amino acids is calculated in the feature quantity calculating step, and

the second invariant feature quantity is calculated using the second feature quantity of the two different kinds of amino acids in the invariant conversion step.

8. The feature quantity calculating method according to claim 1,

wherein a compound is designated as the target structure in the target structure designating step,

a three-dimensional structure of the compound is generated with a plurality of atoms in the three-dimensional structure generating step, and

a third feature quantity which is a feature quantity obtained by quantifying, in the three-dimensional space, a degree of accumulation of the probes in the periphery of the three-dimensional structure of the compound generated in the three-dimensional structure generating step which is the degree of accumulation using one or more selected from one or more kinds of nucleic acid bases, one or more kinds of lipid molecules, one or more kinds of monosaccharide molecules, water, and one or more kinds of ions, as the probes is calculated in the feature quantity calculating step.

9. The feature quantity calculating method according to claim 8, further comprising:
an invariant conversion step of converting the third feature quantity into an invariant with respect to rotation and translation of the compound to calculate a third invariant feature quantity.

10. The feature quantity calculating method according to claim 9,

wherein the third feature quantity of a first probe that is formed of one or more selected from the one or more kinds of nucleic acid bases, the one or more kinds of lipid molecules, the one or more kinds of monosaccharide molecules, the water, and the one or more kinds of ions and a second probe that is formed of one or more selected from the one or more kinds of nucleic acid bases, the one or more kinds of lipid molecules, the one or more kinds of monosaccharide molecules, the water, and the one or more kinds of ions and different from the first probe is calculated in the feature quantity calculating step, and
the third invariant feature quantity is calculated using the third feature quantity of the first probe and the third feature quantity of the second probe in the invariant conversion step.

11. The feature quantity calculating method according to claim 1,

wherein a compound is designated as the target structure in the target structure designating step,
a three-dimensional structure of the compound is generated with a plurality of atoms in the three-dimensional structure generating step, and
a fourth feature quantity which is a feature quantity obtained by quantifying, in the three-dimensional space, a degree of accumulation of the probes in the periphery of the three-dimensional structure of the compound generated in the three-dimensional structure generating step which is the degree of accumulation using a first probe that is one or more kinds of amino acids and a second probe that is one or more selected from one or more kinds of nucleic acid bases, one or more kinds of lipid molecules, water, one or more kinds of monosaccharide molecules, and one or more kinds of ions, as the probes is calculated in the feature quantity calculating step.

12. The feature quantity calculating method according to claim 11, further comprising:
an invariant conversion step of converting the fourth feature quantity into an invariant with respect to rotation and translation of the compound to calculate a fourth invariant feature quantity.

13. The feature quantity calculating method according to claim 12,

wherein the fourth feature quantity of two kinds of the probes in which at least one of the first probe or the second probe varies is calculated in the feature quantity calculating step, and
the fourth invariant feature quantity is calculated using the fourth feature quantity of the two different kinds of the probes in the invariant conversion step.

14. The feature quantity calculating method according to claim 1,

wherein a compound is designated as the target structure in the target structure designating step,
a three-dimensional structure of the compound is generated with a plurality of atoms in the three-dimensional structure generating step, and
a fifth feature quantity which is a feature quantity obtained by quantifying, in the three-dimensional space, the degree of accumulation of the probes in the periphery of the three-dimensional structure of the compound generated in the three-dimensional structure generating step which is the degree of accumulation using one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0, as the probes is calculated in the feature quantity calculating step.

**15.** The feature quantity calculating method according to claim 14, further comprising:
an invariant conversion step of converting the fifth feature quantity into an invariant with respect to rotation and translation of the compound to calculate a fifth invariant feature quantity.

**16.** The feature quantity calculating method according to claim 15,

wherein the fifth feature quantity of a first probe that is formed of one or more selected from the first point electric charge, the second point electric charge, the third point electric charge, the fourth point electric charge, the dipole, and the fifth point electric charge and a second probe that is formed of one or more selected from the first point electric charge, the second point electric charge, the third point electric charge, the fourth point electric charge, the dipole, and the fifth point electric charge and different from the first probe is calculated in the feature quantity calculating step, and
the fifth invariant feature quantity is calculated using the fifth feature quantity of the first probe and the fifth feature quantity of the second probe in the invariant conversion step.

**17.** The feature quantity calculating method according to claim 1,

wherein a compound is designated as the target structure in the target structure designating step,
a three-dimensional structure of the compound is generated with a plurality of atoms in the three-dimensional structure generating step, and
a sixth feature quantity which is a feature quantity obtained by quantifying, in the three-dimensional space, the degree of accumulation of the probes in the periphery of the three-dimensional structure of the compound generated in the three-dimensional structure generating step which is the degree of accumulation using a first probe that is one or more kinds of amino acids and a second probe that is one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0, as the probes is calculated in the feature quantity calculating step.

**18.** The feature quantity calculating method according to claim 17, further comprising:
an invariant conversion step of converting the sixth feature quantity into an invariant with respect to rotation and translation of the compound to calculate a sixth invariant feature quantity.

**19.** The feature quantity calculating method according to claim 18,

wherein the sixth feature quantity of two kinds of the probes in which at least one of the first probe or the second probe varies is calculated in the feature quantity calculating step, and
the sixth invariant feature quantity is calculated using the sixth feature quantity of the two different kinds of the probes in the invariant conversion step.

**20.** The feature quantity calculating method according to claim 1,

wherein a compound is designated as the target structure in the target structure designating step,
a three-dimensional structure of the compound is generated with a plurality of atoms in the three-dimensional structure generating step, and
a seventh feature quantity which is a feature quantity obtained by quantifying, in the three-dimensional space, the degree of accumulation of the probes in the periphery of the three-dimensional structure of the compound generated in the three-dimensional structure generating step which is the degree of accumulation using a first probe that is one or more selected from one or more kinds of nucleic acid bases, one or more kinds of lipid molecules, water, one or more kinds of monosaccharide molecules, and one or more kinds of ions and a second probe that is one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0, as the probes is calculated in the feature quantity calculating step.

**21.** The feature quantity calculating method according to claim 20, further comprising:

an invariant conversion step of converting the seventh feature quantity into an invariant with respect to rotation and translation of the compound to calculate a seventh invariant feature quantity.

22. The feature quantity calculating method according to claim 21,

wherein the seventh feature quantity of two kinds of the probes in which at least one of the first probe or the second probe varies is calculated in the feature quantity calculating step, and
the seventh invariant feature quantity is calculated using the seventh feature quantity of the two different kinds of the probes in the invariant conversion step.

23. The feature quantity calculating method according to claim 1,

wherein a compound is designated as the target structure in the target structure designating step,
a three-dimensional structure of the compound is generated with a plurality of atoms in the three-dimensional structure generating step, and
an eighth feature quantity which is a feature quantity obtained by quantifying, in the three-dimensional space, the degree of accumulation of the probes in the periphery of the three-dimensional structure of the compound generated in the three-dimensional structure generating step which is the degree of accumulation using a first probe that is one or more kinds of amino acids, a second probe that is one or more selected from one or more kinds of nucleic acid bases, one or more kinds of lipid molecules, water, one or more kinds of monosaccharide molecules, and one or more kinds of ions, and a third probe that is one or more selected from a first point electric charge having an electric charge of +1, a second point electric charge having an electric charge of -1, a third point electric charge having an electric charge of +0.1, a fourth point electric charge of having an electric charge of -0.1, a dipole in which the first point electric charge and the second point electric charge are disposed to be separated from each other, and a fifth point electric charge having an electric charge of 0, as the probes is calculated in the feature quantity calculating step.

24. The feature quantity calculating method according to claim 23, further comprising:
an invariant conversion step of converting the eighth feature quantity into an invariant with respect to rotation and translation of the compound to calculate an eighth invariant feature quantity.

25. The feature quantity calculating method according to claim 24,

wherein the eighth feature quantity of two kinds of the probes in which at least one of the first probe, the second probe, or the third probe varies is calculated in the feature quantity calculating step, and
the eighth invariant feature quantity is calculated using the eighth feature quantity of the two different kinds of the probes in the invariant conversion step.

26. A feature quantity calculating program which causes a computer to execute the feature quantity calculating method according to any one of claims 1 to 25.

27. A feature quantity calculating device comprising:

a target structure designation unit which designates a target structure formed of a plurality of unit structures having chemical properties;
a three-dimensional structure generation unit which generates a three-dimensional structure using the plurality of unit structures for the target structure; and
a feature quantity calculation unit which calculates a feature quantity obtained by quantifying, in a three-dimensional space, a degree of accumulation of one or more kinds of probes in a periphery of the three-dimensional structure,
wherein the probe is a structure in which a plurality of points having a real electric charge and generating a van der Waals force are disposed to be separated from each other.

28. A screening method of extracting a target compound which is bound to a target protein from a plurality of compounds, the method comprising:

a storing step of storing a three-dimensional structure of a compound formed of a plurality of atoms and the first feature quantity of the three-dimensional structure of the compound calculated using the feature quantity calculating method according to claim 2 in association with each other for each of the plurality of compounds;

a feature quantity calculating step of calculating the first feature quantity of a ligand that is a compound whose binding to the target protein has been confirmed;

a similarity calculating step of calculating a similarity between the first feature quantity of the plurality of compounds and the first feature quantity of the ligand; and

a compound extracting step of extracting the target compound from the plurality of compounds based on the similarity.

29. A screening method of extracting a target compound which is bound to a target protein from a plurality of compounds, the method comprising:

a storing step of storing a three-dimensional structure of a compound formed of a plurality of atoms and the first invariant feature quantity of the three-dimensional structure of the compound in association with each other for each of the plurality of compounds;

a feature quantity calculating step of calculating the first invariant feature quantity of a ligand that is a compound whose binding to the target protein has been confirmed using the feature quantity calculating method according to claim 3;

a similarity calculating step of calculating a similarity between the first invariant feature quantity of the plurality of compounds and the first invariant feature quantity of the ligand; and

a compound extracting step of extracting the target compound from the plurality of compounds based on the similarity.

30. The screening method according to claim 28 or 29,
wherein a compound having a similarity greater than or equal to a threshold is extracted in the compound extracting step.

31. The screening method according to any one of claims 28 to 30,
wherein compounds are extracted in a descending order of the similarity in the compound extracting step.

32. A screening method of extracting a target compound which is bound to a target protein from a plurality of compounds, the method comprising:

a storing step of storing a three-dimensional structure of a compound formed of a plurality of atoms and the first feature quantity calculated using the feature quantity calculating method according to claim 2 in association with each other for each of the plurality of compounds;

a feature quantity calculating step of calculating the second feature quantity of the pocket structure of the target protein using the feature quantity calculating method according to claim 5;

a similarity calculating step of calculating a similarity between the first feature quantity of the plurality of compounds and the second feature quantity of the pocket structure; and

a compound extracting step of extracting the target compound from the plurality of compounds based on the similarity.

33. A screening method of extracting a target compound which is bound to a target protein from a plurality of compounds, the method comprising:

a storing step of storing a three-dimensional structure of a compound formed of a plurality of atoms and the first invariant feature quantity calculated using the feature quantity calculating method according to claim 3 in association with each other for each of the plurality of compounds;

a feature quantity calculating step of calculating the second invariant feature quantity of the pocket structure of the target protein using the feature quantity calculating method according to claim 6;

a similarity calculating step of calculating a similarity between the first invariant feature quantity of the plurality of compounds and the second invariant feature quantity of the pocket structure; and

a compound extracting step of extracting the target compound from the plurality of compounds based on the similarity.

34. The screening method according to claim 32 or 33,
wherein a compound having a similarity greater than or equal to a threshold is extracted in the compound extracting step.

**35.** The screening method according to any one of claims 32 to 34,
wherein compounds are extracted in a descending order of the similarity in the compound extracting step.

**36.** A screening method of extracting a target compound which is bound to a target biopolymer other than a protein from a plurality of compounds, the method comprising:

a storing step of storing a three-dimensional structure of a compound formed of a plurality of atoms and the third feature quantity of the three-dimensional structure of the compound calculated using the feature quantity calculating method according to claim 8 in association with each other for each of the plurality of compounds;
a feature quantity calculating step of calculating the third feature quantity of a binding compound that is a compound whose binding to the target biopolymer other than the protein has been confirmed;
a similarity calculating step of calculating a similarity between the third feature quantity of the plurality of compounds and the third feature quantity of the binding compound; and
a compound extracting step of extracting the target compound from the plurality of compounds based on the similarity.

**37.** A screening method of extracting a target compound which is bound to a target biopolymer from a plurality of compounds, the method comprising:

a storing step of storing a three-dimensional structure of a compound formed of a plurality of atoms and the fourth feature quantity of the three-dimensional structure of the compound calculated using the feature quantity calculating method according to claim 11 in association with each other for each of the plurality of compounds;
a feature quantity calculating step of calculating the fourth feature quantity of a binding compound that is a compound whose binding to the target biopolymer has been confirmed;
a similarity calculating step of calculating a similarity between the fourth feature quantity of the plurality of compounds and the fourth feature quantity of the binding compound; and
a compound extracting step of extracting the target compound from the plurality of compounds based on the similarity.

**38.** A screening method of extracting a target compound which is bound to a target biopolymer from a plurality of compounds, the method comprising:

a storing step of storing a three-dimensional structure of a compound formed of a plurality of atoms and the fifth feature quantity of the three-dimensional structure of the compound calculated using the feature quantity calculating method according to claim 14 in association with each other for each of the plurality of compounds;
a feature quantity calculating step of calculating the fifth feature quantity of a binding compound that is a compound whose binding to the target biopolymer has been confirmed;
a similarity calculating step of calculating a similarity between the fifth feature quantity of the plurality of compounds and the fifth feature quantity of the binding compound; and
a compound extracting step of extracting the target compound from the plurality of compounds based on the similarity.

**39.** A screening method of extracting a target compound which is bound to a target biopolymer from a plurality of compounds, the method comprising:

a storing step of storing a three-dimensional structure of a compound formed of a plurality of atoms and the sixth feature quantity of the three-dimensional structure of the compound calculated using the feature quantity calculating method according to claim 17 in association with each other for each of the plurality of compounds;
a feature quantity calculating step of calculating the sixth feature quantity of a binding compound that is a compound whose binding to the target biopolymer has been confirmed;
a similarity calculating step of calculating a similarity between the sixth feature quantity of the plurality of compounds and the sixth feature quantity of the binding compound; and
a compound extracting step of extracting the target compound from the plurality of compounds based on the similarity.

**40.** A screening method of extracting a target compound which is bound to a target biopolymer from a plurality of compounds, the method comprising:

a storing step of storing a three-dimensional structure of a compound formed of a plurality of atoms and the seventh feature quantity of the three-dimensional structure of the compound calculated using the feature quantity calculating method according to claim 20 in association with each other for each of the plurality of compounds;
a feature quantity calculating step of calculating the seventh feature quantity of a binding compound that is a compound whose binding to the target biopolymer has been confirmed;
a similarity calculating step of calculating a similarity between the seventh feature quantity of the plurality of compounds and the seventh feature quantity of the binding compound; and
a compound extracting step of extracting the target compound from the plurality of compounds based on the similarity.

41. A screening method of extracting a target compound which is bound to a target biopolymer from a plurality of compounds, the method comprising:

a storing step of storing a three-dimensional structure of a compound formed of a plurality of atoms and the eighth feature quantity of the three-dimensional structure of the compound calculated using the feature quantity calculating method according to claim 23 in association with each other for each of the plurality of compounds;
a feature quantity calculating step of calculating the eighth feature quantity of a binding compound that is a compound whose binding to the target biopolymer has been confirmed;
a similarity calculating step of calculating a similarity between the eighth feature quantity of the plurality of compounds and the eighth feature quantity of the binding compound; and
a compound extracting step of extracting the target compound from the plurality of compounds based on the similarity.

42. A screening program which causes a computer to execute the screening method according to any one of claims 28 to 41.

43. A screening device which extracts a target compound that is bound to a target protein from a plurality of compounds, the device comprising:

a storage unit which stores a three-dimensional structure of a compound formed of a plurality of atoms and the first feature quantity of the three-dimensional structure of the compound calculated using the feature quantity calculating method according to claim 2 in association with each other for each of the plurality of compounds;
a feature quantity calculation unit which calculates the first feature quantity of a ligand that is a compound whose binding to the target protein has been confirmed;
a similarity calculation unit which calculates a similarity between the first feature quantity of the plurality of compounds and the first feature quantity of the ligand; and
a compound extraction unit which extracts the target compound from the plurality of compounds based on the similarity.

44. A screening device which extracts a target compound that is bound to a target protein from a plurality of compounds, the device comprising:

a storage unit which stores a three-dimensional structure of a compound formed of a plurality of atoms and the first invariant feature quantity of the three-dimensional structure of the compound in association with each other for each of the plurality of compounds;
a feature quantity calculation unit which calculates the first invariant feature quantity of a ligand that is a compound whose binding to the target protein has been confirmed using the feature quantity calculating method according to claim 3;
a similarity calculation unit which calculates a similarity between the first invariant feature quantity of the plurality of compounds and the first invariant feature quantity of the ligand; and
a compound extraction unit which extracts the target compound from the plurality of compounds based on the similarity.

45. The screening device according to claim 43 or 44,
wherein the compound extraction unit extracts a compound having a similarity greater than or equal to a threshold.

46. The screening device according to any one of claims 43 to 45,
wherein the compound extraction unit extracts a compound in a descending order of the similarity.

**47.** A screening device which extracts a target compound that is bound to a target protein from a plurality of compounds, the device comprising:

a storage unit which stores a three-dimensional structure of a compound formed of a plurality of atoms and the first feature quantity calculated using the feature quantity calculating method according to claim 2 in association with each other for each of the plurality of compounds;
a feature quantity calculation unit which calculates the second feature quantity of the pocket structure of the target protein using the feature quantity calculating method according to claim 5;
a similarity calculation unit which calculates a similarity between the first feature quantity of the plurality of compounds and the second feature quantity of the pocket structure; and
a compound extraction unit which extracts the target compound from the plurality of compounds based on the similarity.

**48.** A screening device which extracts a target compound that is bound to a target protein from a plurality of compounds, the device comprising:

a storage unit which stores a three-dimensional structure of a compound formed of a plurality of atoms and the first invariant feature quantity calculated using the feature quantity calculating method according to claim 3 in association with each other for each of the plurality of compounds;
a feature quantity calculation unit which calculates the second invariant feature quantity of the pocket structure of the target protein using the feature quantity calculating method according to claim 6;
a similarity calculation unit which calculates a similarity between the first invariant feature quantity of the plurality of compounds and the second invariant feature quantity of the pocket structure; and
a compound extraction unit which extracts the target compound from the plurality of compounds based on the similarity.

**49.** The screening device according to claim 47 or 48,
wherein the compound extraction unit extracts a compound having a similarity greater than or equal to a threshold.

**50.** The screening device according to any one of claims 47 to 49,
wherein the compound extraction unit extracts a compound in a descending order of the similarity.

**51.** A screening device which extracts a target compound that is bound to a target biopolymer other than a protein from a plurality of compounds, the device comprising:

a storage unit which stores a three-dimensional structure of a compound formed of a plurality of atoms and the third feature quantity of the three-dimensional structure of the compound calculated using the feature quantity calculating method according to claim 8 in association with each other for each of the plurality of compounds;
a feature quantity calculation unit which calculates the third feature quantity of a binding compound that is a compound whose binding to the target biopolymer other than the protein has been confirmed;
a similarity calculation unit which calculates a similarity between the third feature quantity of the plurality of compounds and the third feature quantity of the binding compound; and
a compound extraction unit which extracts the target compound from the plurality of compounds based on the similarity.

**52.** A screening device which extracts a target compound that is bound to a target biopolymer from a plurality of compounds, the device comprising:

a storage unit which stores a three-dimensional structure of a compound formed of a plurality of atoms and the fourth feature quantity of the three-dimensional structure of the compound calculated using the feature quantity calculating method according to claim 11 in association with each other for each of the plurality of compounds;
a feature quantity calculation unit which calculates the fourth feature quantity of a binding compound that is a compound whose binding to the target biopolymer has been confirmed;
a similarity calculation unit which calculates a similarity between the fourth feature quantity of the plurality of compounds and the fourth feature quantity of the binding compound; and
a compound extraction unit which extracts the target compound from the plurality of compounds based on the similarity.

**53.** A screening device which extracts a target compound that is bound to a target biopolymer from a plurality of compounds, the device comprising:

a storage unit which stores a three-dimensional structure of a compound formed of a plurality of atoms and the fifth feature quantity of the three-dimensional structure of the compound calculated using the feature quantity calculating method according to claim 14 in association with each other for each of the plurality of compounds;
a feature quantity calculation unit which calculates the fifth feature quantity of a binding compound that is a compound whose binding to the target biopolymer has been confirmed;
a similarity calculation unit which calculates a similarity between the fifth feature quantity of the plurality of compounds and the fifth feature quantity of the binding compound; and
a compound extraction unit which extracts the target compound from the plurality of compounds based on the similarity.

**54.** A screening device which extracts a target compound that is bound to a target biopolymer from a plurality of compounds, the device comprising:

a storage unit which stores a three-dimensional structure of a compound formed of a plurality of atoms and the sixth feature quantity of the three-dimensional structure of the compound calculated using the feature quantity calculating method according to claim 17 in association with each other for each of the plurality of compounds;
a feature quantity calculation unit which calculates the sixth feature quantity of a binding compound that is a compound whose binding to the target biopolymer has been confirmed;
a similarity calculation unit which calculates a similarity between the sixth feature quantity of the plurality of compounds and the sixth feature quantity of the binding compound; and
a compound extraction unit which extracts the target compound from the plurality of compounds based on the similarity.

**55.** A screening device which extracts a target compound that is bound to a target biopolymer from a plurality of compounds, the device comprising:

a storage unit which stores a three-dimensional structure of a compound formed of a plurality of atoms and the seventh feature quantity of the three-dimensional structure of the compound calculated using the feature quantity calculating method according to claim 20 in association with each other for each of the plurality of compounds;
a feature quantity calculation unit which calculates the seventh feature quantity of a binding compound that is a compound whose binding to the target biopolymer has been confirmed;
a similarity calculation unit which calculates a similarity between the seventh feature quantity of the plurality of compounds and the seventh feature quantity of the binding compound; and
a compound extraction unit which extracts the target compound from the plurality of compounds based on the similarity.

**56.** A screening device which extracts a target compound that is bound to a target biopolymer from a plurality of compounds, the device comprising:

a storage unit which stores a three-dimensional structure of a compound formed of a plurality of atoms and the eighth feature quantity of the three-dimensional structure of the compound calculated using the feature quantity calculating method according to claim 23 in association with each other for each of the plurality of compounds;
a feature quantity calculation unit which calculates the eighth feature quantity of a binding compound that is a compound whose binding to the target biopolymer has been confirmed;
a similarity calculation unit which calculates a similarity between the eighth feature quantity of the plurality of compounds and the eighth feature quantity of the binding compound; and
a compound extraction unit which extracts the target compound from the plurality of compounds based on the similarity.

**57.** A compound creating method of creating a three-dimensional structure of a target compound that is bound to a target protein from a plurality of compounds, the method comprising:

a storing step of storing a three-dimensional structure of a compound formed of a plurality of atoms and the first feature quantity in association with each other for each of the plurality of compounds;
a feature quantity calculating step of calculating the first feature quantity of a ligand that is a compound whose binding to the target protein has been confirmed using the feature quantity calculating method according to claim 2;

a generator constructing step of constructing a generator through machine learning using the three-dimensional structure of the plurality of compounds as teacher data and the first feature quantity as an explanatory variable; and

a compound three-dimensional structure generating step of generating a three-dimensional structure of the target compound from the first feature quantity of the ligand using the generator.

58. A compound creating method of creating a three-dimensional structure of a target compound that is bound to a target protein from a plurality of compounds, the method comprising:

a storing step of storing a three-dimensional structure of a compound formed of a plurality of atoms and the first invariant feature quantity calculated using the feature quantity calculating method according to claim 3 in association with each other for each of the plurality of compounds;

a feature quantity calculating step of calculating the first invariant feature quantity of a ligand that is a compound whose binding to the target protein has been confirmed;

a generator constructing step of constructing a generator through machine learning using the three-dimensional structure of the plurality of compounds as teacher data and the first invariant feature quantity as an explanatory variable; and

a compound three-dimensional structure generating step of generating a three-dimensional structure of the target compound from the first invariant feature quantity of the ligand using the generator.

59. A compound creating method of creating a three-dimensional structure of a target compound that is bound to a target protein from a plurality of compounds, the method comprising:

a storing step of storing a three-dimensional structure of a compound formed of a plurality of atoms and the first feature quantity calculated using the feature quantity calculating method according to claim 2 in association with each other for each of the plurality of compounds;

a feature quantity calculating step of calculating the second feature quantity of the pocket structure of the target protein using the feature quantity calculating method according to claim 5;

a generator constructing step of constructing a generator through machine learning using the three-dimensional structure of the plurality of compounds as teacher data and the first feature quantity as an explanatory variable; and

a compound three-dimensional structure generating step of generating a three-dimensional structure of the target compound from the second feature quantity of the pocket structure using the generator.

60. A compound creating method of creating a three-dimensional structure of a target compound that is bound to a target protein from a plurality of compounds, the method comprising:

a storing step of storing a three-dimensional structure of a compound formed of a plurality of atoms and the first invariant feature quantity calculated using the feature quantity calculating method according to claim 3 in association with each other for each of the plurality of compounds;

a feature quantity calculating step of calculating the second invariant feature quantity of the pocket structure of the target protein using the feature quantity calculating method according to claim 6;

a generator constructing step of constructing a generator through machine learning using the three-dimensional structure of the plurality of compounds as teacher data and the first invariant feature quantity as an explanatory variable; and

a compound three-dimensional structure generating step of generating a three-dimensional structure of the target compound from the second invariant feature quantity of the pocket structure using the generator.

61. A compound creating method of creating a three-dimensional structure of a target compound that is bound to a target biopolymer other than a protein from a plurality of compounds, the method comprising:

a storing step of storing a three-dimensional structure of a compound formed of a plurality of atoms and the third feature quantity in association with each other for each of the plurality of compounds;

a feature quantity calculating step of calculating the third feature quantity of a binding compound that is a compound whose binding to the target biopolymer other than the protein has been confirmed using the feature quantity calculating method according to claim 8;

a generator constructing step of constructing a generator through machine learning using the three-dimensional structure of the plurality of compounds as teacher data and the third feature quantity as an explanatory variable; and

a compound three-dimensional structure generating step of generating a three-dimensional structure of the target compound from the third feature quantity of the binding compound using the generator.

62. A compound creating method of creating a three-dimensional structure of a target compound that is bound to a target biopolymer from a plurality of compounds, the method comprising:

a storing step of storing a three-dimensional structure of a compound formed of a plurality of atoms and the fourth feature quantity in association with each other for each of the plurality of compounds;

a feature quantity calculating step of calculating the fourth feature quantity of a binding compound that is a compound whose binding to the target biopolymer has been confirmed using the feature quantity calculating method according to claim 11;

a generator constructing step of constructing a generator through machine learning using the three-dimensional structure of the plurality of compounds as teacher data and the fourth feature quantity as an explanatory variable; and

a compound three-dimensional structure generating step of generating a three-dimensional structure of the target compound from the fourth feature quantity of the binding compound using the generator.

63. A compound creating method of creating a three-dimensional structure of a target compound that is bound to a target biopolymer from a plurality of compounds, the method comprising:

a storing step of storing a three-dimensional structure of a compound formed of a plurality of atoms and the fifth feature quantity in association with each other for each of the plurality of compounds;

a feature quantity calculating step of calculating the fifth feature quantity of a binding compound that is a compound whose binding to the target biopolymer has been confirmed using the feature quantity calculating method according to claim 14;

a generator constructing step of constructing a generator through machine learning using the three-dimensional structure of the plurality of compounds as teacher data and the fifth feature quantity as an explanatory variable; and

a compound three-dimensional structure generating step of generating a three-dimensional structure of the target compound from the fifth feature quantity of the binding compound using the generator.

64. A compound creating method of creating a three-dimensional structure of a target compound that is bound to a target biopolymer from a plurality of compounds, the method comprising:

a storing step of storing a three-dimensional structure of a compound formed of a plurality of atoms and the sixth feature quantity in association with each other for each of the plurality of compounds;

a feature quantity calculating step of calculating the sixth feature quantity of a binding compound that is a compound whose binding to the target biopolymer has been confirmed using the feature quantity calculating method according to claim 17;

a generator constructing step of constructing a generator through machine learning using the three-dimensional structure of the plurality of compounds as teacher data and the sixth feature quantity as an explanatory variable; and

a compound three-dimensional structure generating step of generating a three-dimensional structure of the target compound from the sixth feature quantity of the binding compound using the generator.

65. A compound creating method of creating a three-dimensional structure of a target compound that is bound to a target biopolymer from a plurality of compounds, the method comprising:

a storing step of storing a three-dimensional structure of a compound formed of a plurality of atoms and the seventh feature quantity in association with each other for each of the plurality of compounds;

a feature quantity calculating step of calculating the seventh feature quantity of a binding compound that is a compound whose binding to the target biopolymer has been confirmed using the feature quantity calculating method according to claim 20;

a generator constructing step of constructing a generator through machine learning using the three-dimensional structure of the plurality of compounds as teacher data and the seventh feature quantity as an explanatory variable; and

a compound three-dimensional structure generating step of generating a three-dimensional structure of the target compound from the seventh feature quantity of the binding compound using the generator.

66. A compound creating method of creating a three-dimensional structure of a target compound that is bound to a target biopolymer from a plurality of compounds, the method comprising:

a storing step of storing a three-dimensional structure of a compound formed of a plurality of atoms and the eighth feature quantity in association with each other for each of the plurality of compounds;

a feature quantity calculating step of calculating the eighth feature quantity of a binding compound that is a compound whose binding to the target biopolymer has been confirmed using the feature quantity calculating method according to claim 23;

a generator constructing step of constructing a generator through machine learning using the three-dimensional structure of the plurality of compounds as teacher data and the eighth feature quantity as an explanatory variable; and

a compound three-dimensional structure generating step of generating a three-dimensional structure of the target compound from the eighth feature quantity of the binding compound using the generator.

67. A compound creating program which causes a computer to execute the compound creating method according to any one of claims 57 to 66.

68. A compound creating device which creates a three-dimensional structure of a target compound that is bound to a target protein from a plurality of compounds, the device comprising:

a storage unit which stores a three-dimensional structure of a compound formed of a plurality of atoms and the first feature quantity in association with each other for each of the plurality of compounds;

a feature quantity calculation unit which calculates the first feature quantity of a ligand that is a compound whose binding to the target protein has been confirmed using the feature quantity calculating method according to claim 2;

a generator construction unit which constructs a generator through machine learning using the three-dimensional structure of the plurality of compounds as teacher data and the first feature quantity as an explanatory variable; and

a compound three-dimensional structure generation unit which generates a three-dimensional structure of the target compound from the first feature quantity of the ligand using the generator.

69. A compound creating device which creates a three-dimensional structure of a target compound that is bound to a target protein from a plurality of compounds, the device comprising:

a storage unit which stores a three-dimensional structure of a compound formed of a plurality of atoms and the first invariant feature quantity calculated using the feature quantity calculating method according to claim 3 in association with each other for each of the plurality of compounds;

a feature quantity calculation unit which calculates the first invariant feature quantity of a ligand that is a compound whose binding to the target protein has been confirmed;

a generator construction unit which constructs a generator through machine learning using the three-dimensional structure of the plurality of compounds as teacher data and the first invariant feature quantity as an explanatory variable; and

a compound three-dimensional structure generation unit which generates a three-dimensional structure of the target compound from the first invariant feature quantity of the ligand using the generator.

70. A compound creating device which creates a three-dimensional structure of a target compound that is bound to a target protein from a plurality of compounds, the device comprising:

a storage unit which stores a three-dimensional structure of a compound formed of a plurality of atoms and the first feature quantity calculated using the feature quantity calculating method according to claim 2 in association with each other for each of the plurality of compounds;

a feature quantity calculation unit which calculates the second feature quantity of the pocket structure of the target protein using the feature quantity calculating method according to claim 5;

a generator construction unit which constructs a generator through machine learning using the three-dimensional structure of the plurality of compounds as teacher data and the first feature quantity as an explanatory variable; and

a compound three-dimensional structure generation unit which generates a three-dimensional structure of the target compound from the second feature quantity of the pocket structure using the generator.

71. A compound creating device which creates a three-dimensional structure of a target compound that is bound to a target protein from a plurality of compounds, the device comprising:

a storage unit which stores a three-dimensional structure of a compound formed of a plurality of atoms and the first invariant feature quantity calculated using the feature quantity calculating method according to claim 3 in association with each other for each of the plurality of compounds;

a feature quantity calculation unit which calculates the second invariant feature quantity of the pocket structure of the target protein using the feature quantity calculating method according to claim 6;

a generator construction unit which constructs a generator through machine learning using the three-dimensional structure of the plurality of compounds as teacher data and the first invariant feature quantity as an explanatory variable; and

a compound three-dimensional structure generation unit which generates a three-dimensional structure of the target compound from the second invariant feature quantity of the pocket structure using the generator.

72. A compound creating device which creates a three-dimensional structure of a target compound that is bound to a target biopolymer other than a protein from a plurality of compounds, the device comprising:

a storage unit which stores a three-dimensional structure of a compound formed of a plurality of atoms and the third feature quantity in association with each other for each of the plurality of compounds;

a feature quantity calculation unit which calculates the third feature quantity of a binding compound that is a compound whose binding to the target biopolymer other than the protein has been confirmed using the feature quantity calculating method according to claim 8;

a generator construction unit which constructs a generator through machine learning using the three-dimensional structure of the plurality of compounds as teacher data and the third feature quantity as an explanatory variable; and

a compound three-dimensional structure generation unit which generates a three-dimensional structure of the target compound from the third feature quantity of the binding compound using the generator.

73. A compound creating device which creates a three-dimensional structure of a target compound that is bound to a target biopolymer from a plurality of compounds, the device comprising:

a storage unit which stores a three-dimensional structure of a compound formed of a plurality of atoms and the fourth feature quantity in association with each other for each of the plurality of compounds;

a feature quantity calculation unit which calculates the fourth feature quantity of the binding compound that is a compound whose binding to the target biopolymer has been confirmed using the feature quantity calculating method according to claim 11;

a generator construction unit which constructs a generator through machine learning using the three-dimensional structure of the plurality of compounds as teacher data and the fourth feature quantity as an explanatory variable; and

a compound three-dimensional structure generation unit which generates a three-dimensional structure of the target compound from the fourth feature quantity of the binding compound using the generator.

74. A compound creating device which creates a three-dimensional structure of a target compound that is bound to a target biopolymer from a plurality of compounds, the device comprising:

a storage unit which stores a three-dimensional structure of a compound formed of a plurality of atoms and the fifth feature quantity in association with each other for each of the plurality of compounds;

a feature quantity calculation unit which calculates the fifth feature quantity of the binding compound that is a compound whose binding to the target biopolymer has been confirmed using the feature quantity calculating method according to claim 14;

a generator construction unit which constructs a generator through machine learning using the three-dimensional structure of the plurality of compounds as teacher data and the fifth feature quantity as an explanatory variable; and

a compound three-dimensional structure generation unit which generates a three-dimensional structure of the target compound from the fifth feature quantity of the binding compound using the generator.

75. A compound creating device which creates a three-dimensional structure of a target compound that is bound to a target biopolymer from a plurality of compounds, the device comprising:

a storage unit which stores a three-dimensional structure of a compound formed of a plurality of atoms and the sixth feature quantity in association with each other for each of the plurality of compounds;
a feature quantity calculation unit which calculates the sixth feature quantity of the binding compound that is a compound whose binding to the target biopolymer has been confirmed using the feature quantity calculating method according to claim 17;
a generator construction unit which constructs a generator through machine learning using the three-dimensional structure of the plurality of compounds as teacher data and the sixth feature quantity as an explanatory variable; and
a compound three-dimensional structure generation unit which generates a three-dimensional structure of the target compound from the sixth feature quantity of the binding compound using the generator.

76. A compound creating device which creates a three-dimensional structure of a target compound that is bound to a target biopolymer from a plurality of compounds, the device comprising:

a storage unit which stores a three-dimensional structure of a compound formed of a plurality of atoms and the seventh feature quantity in association with each other for each of the plurality of compounds;
a feature quantity calculation unit which calculates the seventh feature quantity of the binding compound that is a compound whose binding to the target biopolymer has been confirmed using the feature quantity calculating method according to claim 20;
a generator construction unit which constructs a generator through machine learning using the three-dimensional structure of the plurality of compounds as teacher data and the seventh feature quantity as an explanatory variable; and
a compound three-dimensional structure generation unit which generates a three-dimensional structure of the target compound from the seventh feature quantity of the binding compound using the generator.

77. A compound creating device which creates a three-dimensional structure of a target compound that is bound to a target biopolymer from a plurality of compounds, the device comprising:

a storage unit which stores a three-dimensional structure of a compound formed of a plurality of atoms and the eighth feature quantity in association with each other for each of the plurality of compounds;
a feature quantity calculation unit which calculates the eighth feature quantity of the binding compound that is a compound whose binding to the target biopolymer has been confirmed using the feature quantity calculating method according to claim 23;
a generator construction unit which constructs a generator through machine learning using the three-dimensional structure of the plurality of compounds as teacher data and the eighth feature quantity as an explanatory variable; and
a compound three-dimensional structure generation unit which generates a three-dimensional structure of the target compound from the eighth feature quantity of the binding compound using the generator.

78. A non-temporary computer-readable recording medium which stores the feature quantity calculating program according to claim 26.

79. A non-temporary computer-readable recording medium which stores the screening program according to claim 42.

80. A non-temporary computer-readable recording medium which stores the compound creating program according to claim 67.

# FIG. 1

# FIG. 2

100

INFORMATION INPUT UNIT — 110

FEATURE QUANTITY
CALCULATION UNIT — 120

SIMILARITY
CALCULATION UNIT — 130

COMPOUND
EXTRACTION UNIT — 140

DISPLAY CONTROL UNIT — 150

CPU — 160

ROM — 170

RAM — 180

# FIG. 3

200

| |
|---|
| STRUCTURE INFORMATION — 210 |
| THREE-DIMENSIONAL STRUCTURE INFORMATION — 220 |
| THREE-DIMENSIONAL AAM DESCRIPTOR — 230 |
| INVARIANT AAM DESCRIPTOR — 240 |
| SIMILARITY INFORMATION — 250 |
| COMPOUND EXTRACTION RESULT — 260 |

# FIG. 4

# FIG. 5

```
           ( START )
              │
              ▼
┌──────────────────────────────┐
│   INPUT STRUCTURAL FORMULA    │── S100
│        OF COMPOUND            │
└──────────────────────────────┘
              │
              ▼
┌──────────────────────────────┐
│ FORM INPUT STRUCTURAL FORMULA │── S102
│ INTO THREE-DIMENSIONAL        │
│ STRUCTURE FORMULA             │
└──────────────────────────────┘
              │
              ▼
┌──────────────────────────────┐
│   CALCULATE SPATIAL           │
│   DISTRIBUTION                │── S104
│   ΔG_{aμ}(r) OF FREE ENERGY   │
│   FELT BY EACH ATOM "μ" OF    │
│   AMINO ACID "a" (r = (x, y, z))│
└──────────────────────────────┘
              │
              ▼
┌──────────────────────────────┐
│  CALCULATE DISTRIBUTION       │
│  FUNCTION g_{aμ}(r) OF EACH   │── S106
│  ATOM "μ" OF AMINO ACID "a"   │
│  FROM ΔG_{aμ}(r)              │
└──────────────────────────────┘
              │
              ▼
┌──────────────────────────────┐
│  CALCULATE DISTRIBUTION       │
│  FUNCTION g_a(r) OF CENTER OF │── S108
│  GRAVITY OF AMINO ACID FROM   │
│  DISTRIBUTION FUNCTION g_{aμ}(r)│
└──────────────────────────────┘
              │
              ▼
           ( END )
```

The process flow calculates spatial distribution $\Delta G_{a\mu}(r)$ of free energy felt by each atom "$\mu$" of amino acid "$a$" ($r = (x, y, z)$) at S104, then calculates distribution function $g_{a\mu}(r)$ of each atom "$\mu$" of amino acid "$a$" from $\Delta G_{a\mu}(r)$ at S106, then calculates distribution function $g_a(r)$ of center of gravity of amino acid from distribution function $g_{a\mu}(r)$ at S108.

## FIG. 6A

## FIG. 6B

## FIG. 7

(a)             (b)

# FIG. 8

| | THREE-DIMENSIONAL AAM DESCRIPTOR (LEVEL SURFACE WITH RESPECT TO THRESHOLD) | THREE-DIMENSIONAL AAM DESCRIPTOR (LEVEL SURFACE WITH RESPECT TO THRESHOLD) AND THREE-DIMENSIONAL STRUCTURE OF COMPOUND |
|---|---|---|
| DIRECTION 1 | | |
| DIRECTION 2 | | |
| DIRECTION 3 | | |

# FIG. 9

START

INPUT ACTUAL MEASUREMENT RESULT
OF X-RAY CRYSTAL STRUCTURE
AND POSITION INFORMATION OF POCKET
OF TARGET STRUCTURE — S200

PACK VIRTUAL SPHERES INTO POCKET
OF TARGET PROTEIN — S202

OBTAIN THREE-DIMENSIONAL STRUCTURE
OF POCKET STRUCTURE FORMED
OF COLLECTION OF PACKED SPHERES — S204

THREE-DIMENSIONALLY QUANTIFY DEGREE
OF ACCUMULATION OF AMINO ACIDS IN
PERIPHERY OF POCKET STRUCTURE USING
ACTUAL MEASUREMENT OF X-RAY CRYSTAL
STRUCTURE OF TARGET PROTEIN — S206

END

## FIG. 10A

## FIG. 10B

## FIG. 10C

## FIG. 11A

## FIG. 11B

# FIG. 12A

# FIG. 12B

## FIG. 13

Chart with Y-axis "EXPECTATION VALUE OF NUMBER OF HITS" (0 to 10) and X-axis "TEAM NUMBER" (0 to 50).

Legend:
- RANDOM
- INVARIANT AAM DESCRIPTOR (THIS TIME)
- Fingerprint (RELATED ART)

# FIG. 14

# FIG. 15

# FIG. 16

```
            ( START )
                |
                v
+-------------------------------------+
|  CALCULATE THREE-DIMENSIONAL        |~S300
|  AAM DESCRIPTOR OF LIGAND           |
+-------------------------------------+
                |
                v
+-------------------------------------+
|  CALCULATE SIMILARITY BETWEEN       |
|  THREE-DIMENSIONAL AAM DESCRIPTOR   |~S302
|  OF COMPOUND AND THREE-DIMENSIONAL  |
|  AAM DESCRIPTOR OF LIGAND           |
+-------------------------------------+
                |
                v
+-------------------------------------+
|  EXTRACT TARGET COMPOUND BASED      |~S304
|  ON SIMILARITY                      |
+-------------------------------------+
                |
                v
            (  END  )
```

# FIG. 17A

| LIGAND | | COMPOUND | | SIMILARITY | EXTRACTION RESULT |
|---|---|---|---|---|---|
| IDENTIFICATION INFORMATION | THREE-DIMENSIONAL AAM DESCRIPTOR | IDENTIFICATION INFORMATION | THREE-DIMENSIONAL AAM DESCRIPTOR | | |
| L00001 | L00001_$g_i(r)$ | C00001 | C00001_g1(r) | 85% | EXTRACTED |
| | | C00002 | C00002_g1(r) | 73% | NOT EXTRACTED |
| | | ... | ... | ... | ... |
| | | C00100 | C00100_g1(r) | 91% | EXTRACTED |
| | | ... | ... | ... | ... |
| | | C01000 | C01000_g1(r) | 80% | EXTRACTED |
| | | ... | ... | ... | ... |

EP 3 699 916 A1

## FIG. 17B

| LIGAND | | COMPOUND | | SIMILARITY | | EXTRACTION RESULT |
| IDENTIFICATION INFORMATION | INVARIANT AAM DESCRIPTOR | IDENTIFICATION INFORMATION | INVARIANT AAM DESCRIPTOR | VALUE | RANK | |
|---|---|---|---|---|---|---|
| L00001 | $L00001\_F_{12}(s)$ | C00100 | $C00100\_F_{12}(s)$ | 91% | 1 | EXTRACTED |
| | | C00001 | $C00001\_F_{12}(s)$ | 85% | 2 | EXTRACTED |
| | | ... | ... | ... | ... | ... |
| | | C01000 | $C01000\_F_{12}(s)$ | 80% | 100 | EXTRACTED |
| | | ... | ... | ... | ... | ... |
| | | C00002 | $C00002\_F_{12}(s)$ | 73% | 265 | NOT EXTRACTED |
| | | ... | ... | ... | ... | ... |

EXTRACTED

EP 3 699 916 A1

# FIG. 18

START

CALCULATE THREE-DIMENSIONAL AAM
DESCRIPTOR OF POCKET STRUCTURE OF
TARGET PROTEIN — S400

CALCULATE SIMILARITY BETWEEN
THREE-DIMENSIONAL AAM DESCRIPTOR
OF COMPOUND AND THREE-DIMENSIONAL
AAM DESCRIPTOR OF POCKET STRUCTURE — S402

EXTRACT TARGET COMPOUND BASED
ON SIMILARITY — S404

END

# FIG. 19A

| POCKET STRUCTURE | | COMPOUND | | SIMILARITY | EXTRACTION RESULT |
|---|---|---|---|---|---|
| IDENTIFICATION INFORMATION | THREE-DIMENSIONAL AAM DESCRIPTOR | IDENTIFICATION INFORMATION | THREE-DIMENSIONAL AAM DESCRIPTOR | | |
| P00001 | P00001_$g_i$(r) | C00001 | C00001_g1(r) | 85% | EXTRACTED |
| | | C00002 | C00002_g1(r) | 73% | NOT EXTRACTED |
| | | ... | ... | ... | ... |
| | | C00100 | C00100_g1(r) | 91% | EXTRACTED |
| | | ... | ... | ... | ... |
| | | C01000 | C01000_g1(r) | 80% | EXTRACTED |
| | | ... | ... | ... | ... |

EP 3 699 916 A1

## FIG. 19B

| POCKET STRUCTURE | | COMPOUND | | SIMILARITY | | EXTRACTION RESULT |
|---|---|---|---|---|---|---|
| IDENTIFICATION INFORMATION | INVARIANT AAM DESCRIPTOR | IDENTIFICATION INFORMATION | INVARIANT AAM DESCRIPTOR | VALUE | RANK | |
| P00001 | $P00001\_F_{12}(s)$ | C00100 | $C00100\_F_{12}(s)$ | 91% | 1 | EXTRACTED |
| | | C00001 | $C00001\_F_{12}(s)$ | 85% | 2 | EXTRACTED |
| | | ... | ... | ... | ... | ... |
| | | C01000 | $C01000\_F_{12}(s)$ | 80% | 100 | EXTRACTED |
| | | ... | ... | ... | ... | ... |
| | | C00001 | $C00001\_F_{12}(s)$ | 73% | 265 | NOT EXTRACTED |
| | | ... | ... | ... | ... | ... |

EXTRACTED

EP 3 699 916 A1

# FIG. 20

# FIG. 21

101

INFORMATION INPUT UNIT — 110

FEATURE QUANTITY
CALCULATION UNIT — 120

GENERATOR
CONSTRUCTION UNIT — 132

COMPOUND
THREE-DIMENSIONAL
STRUCTURE GENERATION UNIT — 142

DISPLAY CONTROL UNIT — 150

CPU — 160

ROM — 170

RAM — 180

# FIG. 22

201

STRUCTURE INFORMATION ⎯ 210

THREE-DIMENSIONAL
STRUCTURE INFORMATION ⎯ 220

THREE-DIMENSIONAL
AAM DESCRIPTOR ⎯ 230

INVARIANT
AAM DESCRIPTOR ⎯ 240

SIMILARITY INFORMATION ⎯ 250

THREE-DIMENSIONAL
STRUCTURE GENERATION
RESULT ⎯ 270

# FIG. 23

START

CALCULATE THREE-DIMENSIONAL
AAM DESCRIPTOR OF LIGAND — S500

GENERATE GENERATOR THROUGH
MACHINE LEARNING — S502

GENERATE THREE-DIMENSIONAL
STRUCTURE OF TARGET COMPOUND
FROM THREE-DIMENSIONAL
AAM DESCRIPTOR OF LIGAND USING
CONSTRUCTED GENERATOR — S504

END

## FIG. 24

**Step1:** CALCULATE THREE-DIMENSIONAL AAM DESCRIPTOR OF PLURALITY OF COMPOUNDS AND CREATE PAIR OF STRUCTURAL FORMULA AND THREE-DIMENSIONAL AAM DESCRIPTOR

**Step2:** THREE-DIMENSIONAL DESCRIPTOR (EXPLANATORY VARIABLE)  THREE-DIMENSIONAL STRUCTURE OF COMPOUND (TEACHER DATA)

DEEP LEARNING

LEARNED MACHINE = GENERATOR

FIG. 25

## FIG. 26A

STRUCTURAL FORMULA GENERATED
FROM THREE-DIMENSIONAL
AAM DESCRIPTOR

## FIG. 26B

CORRECT STRUCTURAL
FORMULA

# FIG. 27

START

CALCULATE THREE-DIMENSIONAL
AAM DESCRIPTOR OF POCKET STRUCTURE
OF TARGET PROTEIN — S600

GENERATE GENERATOR THROUGH
MACHINE LEARNING — S602

GENERATE THREE-DIMENSIONAL
STRUCTURE OF TARGET COMPOUND
FROM THREE-DIMENSIONAL AAM DESCRIPTOR
OF POCKET STRUCTURE USING GENERATOR — S604

END

# FIG. 28

500

510

NW

30

102

PROCESSING UNIT

202

STORAGE UNIT

300

OPERATION UNIT

410

KEYBOARD

420

MOUSE

400

DISPLAY UNIT

310

MONITOR

## FIG. 29

102

| INFORMATION INPUT UNIT | ——110 |

| FEATURE QUANTITY CALCULATION UNIT | ——120 |

| SIMILARITY CALCULATION UNIT | ——130 |

| GENERATOR CONSTRUCTION UNIT | ——132 |

| COMPOUND EXTRACTION UNIT | ——140 |

| COMPOUND THREE-DIMENSIONAL STRUCTURE GENERATION UNIT | ——142 |

| DISPLAY CONTROL UNIT | ——150 |

| CPU | ——160 |

| ROM | ——170 |

| RAM | ——180 |

# FIG. 30

202

STRUCTURE INFORMATION — 210

THREE-DIMENSIONAL
STRUCTURE INFORMATION — 220

THREE-DIMENSIONAL
AAM DESCRIPTOR — 230

INVARIANT
AAM DESCRIPTOR — 240

SIMILARITY INFORMATION — 250

COMPOUND
EXTRACTION RESULT — 260

THREE-DIMENSIONAL
STRUCTURE GENERATION
RESULT — 270

# FIG. 31

Legend:
- INVARIANT AAM DESCRIPTOR (FIRST INVARIANT FEATURE QUANTITY)
- INVARIANT ION (Na+, Cl-) (THIRD INVARIANT FEATURE QUANTITY)
- INVARIANT AAM DESCRIPTOR AND ION (Na+, Cl-) (FOURTH INVARIANT FEATURE QUANTITY)
- INVARIANT DIPOLE (FIFTH INVARIANT FEATURE QUANTITY)
- INVARIANT AAM DESCRIPTOR AND DIPOLE (SIXTH INVARIANT FEATURE QUANTITY)
- INVARIANT ION (Na+, Cl-) AND DIPOLE (SEVENTH INVARIANT FEATURE QUANTITY)
- INVARIANT AAM DESCRIPTOR, ION (Na+, Cl-), AND DIPOLE (EIGHTH INVARIANT FEATURE QUANTITY)

Y-axis: EXPECTATION VALUE

X-axis: TEAM NUMBER

## INTERNATIONAL SEARCH REPORT

| International application No. |
| --- |
| PCT/JP2018/037051 |

**A. CLASSIFICATION OF SUBJECT MATTER**
Int. Cl. G06F19/16(2011.01)i

According to International Patent Classification (IPC) or to both national classification and IPC

**B. FIELDS SEARCHED**

Minimum documentation searched (classification system followed by classification symbols)
Int. Cl. G06F19/16

Documentation searched other than minimum documentation to the extent that such documents are included in the fields searched
Published examined utility model applications of Japan        1922-1996
Published unexamined utility model applications of Japan      1971-2018
Registered utility model specifications of Japan              1996-2018
Published registered utility model applications of Japan      1994-2018

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2004-287812 A (THE JAPAN RESEARCH INSTITUTE, LTD.) 14 October 2004, entire text, all drawings (Family: none) | 1-80 |
| A | WO 2005/103994 A1 (KISSEI PHARMACEUTICAL CO., LTD.) 03 November 2005, entire text, all drawings (Family: none) | 1-80 |
| A | JP 2007-299125 A (TSUJI, Kazunori) 15 November 2007, entire text, all drawings (Family: none) | 1-80 |
| A | US 2012/0232856 A1 (BENDTSEN, Claus) 03 September 2012, whole document & WO 2011/036143 A1 & EP 2481003 A1 | 1-80 |

☐ Further documents are listed in the continuation of Box C.      ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 16.10.2018 | 30.10.2018 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9373059 B
- JP 5946045 B
- JP 4564097 B